# EUROPEAN PATENT APPLICATION

(11) **EP 4 023 218 A1**
(43) Date of publication of application: **06.07.2022**
(21) Application number: 21212116.4
(22) Date of filing: 02.12.2021
(51) Int. Cl.: A61K 31/138, A61K 31/341, A61K 31/585, A61K 31/165, A61K 31/403, A61K 31/353, A61K 31/453, A61K 31/196, A61K 31/401, A61K 31/216, A61K 31/41, A61K 31/4178, A61K 31/7042, A61K 31/7048, A61K 45/06, A61P 11/00, A61P 9/04

(54) **COMBINATION THERAPY FOR PATIENTS HAVING ACUTE AND/OR PERSISTENT DYSPNEA**

(30) Priority: 02.12.2020 EP 20211324
(71) Applicant: S-Form Pharma, 1180 Uccle (BE)
(72) Inventor: HALIOUA, Eric, 1180 Uccle (BE); SCORSIN, Marcio, 1180 Uccle (BE); MEBAZAA, Alexandre, 74014 Paris (FR)
(74) Representative: Kilger, Ute

(57) **Abstract**

Subject matter of the present invention is a combination medication for use in the treatment of a patient having acute and/or persistent dyspnea comprising at least two of the following components: a beta blocker, and one agent of the group consisting of an angiotensin-converting enzyme (ACE) inhibitor, an angiotensin II inhibitor and a combination of angiotensin II inhibitor or an angiotensin receptor-neprilysin inhibitor (ARNi), and mineralo receptor antagonist (MRA), gliflozine, a loop-acting diuretic, wherein a sample of bodily fluid of said patient has a level of BNP > 200 pg/mL and/or has a level NT-proBNP > 600 pg/mL, and wherein said sample of bodily fluid is selected from the group comprising blood, plasma and serum, and wherein said patient is either a patient with heart failure with left ventricular ejection fraction (LVEF) greater and equal to 40 %, preferably 50 %, or a patient with no heart failure.

SEQUENCE LISTING

<110>  S-Form Pharma

<120>  Combination Therapy for Patients having acute and/or persistent
       dyspnea

<130>  S75364EP01

<160>  9

<170>  PatentIn version 3.5

<210>  1
<211>  164
<212>  PRT
<213>  Human

<400>  1

Ala Arg Leu Asp Val Ala Ser Glu Phe Arg Lys Lys Trp Asn Lys Trp
1               5                   10                  15

Ala Leu Ser Arg Gly Lys Arg Glu Leu Arg Met Ser Ser Ser Tyr Pro
            20                  25                  30

Thr Gly Leu Ala Asp Val Lys Ala Gly Pro Ala Gln Thr Leu Ile Arg
        35                  40                  45

Pro Gln Asp Met Lys Gly Ala Ser Arg Ser Pro Glu Asp Ser Ser Pro
    50                  55                  60

Asp Ala Ala Arg Ile Arg Val Lys Arg Tyr Arg Gln Ser Met Asn Asn
65                  70                  75                  80

Phe Gln Gly Leu Arg Ser Phe Gly Cys Arg Phe Gly Thr Cys Thr Val
                85                  90                  95

Gln Lys Leu Ala His Gln Ile Tyr Gln Phe Thr Asp Lys Asp Lys Asp
            100                 105                 110

Asn Val Ala Pro Arg Ser Lys Ile Ser Pro Gln Gly Tyr Gly Arg Arg
        115                 120                 125

Arg Arg Arg Ser Leu Pro Glu Ala Gly Pro Gly Arg Thr Leu Val Ser
    130                 135                 140

Ser Lys Pro Gln Ala His Gly Ala Pro Ala Pro Pro Ser Gly Ser Ala
145                 150                 155                 160

Pro His Phe Leu

<210>  2
<211>  20
<212>  PRT
<213>  Human

<400>  2

Ala Arg Leu Asp Val Ala Ser Glu Phe Arg Lys Lys Trp Asn Lys Trp
1               5                   10                  15

Ala Leu Ser Arg
            20

<210>  3
<211>  48
<212>  PRT
<213>  Human

<400>  3

Glu Leu Arg Met Ser Ser Ser Tyr Pro Thr Gly Leu Ala Asp Val Lys
1               5                   10                  15

Ala Gly Pro Ala Gln Thr Leu Ile Arg Pro Gln Asp Met Lys Gly Ala
            20                  25                  30

Ser Arg Ser Pro Glu Asp Ser Ser Pro Asp Ala Ala Arg Ile Arg Val
        35                  40                  45

<210>  4
<211>  52
<212>  PRT
<213>  Human

<400>  4

Tyr Arg Gln Ser Met Asn Asn Phe Gln Gly Leu Arg Ser Phe Gly Cys
1               5                   10                  15

Arg Phe Gly Thr Cys Thr Val Gln Lys Leu Ala His Gln Ile Tyr Gln
            20                  25                  30

Phe Thr Asp Lys Asp Lys Asp Asn Val Ala Pro Arg Ser Lys Ile Ser
        35                  40                  45

Pro Gln Gly Tyr
    50

<210>  5
<211>  53
<212>  PRT
<213>  Human

<400>  5

Tyr Arg Gln Ser Met Asn Asn Phe Gln Gly Leu Arg Ser Phe Gly Cys
1               5                   10                  15

Arg Phe Gly Thr Cys Thr Val Gln Lys Leu Ala His Gln Ile Tyr Gln
            20                  25                  30

Phe Thr Asp Lys Asp Lys Asp Asn Val Ala Pro Arg Ser Lys Ile Ser
        35                  40                  45

Pro Gln Gly Tyr Gly
    50

<210>  6
<211>  38
<212>  PRT
<213>  Human

<400>  6

Arg Arg Arg Arg Arg Ser Leu Pro Glu Ala Gly Pro Gly Arg Thr Leu
1               5                   10                  15

Val Ser Ser Lys Pro Gln Ala His Gly Ala Pro Ala Pro Pro Ser Gly
            20                  25                  30

Ser Ala Pro His Phe Leu
        35

<210>  7
<211>  16
<212>  PRT
<213>  Human

<400>  7

Tyr Arg Gln Ser Met Asn Asn Phe Gln Gly Leu Arg Ser Phe Gly Cys
1               5                   10                  15

<210>  8
<211>  12
<212>  PRT
<213>  Human

<400>  8

Cys Thr Val Gln Lys Leu Ala His Gln Ile Tyr Gln
1               5                   10

<210>  9
<211>  12
<212>  PRT
<213>  Human

<400>  9

Cys Ala Pro Arg Ser Lys Ile Ser Pro Gln Gly Tyr
1               5                   10

## Description

Subject matter of the present invention is a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising at least three of the following compounds wherein each of said chemical compounds is a chemical entity: a beta blocker, and one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi), and mineralo receptor antagonist (MRA), an inhibitor of the sodium glucose co-transporter-2 (SGLT2), e.g. dapagliflozin or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea, a diurectic to reduce signs and/or symptoms of congestion in patients, and wherein a sample of bodily fluid of said patient has a level of BNP > 200 pg/mL and/or has a level NT-proBNP > 600 pg/mL and/or the patient is congestive, and wherein said sample of bodily fluid is selected from the group comprising blood, plasma and serum.

### State of the Art

Breathlessness is an unpleasant sensation of uncomfortable, rapid or difficult breathing. Everyone can experience breathlessness if they run or exert themself to an unusual extent, but breathlessness due to exercice is transient and respiration comes back to normal after minutes. By contrast, when breathlessness is present in resting condition or for moderate exercice and persistent for hours or days, it seeks medical attention, as it may be due to a serious underlying problem. The medical term of breathlessness is dyspnea that leads to hospital visit and/or admission and is associated with high risk of death.

Rapid onset (also named acute) dyspnea is considered as severe and need hospital visit when respiration rate is greater than 15 and oxygen saturation lower than 95% and even intensive care attention when associated with rate of breathing greater than 22 /min and/or hypoxia with oxygen saturation below 92 %. Those patients admitted with acute dyspnea may have metabolic disorders including acidosis and respiratory fatigue (Respiratory fatigue in patients with acute cardiogenic pulmonary edema, F. Mojoli, L. Monti, M. Zanierato, C. Campana, S. Mediani, L. Tavazzi, A. Braschi, European Heart Journal Supplements, Volume 6, Issue suppl_F, 1 November 2004, Pages F74-F80). Indeed, the rate of work of breathing of respiratory muscles markedly increase in acute dyspneic patients due to many combined mechanisms. This includes increased rate of breathing, greater contribution of abdominal and accessory inspiratory muscles of the neck to total respiration and/or increase in airway resistance. Concerning muscle contribution, with incremental increases in the rate of work of breathing, it has been shown that blood flow to the diaphragm raises exponentially suggesting a marked increase in diaphragm oxygen consumption though cardiac output and blood pressure do not change (J Clin Invest . 1977 Jan;59(1):31-42. doi: 10.1172/JCI108619. The relationship of respiratory failure to the oxygen consumption of, lactate production by, and distribution of blood flow among respiratory muscles during increasing inspiratory resistance, C H Robertson Jr, G H Foster, R L Johnson Jr).

Furthermore, there is an associated increase in airway resistance due to the presence of secretions, airway edema and/or bronchospasm seen in many causes of acute dyspnea including exacerbation of chronic obtructive pulmonary disease and/or acute pulmonary edema. Increase in airway resistance leads to increased lung volumes that is a costly compensatory mechanism as it is associated with a reduction in inspiratory capacity, worsening energetics of the inspiratory and expiratory muscles and overall further increases work of breathing (J Appl Physiol (1985). 2013 May;114(9): 1222-34. doi: 10.1152/japplphysiol.00981.2012. Epub 2013 Mar 21. Pathophysiology of muscle dysfunction in COPD, Joaquim Gea ¹, Alvar Agustí, Josep Roca). The latter results in increased anxiety and tachypnea further increasing work of breathing. In addition, there is direct evidence of marked sympathetic activation in patients with chronic respiratory failure (Marked Sympathetic Activation in Patients with Chronic Respiratory Failure

SILKE HEINDL , MATTHIAS LEHNERT , CARL-PETER CRIEE , GERD HASENFUSS , and STEFAN ANDREAS https://doi.org/10.1164/ajrccm.164.4.2007085 , PubMed: 11520722, Received: July 18, 2000). Many of those mechanisms are much more prononced in the elderly. In addition, peripheral and respiratory muscles are chronically impaired in old patients with comorbidities, such COPD, chronic heart failure or kidney disease. Impairment includes the loss of strength and/or endurance that can lead to ventilatory insufficiency and limits exercise capacity and activities of daily life.

Recommendend initial treatment of dyspnea usually combines oxygen therapy in case of hypoxia and the treatment of underlying cause(s), namely cardiac or non-cardiac or the combination of both cardiac and non-cardiac causes. Often, patients admitted with acute dyspnea are likely to rapidly restore normal gas exchange when treated with non-invasive ventilation, when indicated. More globally, on a 5-point Likert scale, patients admitted for acute dyspnea improve breathlessness by 76% after 6 h of standard therapy (Eur Heart J . 2010 Apr;31(7):832-41. doi: 10.1093/eurheartj/ehp458. Epub 2009 Nov 11; The impact of early standard therapy on dyspnoea in patients with acute heart failure: the URGENT-dyspnoea study, Alexandre Mebazaa ¹, Peter S Pang, Miguel Tavares, Sean P Collins, Alan B Storrow, Said Laribi, Stephanie Andre, Daniel Mark Courtney, Jennifer Hasa, Jindrich Spinar, Josep Masip, William Frank Peacock, Karen Sliwa, Etienne Gayat, Gerasimos Filippatos, John G F Cleland, Mihai Gheorghiade).

However, though breathlessness is markedly improved within hours, breathing rate remains abnormally high for several days and even after hospital discharge. However, there is no recommended therapies on persistent dyspnea. In addition, at least one third of patients with acute dyspnea are readmitted for new episode(s) of acute dyspnea in the following 90 days and no therapies succeded to prevent readmission (Kamile Ĉerlinskaitė et al., Readmission following both cardiac and non-cardiac acute dyspnoea is associated with a striking risk of death, ESC Heart Failure 2021 Aug;8(4):2473-2484. doi: 10.1002/ehf2.13369. Epub 2021 Jun 10).

Treatment of dyspnea also includes diagnosis and management of the cause of acute dyspnea. Dyspnea from non-cardiac origin represents roughly half of causes of acute dyspnea presenting to the emergency room. Various causes includes respiratory tract infections, exacerbation of chronic obstructive pulmonary disease (COPD) or lung cancer. Respiratory tract infections are one of the most common problems prompting visits to the emergency department. Although many are the result of self-limited viral illnesses, these infections may result in substantial morbidity and, rarely, mortality. Prompt recognition and appropriate treatment are needed. In addition, careful selection of antimicrobial agents is essential to maximize benefit. Acute exacerbations of chronic obstructive pulmonary disease (COPD) is another common cause of emergency room visit. Those patients are treated with oxygen (in hypoxemic patients), inhaled beta2 agonists, inhaled anticholinergics, antibiotics and systemic corticosteroids. In those patients, non-cardiac causes may be associated with congestion though no therapeutic guidance is described and no therapies have shown long-term benefits.

Dyspnea can also be due to cardiac origin, often named acute heart failure (AHF). New York Heart Association (NYHA) Classification has been described to provide a simple way of classifying the extent of heart failure ; NYHA may also be used to determine the severity of dyspnea from non-cardiac origin. NYHA classifies patients with acute and/or persistent dyspnea in one of four categories based on their limitations during physical activity; the limitations/symptoms are in regards to normal breathing and varying degrees in shortness of breath and or angina pain:
Class I - No limitation of physical activity. Ordinary physical activity does not cause undue fatigue, palpitation, dyspnea (shortness of breath).
Class II - Slight limitation of physical activity. Comfortable at rest. Ordinary physical activity results in fatigue, palpitation, dyspnea (shortness of breath).
Class III - Marked limitation of physical activity. Comfortable at rest. Less than ordinary activity causes fatigue, palpitation, or dyspnea.
Class IV Unable to carry on any physical activity without discomfort. Symptoms of heart failure at rest. If any physical activity is undertaken, discomfort increases.
NYHA is commonly used to classify acute and/or persistent dyspnea.

The prognosis of AHF is poor, with a 5-8% rate of in-hospital mortality and 10% additional post-discharge mortality. Almost no treatment has been approved yet for the treatment of acute heart failure. There is an immediate post-discharge period of risk that usually lasts at least 90 days and is known as the "vulnerable phase". The pathophysiology of the vulnerable phase remains controversial. Some studies suggested underutilization of heart failure oral therapies (HFOTs) at discharge . HFOTs, including beta-blockers (BB), renin-angiotensin system inhibitors (RASi: angiotensin conversion enzyme inhibitors or angiotensin receptor blockers or ARNi) and mineralocorticoid receptor antagonists (MRA) have long been shown to markedly improve outcomes in stable heart failure (HF) with reduced left ventricular ejection fraction (HFrEF). Most recent European and North American HF guidelines recommend the maintenance and/or introduction of HFOT at hospital discharge in HFrEF, based on a few studies showing that BB withdrawal in AHF patients was associated with an increased risk of post-discharge death. However, the maintenance or early introduction of HFOTs in patients who are fragile, still suffering or just recovered from the AHF crisis are sub-optimal, especially in old patients. Data on the effect of the maintenance, introduction, and withdrawal of RASi and/or MRA, alone or combined with BB, during AHF hospitalization are therefore needed. Recently, inhibitors of the sodium glucose co-transporter-2 (SGLT2) have been included in HFOTs.

Moreover, since many AHF patients present with preserved left ventricular ejection fraction (HFpEF), no data exist whether HFOT are beneficial in those patients. Despite demographic and clinical differences, the outcomes following AHF hospitalization in HFpEF patients are as poor as those in patients with HFrEF. Due to neutral trials, recent European and North American heart failure guidelines recommended not administering oral medications either in chronic or, by extension, decompensated HFpEF patients; recents trials suggested benefits of inhibitors of the sodium glucose co-transporter-2 (SGLT2) in HFpEF. It has been described that heart failure oral therapies (HFOTs), including beta-blockers (BB), renin-angiotensin system inhibitors (RASi) and mineralocorticoid receptor antagonists (MRA), administered before hospital discharge after acute heart failure (AHF) might improve outcome. However, concerns have been raised because early administration of HFOTs may worsen patient's condition. However, retrospective analysis of large international cohorts suggest that HFOTs at hospital discharge, whether alone or combined, might be associated with better outcomes 1) during the "post-discharge vulnerable phase" and 2) in all AHF patients, including those with HFpEF (Gayat E et al.; Heart failure oral therapies at discharge are associated with better outcome in acute heart failure: a propensity-score matched study. ; GREAT Network. Eur J Heart Fail. 2018 Feb;20(2):345-354).

### Description of the invention

Subject-matter of the present application is a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising at least three of the following compounds, wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II receptor-neprilysin inhibitors (ARNi)
c. and mineralo receptor antagonist (MRA),
d. an inhibitor of the sodium glucose co-transporter-2 (SGLT2), e.g. dapagliflozine or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea;
e. a diuretic to reduce or prevent occurrence of signs and/or symptoms of congestion in patients,
   and wherein a sample of bodily fluid of said patient has a level of Brain Natriuretic peptide (BNP) > 200 pg/mL and/or has a level of N-terminal (NT)-pro hormone BNP(NT-proBNP) > 600 pg/mL and/or the patient is congestive and/or has residual congestion, and wherein said sample of bodily fluid is selected from the group comprising blood, plasma and serum;
   and wherein said patient is either
      - a patient with heart failure with left ventricular ejection fraction (LVEF) greater and equal to 40 %, preferably 50 % or
      - a patient with no heart failure.

In the context of the present application dyspnea is defined/characterized as shortness of breath that is labored breathing, an uncomfortable condition that makes it difficult to fully get air into your lungs, at rest. Dyspnea corresponds to breathing discomfort with the feeling as if the patient cannot draw a complete breath. Dyspnea may be associated with increased respiratory rate and/or impaired arterial oxygen saturation and/or sympathetic tome stimulation including increased heart rate.

Patients with dyspnea may feel the need of medical attention. Acute dyspnea might be secondary to an acute problem, or it might be an exacerbation of an existing disease (eg, asthma, chronic obstructive pulmonary disease, heart failure) (John E Delzell Jr, FP Essent Action,. 2013 Jun;409:17-22., Common lung conditions: acute dyspnea).

The term "acute" is used to mean any condition with a short (less than 2 weeks, more preferably less than one week, more preferably less than one day, most preferably few hours) clinical course. In the context of the present application, acute is defined as dyspnea or shortness of breath that appears suddenly or gradually over hours or days. Dyspnea is associated with inflammation and endothelial dysfunction whether the cause of dyspnea is from cardiac or non cardiac origin. In case of dyspnea from cardiac origin, heart dysfunction is associated with congestion of vital organs, namely lungs, kidney, liver. Congestion leads to endothelial dysfunction that in turn worsens organ dysfunction. In non cardiac cause of dyspnea, there is often a lung infection and/or inflammation that is consistently associated with endothelial dysfunction that aggravates lung dysfunction.

In the context of the present application, persistent is defined as shortness of breath, including a respiration rate greater than 16 and/or an oxygen saturation lower than 95% that lasts several days, several weeks or several months. Persistent short of breath, breathlessness or dyspnea prevent the patient to perform exercice or effort.

In the context of the present application, the respiration rate (rate of breathing) is the number of breaths a person takes per minute. The rate is usually measured when a person is at rest and simply involves counting the number of breaths for one minute by counting how many times the chest rises. Respiration rates may increase with fever, illness, and other medical conditions. When checking respiration, it is important to also note whether a person has any difficulty breathing. Normal respiration rates for an adult person at rest range from 12 to 16 breaths per minute. Breath rate sensors can be used for monitoring respiration rate. However, a person can count the number of breath cycles (inspiration and expiration) over one minute: this is the respiration rate. A respiration rate greater than 16 breaths per minute is part of the definition of breathlessness, shortness of breath or dyspnea.

Oxygen saturation is the fraction of oxygen-saturated hemoglobin relative to total hemoglobin (unsaturated + saturated) in the blood. The human body requires and regulates a very precise and specific balance of oxygen in the blood. Normal arterial blood oxygen saturation levels in humans are 95-100 percent. If the level is below 92 percent, it is considered low and called hypoxemia. Arterial blood oxygen levels below 80 percent may compromise organ function, such as the brain and heart, and should be promptly addressed. Continued low oxygen levels may lead to respiratory or cardiac arrest. Oxygen therapy may be used to assist in raising blood oxygen levels. Oxygenation is commonly used to refer to medical oxygen saturation. Hypoxemia is a sign of a problem related to breathing or circulation, and may result in various symptoms, such as shortness of breath. By contrast, restoration of oxygen saturation indicates improved health condition. Blood oxygenation is most commonly assessed non-invasively by pulse oximetry. It is a noninvasive device placed over a person's finger. It measures light wavelengths to determine the ratio of the current levels of oxygenated hemoglobin to deoxygenated hemoglobin.

B-type natriuretic peptide (brain natriuretic peptide; BNP) is a 32-amino acid-ringed peptide secreted by the heart to regulate blood pressure and fluid balance.(1) BNP is stored in and secreted predominantly from membrane granules in the heart ventricles, and is continuously released from the heart in response to both ventricle volume expansion and pressure overload.

NT-Pro-BNP is a known predictor of cardiovascular events in the population (Melander et. Al, JAMA. 2009;302(1):49-57).

BNP > 200 pg/mL and/or a level of NT-proBNP > 600 pg/mL, preferably BNP > 300 pg/mL and/or a level of NT-proBNP > 800 pg/mL may be associated with alteration of cardio-vascular function, including congestion, atrial fibrillation and/or alteration of renal function in dyspneic patients regardless to their left ventricular ejection fraction. N-BNP can be measured as described by Melander et al., JAMA. 2009;302(1):49-57.

Biologically active adrenomedullin is a free-circulating peptide that is mainly expressed and secreted by vascular endothelial cells and is known for its vasodilatory activity in the interstitium resulting in decreased blood pressure. In the circulation, adrenomedullin directly tightens the gaps between endothelial cells, subsequently preventing vascular leakage.

Angiotensin-converting-enzyme inhibitors, angiotensin II receptor blockers with or without neprilysin inhibitors, antimineralocorticoid and beta-blockers are inhibitors of neuroendocrine (including sympethatic) system.

Angiotensin-converting-enzyme inhibitors inhibit the activity of angiotensin-converting enzyme, an important component of the renin-angiotensin system liable to convert angiotensin I to angiotensin II, and hydrolyze bradykinin. ACE inhibitors are used for the treatment of high blood pressure and heart failure and/or have been shown to improve outcome in patients with cardiovascular disease.

Angiotensin II receptor blockers (ARBs), formally angiotensin II receptor type 1 (AT1) antagonists, also known as angiotensin receptor blocker, angiotensin II receptor antagonists, or AT1 receptor antagonists, selectively block the activation of AT1 receptors, preventing the binding of angiotensin II. ARBs are used for the treatment of high blood pressure, diabetic nephropathy and heart failure.

The combination of the neprilysin inhibitor sacubitril and the angiotensin receptor blocker valsartan is recommended for use as a replacement for an ACE inhibitor or an angiotensin receptor blocker in patients with heart failure with reduced ejection fraction.

An antimineralocorticoid, MRA or an aldosterone antagonist, steroidal or non-steroidal, is a diuretic drug which antagonizes the action of aldosterone at mineralocorticoid receptors. This group of drugs is often used as adjunctive therapy, in combination with other drugs, for long-term management of heart failure. They improve long-term mortality of heart failure patients with reduced left ventricular ejection fraction.

Beta blockers are competitive antagonists that block the receptor sites for endogenous catecholamines on adrenergic beta receptors, of the sympathetic nervous system. Long-term administration of beta-blockers has been shown to improve outcome in many cardiovascular disease including coronary artery disease, arrythmia, hypertension, heart failure.

SGLT2 inhibitors, also called gliflozins, are a class of medications that act by inhibiting sodium-glucose co-transporter protein 2 (SGLT2) and inhibits reabsorption of glucose in the kidney and therefore lower blood sugar. Long-term administration of SGLT2-inhibitors has been shown to improve outcome in cardiovascular diseases (see also sodium-glucose co-transporter 2 inhibitors according to the substance class A10BK under WHO ATC classification).

A diuretic is a substance that promotes diuresis, the increased production of urine.

As used herein, a diuretic is selected from the group comprising loop-acting diuretics such as bumetanide, ethacrynic acid, furosemide, torsemide; potassium-sparing diuretics such as amiloride, eplerenone, spironolactone, triamterene , thiazide diuretics such as chlorothiazide, chlorthalidone, hydrochlorothiazide, indapamide, metolazone and/or mixtures thereof.

Loop diuretics are a powerful type of diuretic that work by inhibiting the sodium-potassium-chloride (Na+/K+/2Cl) co-transporter in the thick ascending loop of Henle (hence the name loop diuretic), which is located in the kidneys. This reduces or abolishes sodium, chloride, and potassium reabsorption, leading to increased loss of sodium, chloride, and potassium into the nephron (the functional unit of a kidney). As a result, water is also drawn into the nephron and urine volume increases. Loop diuretics also reduce the reabsorption of calcium and magnesium. Furosemide, bumetanide and torsemide are, for example, loop diuretics.

The term "patient" as used herein refers to a living human or non-human organism. Preferably herein the subject is a human subject.

Subject-matter of the present application is also a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising at least four of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), e.g. dapagliflozin or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea;
e. a diuretic to reduce or prevent occurence of signs and/or symptoms of congestion in patients,
   and wherein a sample of bodily fluid of said patient has a level of Brain Natriuretic peptide (BNP) > 200 pg/mL and/or has a level of N-terminal (NT)-pro hormone BNP(NT-proBNP) > 600 pg/mL and/or the patient is congestive and/or has residual congestion, and wherein said sample of bodily fluid is selected from the group comprising blood, plasma and serum;
   and wherein said patient is either
      - a patient with heart failure with LVEF, greater and equal to 40 %, preferably 50 % or
      - a patient with no heart failure.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said combination comprises an inhibitor of the sodium glucose co-transporter-2, e.g. dapagliflozin or empagliflozin that is used for restoring the respiration rate and/or maintaining the respiration rate and/or restoring and/or maintaining the oxygen saturation and/or for ameliorating the symptoms of dyspnea.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient does not have diabetes.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient has a myocardial dysfunction.

In the context of the present application, the term myocardial dysfunction relates to the alteration in systolic and diastolic function of the myocardium. This induces know or unknown dilatation of the one to four chambers of the hearts. In addition, it is associated with impaired contraction and relaxation of part or the total of the heart.It may related to many causes including myocardial acute or chronic ischemia, other cardiovascular disorders, metabolic disorders including diabetis and kidney failure, infection by viruses or others.

In the context of the present application, the term fibrosis relates to modification of myocardial structure with presence of fibrosis that is associated with myocardial dysfunction.

In the context of the present application, the term sclerosis relates to adnavced stage of myocardial fibrosis often following myocardial ischemia and cell deaths.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the treatment is a daily administration of the combination medication, preferably once a day.

The goal of the treatment of the present application is to improve respiratory conditions including to statistically decrease respiratory rate, increase oxygen saturation and improve quality of life, all toward normal values and to statiscally decrease mortality associated with acute and/or persistent dyspnea.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient is a patient with heart failure and/or wherein said patient has a reduced left-ventricular ejection fraction (LVEF) with less than 40%.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient is a patient with heart failure and/or wherein said patient has a left-ventricular ejection fraction (LVEF) greater than 40%.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient is a patient with heart failure and/or wherein said patient has a left-ventricular ejection fraction (LVEF) greater and equal to 50%.

Another embodiment of the present application relates to a combination medication for use in the treatment of a patient having acute and/or persistent dyspnea, wherein said patient is a patient with no heart-failure.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the patient has no heart failure, but has another cardiovascular disease and/or another disease with non cardiac cause which maybe selected from the group comprising diabetes and kidney disease, and wherein a sample of bodily fluid of said patient has a level of BNP > 200 pg/mL and/or has a level NT-proBNP > 600 pg/mL.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the patient has no heart failure, but has another cardiovascular disease and/or another disease with non cardiac cause which maybe selected from the group comprising diabetes and kidney disease, and wherein a sample of bodily fluid of said patient has a level of BNP > 300 pg/mL and/or has a level NT-proBNP > 800 pg/mL.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 5, wherein the patient has no heart failure, but has another cardiovascular disease and/or another disease with non cardiac cause which maybe selected from the group comprising diabetes and kidney disease, and wherein a sample of bodily fluid of said patient has a level of BNP > 200 pg/mL and/or has a level NT-proBNP > 600 pg/mL, wherein the bodily fluid is a plasma sample, preferably a fasting plasma sample.

For avoidance of doubts, tresholds for BNP and NT-proBNP refer to measured plasma samples, preferably fasting plasma samples (Melander et al., JAMA. 2009;302(1):49-57).

One further embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient has another disease with non cardiac cause which maybe selected from the group comprising diabetes and kidney disease. In a further embodiment, said patient may have several comorbidities.

In the context of the present application, a patient with a reduced left-ventricular ejection fraction (LVEF), a patient with a mildly reduced left-ventricular ejection fraction and a patient with a preserved ventricular ejection fraction is a patient with heart failure.

Surprisingly, in the study on which the application is based, it was observed that patients with no heart failure, but with other diseases with non cardiac cause, which were treated with the claimed combination medication showed a positive outcome.

Another embodiment of the present application relates to a combination medication for use in the treatment of a patient having acute and/or persistent dyspnea in a patient, wherein said patient
- is a patient with heart failure with left-ventricular ejection fraction (LVEF), greater and equal to 50 %, or
- a patient with no heart failure with a LVEF greater than 50% or a patient with no heart failure for whom LVEF was not measured.

In the context of the present application, heart failure with mildly reduced ejection fractionoccurs when the left chamber (left ventricle) is not able to fill properly with blood during the diastolic (filling) phase. However the amount of blood pumped out to the body is still adequate or midly altered. It is also called diastolic heart failure. The types of heart failure are based on a measurement called the left ventricle ejection fraction. The ejection fraction is usually measured only in the left ventricle (LV). The left ventricle is the heart's main pumping chamber. It pumps oxygen-rich blood up into the upward (ascending) aorta to the rest of the body. The left ventricle ejection fraction measures how much blood inside the ventricle is pumped out with each contraction. The left ventricle squeezes and pumps some (but not all) of the blood in the ventricle out to your body. An LV ejection fraction of 40 to 49%. is considered as mildly reduced. An LV ejection fraction equally 50 % or between 50 and 55 percent or greater than 55% is usually considered "preserved" in heart failure patients or normal in non-heart failure patients. Ejection fraction can be measured with imaging techniques, including:
- **Echocardiogram** (most common test used to measure ejection fraction) During an echocardiogram, sound waves are used to produce images of your heart and the blood pumping through your heart.
- **Cardiac catheterization.** During cardiac catheterization, a thin, plastic tube (catheter) is inserted into an artery in your arm or leg and then gently guided to your heart. Images taken during catheterization can measure the ejection fraction of your heart.
- **Magnetic resonance imaging (MRI).** An MRI uses magnetic field and radio waves to create cross-sectional images of specific parts of your body. When an MRI is used to study the heart, it's known as a cardiovascular MRI.
- **Computerized tomography (CT).** During a CT scan, a special X-ray technique is used to create cross-sectional images of specific parts of your body. When a CT scan is used to study the heart, it's known as a cardiac CT.
- **Nuclear medicine scan.** During a nuclear scan, trace amounts of radioactive material are injected into your bloodstream. Special cameras then detect the radioactive material in your blood as it flows through your heart and lungs.

For the avoidance of doubts for the present invention, the term "left-ventricular ejection fraction (LVEF) in %" is determined in an echocardiogram.

In the context of the present application, the term "mildly reduced left-ventricular ejection fraction (LVEF)" means 40% to 49 % LVEF, wherein said LVEF is measured with an echocardiogram (Ponikowski et al. 2016. European Heart Journal 18(8): 891-975*).*

In case of doubt, an LV ejection fraction of 55 % or higher, which is is considered as normal, is determined with an echocardiogram.

This means the group of patients which are treated with the claimed combination medication comprises patients with heart failure with left-ventricular ejection fraction (LVEF) greater and equal to 40%, or greater and equal to 50 % ,patients with no heart-failure with a LVEF greater than 50% or a patient with no heart failure for whom LVEF was not measured.

Heart failure (HF) is a cardiac condition that occurs, when a problem with the structure or function of the heart impairs its ability to supply sufficient blood flow to meet the body's needs. It can cause a large variety of symptoms, particularly shortness of breath (SOB, or breathlessness) at rest or during exercise, signs of fluid retention such as pulmonary congestion or ankle swelling and objective evidence of an abnormality of the structure or function of the heart at rest.

Heart failure is a clinical syndrome characterized by a constellation of symptoms and signs caused by cardiac dysfunction. It is one of the major causes of morbidity and mortality in the developed countries, with a prevalence of 1-2%. Heart failure can be grouped into chronic HF and acute HF. Patients with chronic HF can be grouped into stable chronic HF, worsening signs and symptoms of chronic HF and acute decompensation of chronic HF. Acute heart failure (AHF) is defined as a rapid onset of signs and symptoms of heart failure resulting in the need for urgent therapy or hospitalization. AHF can present as acute de novo HF (new onset of AHF in a patient without previous cardiac dysfunction) or acute decompensation of chronic HF. AHF is the leading cause of hospitalization in adults older than 65 years of age. Despite marked improvements in the prognosis of chronic heart failure patients primarily related to therapeutic advances over the past few decades, both short- and long-term outcomes remain very poor once patients are hospitalized for decompensated heart failure. Nearly 25% of patients hospitalized for AHF need readmission within 30 days of hospital discharge while <50% survive beyond 5 years after hospitalization. In addition to significantly reducing survival and quality of life of affected patients, the monetary burden of AHF on health care systems is enormous. The total cost of heart failure care was estimated to be $31 billion in the US alone in 2012 and majority of this cost is associated with in-hospital care. This cost is projected to increase to an unprecedented $70 billion in 2030 due to ageing of populations.

Heart failure comprises a wide range of patients, from those with normal left ventricular ejection fraction (LVEF) typically considered as ≥50%, also known as HF with preserved EF (HFpEF) to those with reduced LVEF, typically considered as <40%, also known as HF with reduced EF (HFrEF). Patients with an LVEF in the range of 40-49% represent a 'grey area', which is defined as HF with mid-range EF (HFmrEF) (Ponikowski et al. 2016. European Heart Journal 18(8): 891-975*).*

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea in a patient, and wherein said patient has a left-ventricular ejection fraction (LVEF) with more than or equal to 50%.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea in a patient, and wherein said patient has a mildly reduced left-ventricular ejection fraction (LVEF) with 40 to 49 %.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea in a patient, and wherein said patient has a preserved left-ventricular ejection fraction (LVEF) with more than or equal to 50%.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea in a patient, and wherein said patient has a left-ventricular ejection fraction (LVEF) with more than or equal to 60%.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea in a patient, and wherein said patient has a left-ventricular ejection fraction (LVEF) with more than or equal to 70%.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea in a patient, and wherein said patient has a left-ventricular ejection fraction (LVEF) with more than or equal to 80%.

Another specific embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea in a patient, and wherein said patient has a reduced left-ventricular ejection fraction (LVEF) with less than 40%.

Another embodiment of the present application relates to a combination medication for use in the treatment of a patient having acute and/or persistent dyspnea in a patient, wherein said patient has no heart failure (HF).

As used herein, heart failure is defined /characterized by clinical signs of heart failure other than dyspnea. There are several methods to measure or determine said cliniccal signs of heart failure other than dyspnea.

Echocardiography is commonly used to support a clinical diagnosis of heart failure. This modality uses ultrasound to determine the stroke volume (SV, the amount of blood in the heart that exits the ventricles with each beat), the end-diastolic volume (EDV, the total amount of blood at the end of diastole), and the SV in proportion to the EDV, a value known as the ejection fraction (EF). In pediatrics, the shortening fraction is the preferred measure of systolic function. Normally, the EF should be between 50 and 70%; in systolic heart failure, it drops below 40%. Echocardiography can also identify valvular heart disease and assess the state of the pericardium (the connective tissue sac surrounding the heart). Echocardiography may also aid in deciding what treatments will help the person, such as medication, insertion of an implantable cardioverter-defibrillator, or cardiac resynchronization therapy. Echocardiography can also help determine if acute myocardial ischemia is the precipitating cause, and may manifest as regional wall motion abnormalities on echo.

Chest X-rays are frequently used to aid in the diagnosis of congestive heart failure (CHF). In a person who is compensated, this may show cardiomegaly (visible enlargement of the heart), quantified as the cardiothoracic ratio (proportion of the heart size to the chest). In left ventricular failure, evidence may exist of vascular redistribution (upper lobe blood diversion or cephalization), Kerley lines, cuffing of the areas around the bronchi, and interstitial edema. Ultrasound of the lung may also be able to detect Kerley lines

An electrocardiogram (ECG/EKG) may be used to identify arrhythmias, ischemic heart disease, right and left ventricular hypertrophy, and presence of conduction delay or abnormalities (e.g. left bundle branch block). Although these findings are not specific to the diagnosis of heart failure, a normal ECG virtually excludes left ventricular systolic dysfunction.

Angiography is the X-ray imaging of blood vessels, which is done by injecting contrast agents into the bloodstream through a thin plastic tube (catheter), which is placed directly in the blood vessel. X-ray images are called angiograms. [46] Heart failure may be the result of coronary artery disease, and its prognosis depends in part on the ability of the coronary arteries to supply blood to the myocardium (heart muscle). As a result, coronary catheterization may be used to identify possibilities for revascularisation through percutaneous coronary intervention or bypass surgery.

Another preferred embodiment of the present application relates to a combination medication for use in the treatment of a patient having acute and/or persistent dyspnea in a patient, wherein said patient has no history of heart failure.

In the context of the present application, "no history" means patients who had former contacts with physician that did not diagnose heart failure.

One embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the patient has no clinical signs of congestion.

One embodiment of the present application relates to a combination medication for use in the treatment of a patient having acute and/or persistent dyspnea, wherein the patient has clinical signs of congestion.

As used herein, the term congestion is defined as abnormal accumulation of fluid, usually plasma, in the vessels and/or in the interstitium.

In the context of the present application, the term clinical signs of congestion are defined as an increase in body weight, elevated jugular venous pressure, jugular distension, hepato-jugular reflux, edema in the lower extremities, hepatomegaly, third heart sound, ascites, pulmonary crepitation.

One specific embodiment of the present application relates to a combination medication for use in the treatment of a patient having acute and/or persistent dyspnea in a patient, wherein the patient has no cardiovascular or pulmonary disease.

In the context of the present application, cardiovascular disease (CVD) is defined as the group of disorders of heart and blood vessels, and include: hypertension (high blood pressure), coronary heart disease (heart attack), cerebrovascular disease (stroke), peripheral vascular disease, heart failure, rheumatic heart disease, congenital heart disease, cardiomyopathies.

As used herein, pulmonary disease is defined as a type of disease that affects the lungs and other parts of the respiratory system. Pulmonary diseases include asthma, chronic obstructive pulmonary disease (COPD), pulmonary fibrosis, pneumonia, and lung cancer.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said combination is a fixed combination and wherein said combination is a fixed combination in one single administration form.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said combination is a fixed combination and wherein said combination is a fixed combination in one single administration form, wherein said administration form is selected from the group comprising a pill, a tablet, a liqiuid medicine, a capsule, a film tablet, a dragee.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said combination is a fixed combination and wherein said combination is a fixed combination in one single administration form, wherein said administration form is a single pill.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said combination is a fixed combination and wherein said combination is a fixed combination in one single administration form, wherein said administration form is a single polypill.

In the context of the present application a polypill refers to a medication that is a drug product in pill form (i.e., tablet or capsule) that *combines* multiple active pharmaceutical ingredients.

Another specific embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the level of proAdrenomedullin (proADM) and/or fragments thereof having at least 5 amino acids in a sample of bodily fluid of said patient is above a certain threshold, wherein said sample of bodily fluid is selected from the group comprising plasma, serum, blood.

In the present application, the fragments of proADM may be mature Adrenomedullin (ADM-NH₂ (SEQ ID No. 4)), ADM 1-52-Gly (SEQ ID No. 5), MR-proADM (SEQ ID No. 3) and/or CT-proADM (SEQ ID No. 6).

The peptide adrenomedullin (ADM) was described for the first time in Kitamura et al. *(*Kitamura et al. 1993. Biochemical and Biophysical Research Communications 192 (2): 553-560*)* as a novel hypotensive peptide comprising 52 amino acids, which had been isolated from a human pheochromocytoma. In the same year, cDNA coding for a precursor peptide comprising 185 amino acids and the complete amino acid sequence of this precursor peptide were also described. The precursor peptide, which comprises, inter alia, a signal sequence of 21 amino acids at theN-terminus, is referred to as "preproadrenomedullin" (pre-proADM). Pre-proADM comprises 185 amino acids. The mature ADM-NH₂ is displayed in SEQ ID No. 4 and ADM-Gly is displayed in SEQ No. 5, MR-proADM is displayed in SEQ ID No. 3 and/or CT-proADM is displayed in SEQ ID No. 6.

The mature adrenomedullin peptide is an amidated peptide (ADM-NH₂), which comprises 52 amino acids (SEQ ID No: 4) and which comprises the amino acids 95 to 146 of pre-proADM, from which it is formed by proteolytic cleavage. To date, substantially only a few fragments of the peptide fragments formed in the cleavage of the pre-proADM have been more exactly characterized, in particular the physiologically active peptides adrenomedullin (ADM) and "PAMP", a peptide comprising 20 amino acids (22-41) which follows the 21 amino acids of the signal peptide in pre-proADM. Furthermore, for both, ADM and PAMP, physiologically active sub-fragments were discovered and investigated in more detail. The discovery and characterization of ADM in 1993 triggered intensive research activity and a flood of publications, the results of which have recently been summarized in various review articles, in the context of the present description, reference being made in particular to the articles Takahashi 2001. Peptides 22: 1691*;* Eto et al. 2001. Peptides 22: 1693-1711 *and* Hinson et al. 2000 Endocrine Reviews 21(2):138-167*.*

In the scientific investigations to date, it has been found, inter alia, that ADM may be regarded as a polyfunctional regulatory peptide. It is released into the circulation partially in an inactive form extended by glycine (Kitamura et al. 1998. Biochem. Biophys. Res. Commun. 244(2): 551-555). There is also a binding protein (Pio et al. 2001. The Journal of Biological Chemistry 276(15): 12292-12300*),* which is specific for ADM and probably likewise modulates the effect of ADM.

Those physiological effects of ADM as well as of PAMP, which are of primary importance in the investigations to date, were the effects influencing blood pressure. Thus, ADM is an effective vasodilator.

It has furthermore been found that the above-mentioned further physiologically active peptide PAMP formed from pre-proADM likewise exhibits a hypotensive effect, even if it appears to have an action mechanism differing from that of ADM (Eto et al. 2001. Peptides 22: 1693-1711*;* Hinson et al. 2000 Endocrine Reviews 21(2):138-167*;* Kuwasako et al. 1997. FEBS Lett 414(1): 105-110*;* Kuwasaki et al. 1999. Ann. Clin. Biochem. 36: 622-628*;* Tsuruda et al. 2001. Life Sci. 69(2): 239-245*;* Kangawa et al. EP 0 622 458*).*

Furthermore, it was found that the concentrations of ADM, which can be measured in the circulation and other biological fluids, are in a number of pathological states, significantly above the concentrations to be found in healthy control persons. Thus, the ADM level in patients with congestive heart failure, myocardial infarction, kidney diseases, hypertensive disorders, diabetes mellitus, in the acute phase of shock and in sepsis and septic shock are significantly increased, although to different extents. The PAMP concentrations are also increased in some of said pathological states, but the plasma levels are reduced relative to ADM (Eto et al. 2001. Peptides 22: 1693-1711*).*

Furthermore, it is known that unusual high concentrations of ADM are to be observed in sepsis or in septic shock (Eto et al. 2001. Peptides 22: 1693-1711*;* Hirata et al. 1996. Journal of Clinical Endocrinology and Metabolism 81(4): 1449-1453*;* Ehlenz et al.1997. Exp Clin Endocrinol Diabetes 105: 156-162*;* Tomoda et al. 2001. Peptides 22: 1783-1794*;* Ueda et al. 1999 Am. J. Respir. Crit. Care Med. 160: 132-136*;* Wang et al. 2001. Peptides 22: 1835-1840*).* The findings are related to the typical hemodynamic changes which are known as typical phenomena of the course of a disease in patients with sepsis and other severe syndromes, such as, for example, SIRS. Adrenomedullin plays pivotal roles during sepsis development (Wang, Shock 1998, 10(5):383-384*;* Wang et al. 1998. Archives of surgery 133(12): 1298-1304*)* and in numerous acute and chronic diseases (Parlapiano et al. 1999. European Review for Medical and Pharmacological Sciences 3:53-61*;* Hinson etal. 2000 Endocrine Reviews 21(2):138-167).

Several methods were described to measure circulating levels of ADM: either ADM directly or indirectly by determining a more stable fragment of its cognate precursor peptide. Recently a method was published, describing an assay to measure circulating mature ADM (Marino et al. 2014. Crit Care 18: R34*).*

Other methods to quantify fragments derived from the ADM precursor have been described, e.g. the measurement of MR-proADM (Morgenthaler et al. 2005. Clin Chem 51(10):1823-9), PAMP (Washimine et al. 1994. Biochem Biophys Res Commun 202(2):1081-7) and CT-proADM (EP 2 111 552)*.* A commercial homogeneous time-resolved fluoroimmunoassay for the measurement of MR-proADM in plasma on a fully automated system is available (BRAHMS MR-proADM KRYPTOR; BRAHMS GmbH, Hennigsdorf, Germany) (Caruhel et al. 2009. Clin Biochem 42(7-8): 725-8*).* As these peptides are generated in a stoichiometric ratio from the same precursor, their plasma levels are correlated to a certain extent.

Plasma concentrations of ADM are elevated in patients with heart failure and correlate with disease severity (Hirayama et al. 1999. J Endocrinol 160: 297-303*;* Yu et al. 2001. Heart 86: 155-160). High plasma ADM is an independent negative prognostic indicator in these subjects *(*Poyner et al. 2002. Pharmacol Rev 54: 233-246).

The role of MR-proADM in heart failure was explored in several studies. In the BACH study (Maisel et al. 2010. J. Am. Coll. Cardiol. 55: 2062-2076), MR-proADM was powerfully prognostic for death at 90 days, adding prognostic value beyond natriuretic peptides. Subsequent data from the PRIDE study (Shah et al. 2012. Eur. Heart J. 33: 2197-2205) solidified a potential prognostic role for MR-proADM; among patients MR-proADM had the best area under the curve (AUC) for mortality at 1 year. Similarly, levels of MR-proADM in patients with chronic heart failure (CHF) were strongly correlated with disease severity and elevated levels of the peptide were strongly associated with an increased risk of death at 12 months of follow-up (van Haehling et al. 2010. European Journal of Heart Failure 12: 484-491*;* Adlbrecht et al. 2009. European Journal of Heart Failure 11: 361-366)*.*

MR-proADM was investigated during treatment in patients with acute decompensated heart failure (Boyer et al. 2012. Congest Heart Fail 18 (2): 91-97): patients whose MR-proADM levels tended to increase during acute therapy had findings associated with persistent congestion. In the 12-24 hour time-period after therapy, patients with elevations of MR-proADM had increased peripheral edema. Kaiser et al. measured MR-proADM in patients with univentricular hearts (Kaiser et al. 2014. Europ J Heart Failure 16: 1082-1088)*.* Levels in patients with a failed Fontan circuit (exhibiting ascites and peripheral edema) were significantly higher as compared to patients without Fontan failure. Moreover, Eisenhut speculated whether treatments leading to a reduction of adrenomedullin levels can reduce the severity and extent of alveolar edema in pneumonia and septicemia (Eisenhut 2006. Crit Care 10: 418)

Therapy or intervention of congestion in a subject having acute heart failure and/or a subject having heart failure presenting with worsening signs and/or a subject with symptoms of heart failure or acute heart failure may be selected from the group comprising administration of diuretics, administration of inotropes, administration of vasodilators, ultrafiltration, in particular diuretics.

Pro-Adrenomedullin or fragments thereof are an early, quantitative and accurate surrogate for congestion in the setting of acute heart failure and heart failure, in particular in a subject having acute heart failure and/or a subject having heart failure presenting with worsening signs and/or a subject with symptoms of heart failure or acute heart failure. Early and accurate surrogate for congestion in the setting of acute heart failure or heart failure means that their concentration and/or level of immune reactivity reflects the extent of congestion.

Previously, amongst others the markers BNP and proBNP have been considered as congestion marker. Surprisingly the biomarker Pro-Adrenomedullin or the respective fragments of the present invention are far superior than any other clinical or biochemical parameter used so far as congestion marker. This has been shown e.g. by Kremer et al. European Journal of Heart Failure (2017) 19 (Suppl SI) 5-601, Kremer et al. studied the potential role of bio-ADM, a biomarker representing biologically active adrenomedullin, as a potential marker of congestion in ADHF, and evaluated added prognostic value on top of clinically assessed congestion. Bio-ADM was evaluated at baseline, days 2 and 7 in 1562 patients admitted for ADHF. Clinical congestion was assessed using a composite clinical congestion score (CCS) encompassing edema, orthopnea and jugular venous distension. This has been compared to other clinical and biochemical parameter s, including BNP. Amongst a large number of clinical and biochemical parameters, bio-ADM was the strongest predictor of clinically assessed congestion. Baseline bio-ADM levels, but not BNP levels, were strongly and independently associated with presence of significant residual congestion by day 7. In patients with residual congestion at day 7, bio-ADM levels remained high, while BNP levels decreased. Bio-ADM, but not BNP, was a strong independent predictor of 60-day heart failure rehospitalization, on top of clinically assessed congestion. Thus, the authors concluded that Bio-ADM is elevated in patients with ADHF and is a promising marker of (residual) congestion in patients admitted for ADHF. Bio-ADM was a strong and independent predictor of rehospitalization early after discharge.

The term "acute" is used to mean rapid onset and to describe exacerbated or decompensated heart failure, referring to episodes in which a patient can be characterized as having a change in heart failure signs and symptoms resulting in a need for urgent therapy or hospitalization.

The term "chronic" refers to long duration. Chronic heart failure is a long-term condition, usually kept stable by the treatment of symptoms (stable chronic HF).

Stable chronic HF is characterized by:
(i) the presence of structural or functional failure of the heart that impairs its ability to supply sufficient blood flow to meet body's needs,
(ii) the absence of volume overload (manifested by pulmonary and/ or systemic congestion) and/ or profound depression of cardiac output (manifested by hypotension, renal insufficiency and/ or a shock syndrome),
and whereas the patient is not in need of urgent therapy or therapy adjustment and does not require hospitalization.

Chronic HF with worsening signs and symptoms is characterized by:
(i) the presence of structural or functional failure of the heart that impairs its ability to supply sufficient blood flow to meet body's needs,
(ii) volume overload (manifested by pulmonary and/ or systemic congestion) and/ or profound depression of cardiac output (manifested by hypotension, renal insufficiency and/ or a shock syndrome)
and whereas the patient is not in need of urgent therapy and does not require hospitalization, but is in need of therapy adjustment.

Chronic heart failure may also decompensate (termed acute decompensated heart failure or acute decompensated chronic heart failure), which is most commonly the result from an intercurrent illness (such as pneumonia), myocardial infarction, arrhythmias, uncontrolled hypertension or a patient's failure to maintain fluid restriction, diet or medication. After treatment, patients with acute decompensated chronic HF may return to a stable chronic compensated status (stable chronic HF).

New onset acute HF and acute decompensated chronic HF are characterized by:
(i) the presence of structural or functional failure of the heart that impairs its ability to supply sufficient blood flow to meet body's needs,
(ii) volume overload (manifested by pulmonary and/ or systemic congestion) and/ or profound depression of cardiac output (manifested by hypotension, renal insufficiency and/ or a shock syndrome)
and whereas the patient is in need of urgent therapy or therapy adjustment and does require hospitalization.

| **Acute HF** | | **Chronic HF** | | |
|---|---|---|---|---|
| New-onset AHF | Acute decompensated HF = acute decompensated chronic HF | | Worsening signs/ symptoms of chronic HF | Stable chronic HF |

The above definitions of acute heart failure that either new-onset AHF or acute decompensated HF or acute decompensated chronic HF or worsening signs/symptoms of chronic heart failure are in line with Voors et al., European Journal of Heart Failure (2016), 18, 716 - 726.

Congestion in HF is defined as a high left ventricular diastolic pressure associated with signs and symptoms of HF such as dyspnea, rales, and/or edema. These signs and symptoms related to congestion are the main reasons for HF-related hospitalizations.

Although relief of congestion (and associated signs/symptoms) and attainment of euvolemic status remain the main goal of inhospital AHF therapy, there is no standard algorithm or clinical tool for the assessment of congestion. Current practices regarding the clinical evaluation of congestion revolve around signs and symptoms. Physical examination findings such as elevated jugular venous pressure (JVP), peripheral edema, orthopnea, S3 heart sound and hepatomegaly or chest X-ray findings like cardiomegaly and interstitial/alveolar edema are used as surrogates for congestion. It must be noted that, besides a carefully performed JVP assessment, the predictive value of these parameters for detecting congestion is modest to moderate. There is a high unmet need to have a reliable and accurate surrogate marker for congestion. There is a high and unmet medical need to determining, predicting, assessing and/or monitoring congestion and decongestion in a quantitative and qualitative manner. There is the need to determining, predicting, assessing and/or monitoring the extent of congestion, i.e. the grade of congestion.

For the present application the extent of congestion may be expressed as grade severity of congestion and has been determined as described below. However, the person skilled in the art knows that the extent of congestion may be expressed by other scores or surrogates, such as for instance the score utilized by Ambrosy et al. (Ambrosy et al. 2013. European Heart Journal 34 (11): 835-843*).*

As highlighted previously, clinical surrogates have less than optimal predictive value for the detection of congestion. In the application WO2018/007588, it is described in the analysis of one of the examples shown in the application (PROTECT study), that they combined three of the strongest clinical surrogates of congestion (i.e. JVP, peripheral edema and orthopnea) to enhance accuracy and developed a composite clinical congestion score (CCS) using the scheme presented below:

| | | | | |
|---|---|---|---|---|
| Parameter | 0 | 1 | 2 | 3 |
| Peripheral edema | 0 | Ankle | Below knee | Above knee |
| Orthopnea | 0 pillow | 1 pillow | 2 pillows | 3 pillows |
| JVP | <6cm | 6-10cm | >10cm | - |

The score on each of these three parameters were then added to obtain a composite congestion score, which ranged from 0 to 8.

The following algorithm was then utilized to grade severity of congestion:
CCS=0, No clinical congestion
CCS 1-3, Mild clinical congestion
CCS 4-5, Moderate clinical congestion
CCS ≥6, Severe clinical congestion

As above explained, congestion can be classified in many different ways. The person skilled in the art knows that the extent of congestion may be expressed by other scores or surrogates. Clinical classification can be based on bedside physical examination in order to detect the presence of clinical symptoms/signs of congestion ('wet' vs. 'dry' if present vs. absent) and/or peripheral hypoperfusion ('cold' vs. 'warm' if present vs. absent) (for review see Ponikowski et al. 2016. Eur Heart J. ehw128). The combination of these options identifies four groups: warm and wet (well perfused and congested) - most commonly present; cold and wet (hypoperfused and congested); cold and dry (hypoperfused without congestion); and warm and dry (compensated, well perfused without congestion). This classification may be helpful to guide therapy in the initial phase and carries prognostic information.

Typically, symptoms and signs of AHF reflect fluid overload (pulmonary congestion and/or peripheral edema) or, less often, reduced cardiac output with peripheral hypoperfusion. Chest X-ray can be a useful test for the diagnosis of AHF. Pulmonary venous congestion, pleural effusion, interstitial or alveolar edema and cardiomegaly are the most specific findings for AHF, although in up to 20% of patients with AHF, chest X-ray is nearly normal.

Symptoms/signs of congestion (left-sided) are defined as orthopnoea, paroxysmal nocturnal dyspnoea, pulmonary rales (bilateral), peripheral edema (bilateral). Symptoms/signs of congestion (right-sided) are defined as jugular venous dilatation, peripheral edema (bilateral), congested hepatomegaly, hepatojugular reflux, ascites, symptoms of gut congestion (for review see table 12.2 in Ponikowski et al. 2016. Eur Heart J. ehw128).

Edema is an accumulation of fluid in the intercellular tissue that results from an abnormal expansion in interstitial fluid volume. The fluid between the interstitial and intravascular spaces is regulated by the capillary hydrostatic pressure gradient and the oncotic pressure gradient across the capillary (Trayes et al. 2013. Am Fam Physician 88(2):102-110). The accumulation of fluid occurs when local or systemic conditions disrupt this equilibrium, leading to increased capillary hydrostatic pressure, increased plasma volume, decreased plasma oncotic pressure (hypoalbuminemia), increased capillary permeability, or lymphatic obstruction.

Clinically, edema manifests as swelling: the amount of interstitial fluid is determined by the balance of fluid homeostasis, and the increased secretion of fluid into the interstitium, or the impaired removal of the fluid can cause edema. A rise in hydrostatic pressure occurs in cardiac failure. Causes of edema which are generalized to the whole body can cause edema in multiple organs and peripherally. For example, severe heart failure can cause pulmonary edema, pleural effusions, ascites and peripheral edema.

Pulmonary edema is fluid accumulation in the air spaces and parenchyma of the lungs. It leads to impaired gas exchange and may cause respiratory failure. It is due to either failure of the left ventricle of the heart to adequately remove blood from the pulmonary circulation ("cardiogenic pulmonary edema"), or an injury to the lung parenchyma or vasculature of the lung ("noncardiogenic pulmonary edema") (Ware and Matthay 2005. N. Engl. J. Med. 353 (26): 2788-96). Treatment is focused on three aspects: firstly improving respiratory function, secondly, treating the underlying cause, and thirdly avoiding further damage to the lung. Pulmonary edema, especially acute, can lead to fatal respiratory distress or cardiac arrest due to hypoxia. It is a cardinal feature of congestive heart failure.

The overwhelming symptom of pulmonary edema is difficulty breathing, but may also include coughing up blood (classically seen as pink, frothy sputum), excessive sweating, anxiety, and pale skin. Shortness of breath can manifest as orthopnea (inability to lie down flat due to breathlessness) and/or paroxysmal nocturnal dyspnea (episodes of severe sudden breathlessness at night). These are common presenting symptoms of chronic pulmonary edema due to left ventricular failure. The development of pulmonary edema may be associated with symptoms and signs of "fluid overload"; this is a non specific term to describe the manifestations of left ventricular failure on the rest of the body and includes peripheral edema (swelling of the legs, in general, of the "pitting" variety, wherein the skin is slow to return to normal when pressed upon), raised jugular venous pressure and hepatomegaly, where the liver is enlarged and may be tender or even pulsatile. Other signs include end-inspiratory crackles (sounds heard at the end of a deep breath) on auscultation and the presence of a third heart sound.

It is already known that Pro-Adrenomedullin or fragments thereof is associated with the severity of congestion and extent of edema, predicts decongestion and the applied dose of diuretics.

In a specific embodiment of the application the level of proADM and/or fragments thereof is determined by using at least one binder selected from the group: a binder that binds to a region comprised within the following sequence of mature ADM-NH₂ (SEQ ID No. 4) and/or ADM 1-52-Gly (SEQ ID No. 5) and a second binder that binds to a region comprised within the sequence of mature ADM-NH₂ (SEQ ID NO. 4) and/or ADM 1-52-Gly (SEQ ID No. 5).

In a specific embodiment of the application the level of proADM and/or fragments thereof is determined by using at least one binder selected from the group: a binder that binds to a region comprised within the sequence of MR-proADM (SEQ ID No. 3) and a second binder that binds to a region comprised within the sequence of MR-proADM (SEQ ID No. 3).

In a specific embodiment of the application the level of pro-ADM and/or fragments thereof is determined by using at least one binder selected from the group: a binder that binds to a region comprised within the sequence of CT-proADM (SEQ ID No. 6) and a second binder that binds to a region comprised within the sequence of CT-pro-ADM (SEQ ID No. 6).

In a particular embodiment of the present application said fragment may be selected from MR-proADM according to SEQ ID No.: 3 or mature ADM-NH₂ according to SEQ ID No.: 4.

In another embodiment of the present application, the level of Pro-Adrenomedullin or fragments thereof of at least 5 amino acids is determined by using a binder to Pro-Adrenomedullin or fragments thereof of at least 5 amino acids.

In another embodiment of the present application, the binder is selected from the group comprising an antibody, an antibody fragment or a non-Ig-Scaffold binding to Pro-Adrenomedullin or fragments thereof of at least 5 amino acids.

A bodily fluid according to the present application is in one particular embodiment a blood sample. A blood sample may be selected from the group comprising whole blood, serum and plasma. In a specific embodiment of the invention said sample is selected from the group comprising human citrate plasma, heparin plasma and EDTA plasma.

In another embodiment of the present application, said determination of Pro-Adrenomedullin or fragments thereof of at least 5 amino acids is performed more than once in one patient.

In another embodiment of the present application, the sample is taken on admission to a hospital or prior discharge of the hospital.

In a specific embodiment of the application, an assay is used for determining the level of proADM and/or fragments thereof having at least 5 amino acids, wherein the assay sensitivity of said assay is able to quantify the mature ADM-NH₂ of healthy subjects and is < 70 pg/ml, preferably < 40 pg/ml and more preferably < 10 pg/ml.

In a specific embodiment of the application, an assay is used for determining the level of proADM and/or fragments thereof having at least 5 amino acids, wherein the assay sensitivity of said assay is able to quantify MR-proADM of healthy subjects and is < 0.5 nmol/L, preferably < 0.4 nmol/L and more preferably < 0.2 nmol/L.

In a specific embodiment of the application, an assay is used for determining the level of proADM and/or fragments thereof having at least 5 amino acids, wherein the assay sensitivity of said assay is able to quantify CT-proADM of healthy subjects and is < 100 pmol/L, preferably < 75 pmol/L and more preferably < 50 pmol/L.

In a specific embodiment of the application, said binder exhibits a binding affinity to proADM and/or fragments thereof of at least 10⁷ M⁻¹, preferred 10⁸ M⁻¹, preferred affinity is greater than 10⁹ M⁻¹, most preferred greater than 10¹⁰ M⁻¹. A person skilled in the art knows that it may be considered to compensate lower affinity by applying a higher dose of compounds and this measure would not lead out-of-the-scope of the invention.

To determine the affinity of the antibodies to Adrenomedullin, the kinetics of binding of Adrenomedullin to immobilized antibody was determined as described in the application WO2018/007588 by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare), (Lorenz et al. 2011. Antimicrob Agents Chemother. 55 (1): 165-173).

In a specific embodiment of the application, said binder is selected from the group comprising an antibody or an antibody fragment or a non-Ig scaffold binding to proADM and/or fragments thereof.

In a specific embodiment of the application, an assay is used for determining the level of proADM and/or fragments thereof having at least 5 amino acids, wherein such assay is a sandwich assay, preferably a fully automated assay.

In one embodiment of the application it may be a so-called POC-test (point-of-care) that is a test technology, which allows performing the test within less than 1 hour near the patient without the requirement of a fully automated assay system. One example for this technology is the immunochromatographic test technology.

In one embodiment of the application such an assay is a sandwich immunoassay using any kind of detection technology including but not restricted to enzyme label, chemiluminescence label, electrochemiluminescence label, preferably a fully automated assay. In one embodiment of the invention such an assay is an enzyme labeled sandwich assay. Examples of automated or fully automated assay comprise assays that may be used for one of the following systems: Roche Elecsys^{®}, Abbott Architect^{®}, Siemens Centauer^{®}, Brahms Kryptor^{®}, BiomerieuxVidas^{®}, Alere Triage^{®}.

A variety of immunoassays are known and may be used for the assays and methods of the present invention, these include: radioimmunoassays ("RIA"), homogeneous enzyme-multiplied immunoassays ("EMIT"), enzyme linked immunoadsorbent assays ("ELISA"), apoenzyme reactivation immunoassay ("ARIS"), dipstick immunoassays and immuno-chromotography assays.

In a specific embodiment of the application, at least one of said two binders is labeled in order to be detected.

The preferred detection methods comprise immunoassays in various formats such as for instance radioimmunoassay (RIA), chemiluminescence- and fluorescence-immunoassays, Enzyme-linked immunoassays (ELISA), Luminex-based bead arrays, protein microarray assays, and rapid test formats such as for instance immunochromatographic strip tests.

In a preferred embodiment, said label is selected from the group comprising chemiluminescent label, enzyme label, fluorescence label, radioiodine label.

The assays can be homogenous or heterogeneous assays, competitive and non-competitive assays. In one embodiment, the assay is in the form of a sandwich assay, which is a non-competitive immunoassay, wherein the molecule to be detected and/or quantified is bound to a first antibody and to a second antibody. The first antibody may be bound to a solid phase, *e.g.* a bead, a surface of a well or other container, a chip or a strip, and the second antibody is an antibody which is labeled, *e.g*. with a dye, with a radioisotope, or a reactive or catalytically active moiety. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005*);* Hultschig et al. 2006. Curr Opin Chem Biol. 10 (1):4-10*).*

In another embodiment the assay comprises two capture molecules, preferably antibodies which are both present as dispersions in a liquid reaction mixture, wherein a first labelling component is attached to the first capture molecule, wherein said first labelling component is part of a labelling system based on fluorescence- or chemiluminescence-quenching or amplification, and a second labelling component of said marking system is attached to the second capture molecule, so that upon binding of both capture molecules to the analyte a measurable signal is generated that allows for the detection of the formed sandwich complexes in the solution comprising the sample.

In another embodiment, said labeling system comprises rare earth cryptates or rare earth chelates in combination with fluorescence dye or chemiluminescence dye, in particular a dye of the cyanine type.

In the context of the present application, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present application, chemiluminescence based assays comprise the use of dyes, based on the physical principles described for chemiluminescent materials in (Kirk-Othmer, Encyclopedia of chemical technology, 4th ed. 1993. John Wiley & Sons, Vol. 15: 518-562*, incorporated herein by reference, including citations on pages 551-562*). Preferred chemiluminescent dyes are acridinium esters.

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M⁻¹.

In the context of the present application, "binder molecules" are molecules which may be used to bind target molecules or molecules of interest, *i.e.* analytes (*i.e.* in the context of the present invention ADM-NH₂ and/ or proADM and fragments thereof), from a sample. Binder molecules have thus to be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, binder molecules may for instance be selected from the group comprising a nucleic acid molecule, a carbohydrate molecule, a PNA molecule, a protein, an antibody, a peptide or a glycoprotein. Preferably, the binder molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof.

Chemiluminescent label may be acridinium ester label, steroid labels involving isoluminol labels and the like.

Enzyme labels may be lactate dehydrogenase (LDH), creatine kinase (CPK), alkaline phosphatase, aspartate aminotransferase (AST), alanine aminotransferase (ALT), acid phosphatase, glucose-6-phosphate dehydrogenase and so on.

In one embodiment of the applicatio at least one of said two binders is bound to a solid phase as magnetic particles, and polystyrene surfaces.

In a specific embodiment of the application at least one of said two binders is bound to a solid phase.

For the sake of practicability the person skilled in the art may use threshold(s).

In a specific embodiment of the application the threshold is within a threshold range for plasma ADM-NH₂ that is between 50 and 100 pg/ml, preferably between 60 and 90 pg/ml, most preferred a threshold of 70 pg/ml is applied.

In a specific embodiment of the application the threshold is within a threshold range for plasma MR-proADM that is between 0.5 and 1.5 nmol/L, preferably between 0.7 and 1 nmol/L, most preferred a threshold of 0.8 nmol/L is applied.

In a specific embodiment of the application the threshold is within a threshold range for plasma CT-proADM that is between 85 and 350 pmol/L, preferably between 100 and 250 pmol/L, most preferred a threshold of 150 pmol/L is applied.

The ADM-NH₂ levels of the present application or proADM levels or fragments thereof, respectively, have been determined with the described ADM-NH₂ assay, as described in the application WO2018/007588 (or proADM or fragments thereof assays, respectively). The mentioned threshold values above might be different in other assays, if these have been calibrated differently from the assay systems used in the present invention. Therefore, the mentioned cut-off values above shall apply for such differently calibrated assays accordingly, taking into account the differences in calibration. One possibility of quantifying the difference in calibration is a method comparison analysis (correlation) of the assay in question with the respective biomarker assay used in the present invention by measuring the respective biomarker (e.g. bio-ADM) in samples using both methods. Another possibility is to determine with the assay in question, given this test has sufficient analytical sensitivity, the median biomarker level of a representative normal population, compare results with the median biomarker levels as described in the literature and recalculate the calibration based on the difference obtained by this comparison. With the calibration used in the application WO2018/007588, samples from normal (healthy) subjects have been measured: median plasma bio-ADM (mature ADM-NH₂) was 24.7 pg/ml, the lowest value 11 pg/ml and the 99^{th} percentile 43 pg/ml. Alternatively, commercially available control samples could be used for adjustment of different calibrations (e.g. ICI Diagnostics, Berlin, Germany).

In the application WO2018/007588, the plasma median MR-proADM concentration in normal (healthy) subjects was 0.41 (interquartile range 0.23 - 0.64) nmol/L (Smith et al. 2009. Clin Chem 55:1593-1595*)* using the automated sandwich fluorescence assay for the detection of MR-proADM as described in Caruhel et al. (Caruhel et al. 2009. Clin Biochem 42: 725-8)*.*

The plasma median concentration of CT-proADM in normal healthy subjects (n=200) was 77.6 pmol/L (min 46.6 pmol/L, max 136.2 pmol/L) and the 95% percentile was 113.8 pmol/L (EP 2 111 552 B1).

An antibody according to the present invention is a protein including one or more polypeptides substantially encoded by immunoglobulin genes that specifically binds an antigen. The recognized immunoglobulin genes include the kappa, lambda, alpha (IgA), gamma (IgGi, IgG₂, IgG₃, IgG₄), delta (IgD), epsilon (IgE) and mu (IgM) constant region genes, as well as the myriad immunoglobulin variable region genes. Full-length immunoglobulin light chains are generally about 25 Kd or 214 amino acids in length. Full-length immunoglobulin heavy chains are generally about 50 Kd or 446 amino acid in length. Light chains are encoded by a variable region gene at the NH2-terminus (about 110 amino acids in length) and a kappa or lambda constant region gene at the COOH-terminus. Heavy chains are similarly encoded by a variable region gene (about 116 amino acids in length) and one of the other constant region genes.

The basic structural unit of an antibody is generally a tetramer that consists of two identical pairs of immunoglobulin chains, each pair having one light and one heavy chain. In each pair, the light and heavy chain variable regions bind to an antigen, and the constant regions mediate effector functions. Immunoglobulins also exist in a variety of other forms including, for example, Fv, Fab, and (Fab')₂, as well as bifunctional hybrid antibodies and single chains (*e.g*., Lanzavecchia et al. 1987. Eur. J. Immunol. 17:105*;* Huston et al. 1988, Proc. Natl. Acad. Sci. U.S.A., 85:5879-5883*;* Bird et al. 1988, Science 242:423-426*;* Hood et al., Immunology, Benjamin, N.Y., 2nd ed., 1984*;* Hunkapiller and Hood 1986. Nature 323:15-16). An immunoglobulin light or heavy chain variable region includes a framework region interrupted by three hypervariable regions, also called complementarity determining regions (CDR's) (see, Sequences of Proteins of Immunological Interest, E. Kabatet al., U.S. Department of Health and Human Services, 1983*).* As noted above, the CDRs are primarily responsible for binding to an epitope of an antigen. An immune complex is an antibody, such as a monoclonal antibody, chimeric antibody, humanized antibody or human antibody, or functional antibody fragment, specifically bound to the antigen.

Chimeric antibodies are antibodies whose light and heavy chain genes have been constructed, typically by genetic engineering, from immunoglobulin variable and constant region genes belonging to different species. For example, the variable segments of the genes from a mouse monoclonal antibody can be joined to human constant segments, such as kappa and gamma 1 or gamma 3. In one example, a therapeutic chimeric antibody is thus a hybrid protein composed of the variable or antigen-binding domain from a mouse antibody and the constant or effector domain from a human antibody, although other mammalian species can be used, or the variable region can be produced by molecular techniques. Methods of making chimeric antibodies are well known in the art (*e.g.,* see U.S. Patent No. 5,807,715)*.* A "humanized" immunoglobulin is an immunoglobulin including a human framework region and one or more CDRs from a non-human (such as a mouse, rat, or synthetic) immunoglobulin. The non-human immunoglobulin providing the CDRs is termed a "donor" and the human immunoglobulin providing the framework is termed an "acceptor." In one embodiment, all the CDRs are from the donor immunoglobulin in a humanized immunoglobulin. Constant regions need not be present, but if they are, they must be substantially identical to human immunoglobulin constant regions, *i.e.,* at least about 85-90%, such as about 95% or more identical. Hence, all parts of a humanized immunoglobulin, except possibly the CDRs, are substantially identical to corresponding parts of natural human immunoglobulin sequences. A "humanized antibody" is an antibody comprising a humanized light chain and a humanized heavy chain immunoglobulin. A humanized antibody binds to the same antigen as the donor antibody that provides the CDRs.

The acceptor framework of a humanized immunoglobulin or antibody may have a limited number of substitutions by amino acids taken from the donor framework. Humanized or other monoclonal antibodies can have additional conservative amino acid substitutions, which have substantially no effect on antigen binding or other immunoglobulin functions. Exemplary conservative substitutions are those such as gly, ala; val, ile, leu; asp, glu; asn, gln; ser, thr; lys, arg; and phe, tyr. Humanized immunoglobulins can be constructed by means of genetic engineering (*e.g.,* see U.S. Patent No. 5,585,089)*.* A human antibody is an antibody wherein the light and heavy chain genes are of human origin. Human antibodies can be generated using methods known in the art. Human antibodies can be produced by immortalizing a human B cell secreting the antibody of interest. Immortalization can be accomplished, for example, by EBV infection or by fusing a human B cell with a myeloma or hybridoma cell to produce a trioma cell. Human antibodies can also be produced by phage display methods (see, *e.g.,* Dower et al., PCT Publication No. WO91/17271; McCafferty et al., PCT Publication No. WO92/001047; and Winter, PCT Publication No. WO92/20791), or selected from a human combinatorial monoclonal antibody library (see the Morphosys website). Human antibodies can also be prepared by using transgenic animals carrying a human immunoglobulin gene (for example, see Lonberget al., PCT Publication No. WO93/12227; and Kucherlapati, PCT Publication No. WO91/10741).

Thus, the antibody may have the formats known in the art. In the application WO2018/007588, examples are human antibodies, monoclonal antibodies, humanized antibodies, chimeric antibodies, CDR-grafted antibodies. In a preferred embodiment antibodies are recombinantly produced antibodies as *e.g.* IgG, a typical full-length immunoglobulin, or antibody fragments containing at least the F-variable domain of heavy and/or light chain as *e.g.* chemically coupled antibodies (fragment antigen binding) including but not limited to Fab-fragments including Fab minibodies, single chain Fab antibody, monovalent Fab antibody with epitope tags, *e.g.* Fab-V5Sx2; bivalent Fab (mini-antibody) dimerized with the CH3 domain; bivalent Fab or multivalent Fab, *e.g.* formed via multimerization with the aid of a heterologous domain, *e.g.* via dimerization of dHLXdomains, *e.g.* Fab-dHLX-FSx2; F(ab')2-fragments, scFv-fragments, multimerized multivalent or/and multispecificscFv-fragments, bivalent and/or bispecificdiabodies, BITE^{®} (bispecific T-cell engager), trifunctional antibodies, polyvalent antibodies, *e.g.* from a different class than G; single-domain antibodies, *e.g.* nanobodies derived from camelid or fish immunoglobulines and numerous others.

In addition to antibodies other biopolymer scaffolds are well known in the art to complex a target molecule and have been used for the generation of highly target specific biopolymers. Examples are aptamers, spiegelmers, anticalins and conotoxins.

In a preferred embodiment the antibody format is selected from the group comprising Fv fragment, scFv fragment, Fab fragment, scFab fragment, (Fab)2 fragment and scFv-Fc Fusion protein. In another preferred embodiment the antibody format is selected from the group comprising scFab fragment, Fab fragment, scFv fragment and bioavailability optimized conjugates thereof, such as PEGylated fragments. One of the most preferred formats is the scFab format.

Non-Ig scaffolds may be protein scaffolds and may be used as antibody mimics as they are capable to bind to ligands or antigenes. Non-Ig scaffolds may be selected from the group comprising tetranectin-based non-Ig scaffolds *(e.g.* described in US 2010/0028995), fibronectin scaffolds (e.g. described in EP 1 266 025), lipocalin-based scaffolds (e.g. described in WO 2011/154420); ubiquitin scaffolds (e.g. described in WO 2011/073214), transferring scaffolds (e.g. described in US 2004/0023334), protein A scaffolds (e.g. described in EP 2 231 860), ankyrin repeat based scaffolds (e.g. described in WO 2010/060748), microprotein scaffolds (preferably microproteins forming a cystine knot) (e.g. described in EP 2 314 308), Fyn SH3 domain based scaffolds (e.g. described in WO 2011/023685) EGFR-A-domain based scaffolds (e.g. described in WO 2005/040229) and Kunitz domain based scaffolds (e.g. described in EP 1941867).

In one embodiment of the invention antibodies according to the present invention may be produced as described in the application WO2018/007588 as follows:
A Balb/c mouse was immunized with ADM-100µg Peptide-BSA-Conjugate at day 0 and 14 (emulsified in 100µl complete Freund's adjuvant) and 50µg at day 21 and 28 (in 100µl incomplete Freund's adjuvant). Three days before the fusion experiment was performed, the animal received 50µg of the conjugate dissolved in 100µl saline, given as one intraperitoneal and one intravenous injection.

Splenocytes from the immunized mouse and cells of the myeloma cell line SP2/0 were fused with 1ml 50% polyethylene glycol for 30s at 37°C. After washing, the cells were seeded in 96-well cell culture plates. Hybrid clones were selected by growing in HAT medium [RPMI 1640 culture medium supplemented with 20% fetal calf serum and HAT-Supplement]. After two weeks the HAT medium is replaced with HT Medium for three passages followed by returning to the normal cell culture medium.

The cell culture supernatants were primary screened for antigen specific IgG antibodies three weeks after fusion. The positive tested microcultures were transferred into 24-well plates for propagation. After retesting, the selected cultures were cloned and recloned using the limiting-dilution technique and the isotypes were determined (see also Lane 1985. J. Immunol. Meth. 81: 223-228*;* Ziegler, B. et al. 1996 Horm. Metab. Res. 28: 11-15).

In the application WO2018/007588, antibodies may be produced by means of phage display according to the following procedure:
The human naive antibody gene libraries HAL7/8 were used for the isolation of recombinant single chain F-Variable domains (scFv) against adrenomedullin peptide. The antibody gene libraries were screened with a panning strategy comprising the use of peptides containing a biotin tag linked via two different spacers to the adrenomedullin peptide sequence. A mix of panning rounds using non-specifically bound antigen and streptavidin bound antigen were used to minimize background of non-specific binders. The eluted phages from the third round of panning have been used for the generation of monoclonal scFv expressing E.coli strains. Supernatant from the cultivation of these clonal strains has been directly used for an antigen ELISA testing (see Hust et al. 2011. Journal of Biotechnology 152: 159-170*;* Schütte et al. 2009. PLoS One 4, e6625)*.*

Humanization of murine antibodies may be conducted as described in the application WO2018/007588 with following procedure:
For humanization of an antibody of murine origin the antibody sequence is analyzed for the structural interaction of framework regions (FR) with the complementary determining regions (CDR) and the antigen. Based on structural modelling an appropriate FR of human origin is selected and the murine CDR sequences are transplanted into the human FR. Variations in the amino acid sequence of the CDRs or FRs may be introduced to regain structural interactions, which were abolished by the species switch for the FR sequences. This recovery of structural interactions may be achieved by random approach using phage display libraries or via directed approach guided by molecular modelling (Almagro et al. 2008. Front Biosci. 2008; 13:1619-33).

A bodily fluid according to the present invention is in one particular embodiment a blood sample. A blood sample may be selected from the group comprising whole blood, serum and plasma. In a specific embodiment of the present application said sample is selected from the group comprising human citrate plasma, heparin plasma and EDTA plasma.

Another embodiment of the present application relates to a combination medication for use in the treatment of a patient having acute and/or persistent dyspnea in a patient, wherein Pro-Adrenomedullin or fragments thereof of at least 5 amino acids is used as a marker for congestion comprising:
- Determining the level of Pro-Adrenomedullin or fragments thereof of at least 5 amino acids in a bodily fluid obtained from said subject; and
- Correlating said level of Pro-Adrenomedullin or fragments thereof with the extent of congestion in said subject or diagnosing congestion, wherein an elevated level above a certain threshold is indicative of congestion or the extent of congestion,
wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or MR-proADM according to SEQ ID No.: 4 or ADM-Gly according to SEQ ID No.: 5 or CT-proADM according to SEQ ID No.: 6, and wherein said patient has no congestion, i.e. the level of proADM and/or fragments thereof having at least 5 amino acids in a sample of bodily fluid of said patient is above a certain threshold.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said threshold is within a threshold range that is a threshold range for plasma mature ADM-NH₂ according to SEQ ID No.: 4 between 50 and 100 pg/ml and for plasma MR-proADM between 0.5 and 1.5 nmol/L, and for plasma CT-proADM between 85 and 350 pmol/L.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein an assay is used for determining the level of proADM and/or fragments thereof in a sample of bodily fluid of said patient, wherein the assay sensitivity of said assay is able to quantify the proADM of healthy subjects and is < 70 pg/ml, preferably < 40 pg/ml and more preferably < 10 pg/ml.

Another specific embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein in a sample of bodily fluid of said patient the level of mature ADM-NH2 and/or fragments thereof having at least 5 amino acids is determined.

Another specific embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein in a sample of bodily fluid of said patient the level of mature ADM-NH2 and/or fragments thereof having at least 5 amino acids is determined, and wherein said level of mature ADM-NH2 and/or fragments thereof is correlated with the extent of congestion in said subject or diagnosing congestion, wherein the threshold is within a threshold range between 50 and 100 pg/ml.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein an assay is used for determining the level of mature ADM-NH2 and/or fragments thereof having at least 5 amino acids in a sample of bodily fluid of said patient.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein an assay is used for determining the level of mature ADM-NH2 and/or fragments thereof having at least 5 amino acids in a sample of bodily fluid of said patient, wherein the assay sensitivity of said assay is able to quantify the mature ADM-NH2 of healthy subjects and is < 70 pg/ml, preferably < 40 pg/ml and more preferably < 10 pg/ml.

One embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein in a sample of bodily fluid of said patient the level of CT-proADM and/or fragments thereof having at least 5 amino acids is determined.

One further embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein in a sample of bodily fluid of said patient the level of CT-proADM and/or fragments thereof having at least 5 amino acids is determined, and wherein said level of CT-proADM and/or fragments thereof is correlated with the extent of congestion in said subject or diagnosing congestion, wherein the threshold is within a threshold range between 85 and 350 pmol/L.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein an assay is used for determining the level of CT-proADM and/or fragments thereof having at least 5 amino acids in a sample of bodily fluid of said patient.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein an assay is used for determining the level of CT-proADM and/or fragments thereof having at least 5 amino acids in a sample of bodily fluid of said patient, wherein the assay sensitivity of said assay is able to quantify the CT-proADM of healthy subjects and is < 100 pmol/L, preferably < 75 pmol/L and more preferably < 50 pmol/L.

Another preferred embodiment of the present application relates to a Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein in a sample of bodily fluid of said patient the level of MR-proADM and/or fragments thereof having at least 5 amino acids is determined, and wherein said level of MR-proADM and/or fragments thereof is correlated with the extent of congestion in said subject or diagnosing congestion, wherein the threshold is within a threshold range between 0.5 and 1.5 nmol/L.

Another preferred embodiment of the present application relates to a Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein in a sample of bodily fluid of said patient the level of MR-proADM and/or fragments thereof having at least 5 amino acids is determined.

Another preferred embodiment of the present application relates to a Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein in a sample of bodily fluid of said patient the level of MR-proADM and/or fragments thereof having at least 5 amino acids is determined, and wherein said level of MR-proADM and/or fragments thereof is correlated with the extent of congestion in said subject or diagnosing congestion, wherein the threshold is within a threshold range between 0.5 and 1.5 nmol/L.

One further embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein an assay is used for determining the level of MR-proADM and/or fragments thereof having at least 5 amino acids in a sample of bodily fluid of said patient.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein an assay is used for determining the level of MR-proADM and/or fragments thereof having at least 5 amino acids in a sample of bodily fluid of said patient, wherein the assay sensitivity of said assay is able to quantify the MR-proADM of healthy subjects and is < 0,5 nmol/L, preferably < 0,4npmol/L and more preferably < 0,2 nmol/L.

In a specific embodiment of the present application, said binder exhibits a binding affinity to proADM and/or fragments thereof of at least 10⁷ M⁻¹, preferred 10⁸ M⁻¹, preferred affinity is greater than 10⁹ M⁻¹, most preferred greater than 10¹⁰ M⁻¹. A person skilled in the art knows that it may be considered to compensate lower affinity by applying a higher dose of compounds and this measure would not lead out-of-the-scope of the invention.

To determine the affinity of the antibodies to Adrenomedullin, the kinetics of binding of Adrenomedullin to immobilized antibody was determined by means of label-free surface plasmon resonance using a Biacore 2000 system as described in the application WO2018/007588 (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare), (Lorenz et al. 2011. Antimicrob Agents Chemother. 55 (1): 165-173).

In a specific embodiment of of the present application, said binder is selected from the group comprising an antibody or an antibody fragment or a non-Ig scaffold binding to proADM and/or fragments thereof.

In a specific embodiment of the present invention, an assay is used for determining the level of proADM and/or fragments thereof having at least 5 amino acids, wherein such assay is a sandwich assay, preferably a fully automated assay.

In one embodiment of the invention it may be a so-called POC-test (point-of-care) that is a test technology, which allows performing the test within less than 1 hour near the patient without the requirement of a fully automated assay system. One example for this technology is the immunochromatographic test technology.

In another embodiment such an assay is a sandwich immunoassay using any kind of detection technology including but not restricted to enzyme label, chemiluminescence label, electrochemiluminescence label, preferably a fully automated assay. In one embodiment of the diagnostic method such an assay is an enzyme labeled sandwich assay. Examples of automated or fully automated assay comprise assays that may be used for one of the following systems: Roche Elecsys^{®}, Abbott Architect^{®}, Siemens Centauer^{®}, Brahms Kryptor^{®}, BiomerieuxVidas^{®}, Alere Triage^{®}.

A variety of immunoassays are known and may be used for the assays and methods of the present invention, these include: radioimmunoassays ("RIA"), homogeneous enzyme-multiplied immunoassays ("EMIT"), enzyme linked immunoadsorbent assays ("ELISA"), apoenzyme reactivation immunoassay ("ARIS"), dipstick immunoassays and immuno-chromotography assays.

Another preferred embodiment of the present application relates to a combination medication for use in the treatment of a patient having acute and/or persistent dyspnea, wherein the agent according to group b. (iii) of said combination medication is a combination of angiotensin II inhibitor and neprilysin inhibitor (ARNi), wherein in particular the ARNi is a combination of
- the neprilysin inhibitor 4-[[(2*S*,4*R*)-5-ethoxy-4-methyl-5-oxo-1-(4-phenylphenyl)pentan-2-yl] amino]-4-oxobutanoic acid, and
- the nonpeptide triazole-derived antagonist of angiotensin (AT) II (2*S*)-3-methyl-2-[pentanoyl-[[4-[2-(2*H*-tetrazol-5-yl)phenyl]phenyl]methyl]amino]butanoic acidII,
preferably in a fixed combination.

Another specific embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea , comprising at least three of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), e.g. dapagliflozin or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea;
e. a diuretic to reduce or prevent occurrence of signs and/or symptoms of congestion in patients,
preferably in a fixed combination.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the agent according to group b. (iii) of said combination medication according to the aforementioned embodiments is a combination of angiotensin II inhibitor and neprilysin inhibitor (ARNi), wherein in particular the ARNi is a combination of
- the neprilysin inhibitor 4-[[(2*S*,4*R*)-5-ethoxy-4-methyl-5-oxo-1-(4-phenylphenyl)pentan-2-yl] amino]-4-oxobutanoic acid, and
- the nonpeptide triazole-derived antagonist of angiotensin (AT) II (2*S*)-3-methyl-2-[pentanoyl-[[4-[2-(2*H*-tetrazol-5-yl)phenyl]phenyl]methyl]amino]butanoic acid II;
preferably a fixed combination.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient receives the combination medication in two different doses for a certain period of time, respectively, wherein said doses are named dose No 1 and dose No 2., wherein each dose No. 1 and No.2 refers to the respective dose of each of the chemical entities of the combination.

In the context of the present application, dose No. 1 is administered as the initial dose to a patient for a certain period of time. Thereafter dose No. 2 is administered to a patient for a certain period of time, whereby dose No. 2 comprises increased amounts for each chemical entity of the combination medication.

As used herein, a dose of medicine or a drug is a measured amount of it which is intended to be taken at one time. This can be expressed as the weight of drug (e.g. 250 mg), volume of drug solution (e.g. 10 mL), the number of dosage forms (e.g. 1 capsule,) or some other quantity (e.g. 2 puffs). The dosage regimen is the frequency at which the drug doses are given. The dosage form is the physical form of a dose of drug. The route of administration is the way the dosage form is given. Common routes of administration include oral, rectal, inhalation, nasal and topical. The optimal dosage is the dosage that gives the desired effect with minimum side effects.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient receives the combination medication in two different doses for a certain period of time, respectively, wherein said doses are named dose No 1 and dose No 2, wherein each dose refers to the respective dose of each of the chemical entities of the combination medication, wherein dose No. 1 is initially administered to said patient in an initial daily dose of each of the chemical entities of the combination for a certain period of time and is thereafter administered in a subsequent dose No. 2 for a certain period of time, wherein dose No. 2 is 2 to 4-fold higher than the said initial daily dose No. 1.

In the context of the present application, each single dose No. 1 or No. 2 is composed of specific individual amounts of each of the chemical entities that make up that specific single dose.

At the beginning of the treatment, patients reive with the single dose No. 1 an initial smaller dose with corresponding smaller amounts of each of the individual chemical entities.

This single dose is increased during the course of the patient's treatment so that single dose No. 2 contains higher amounts of each of the chemical entities.

One embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient receives a dose No 1 comprising a small dose of each entity in the beginning and a dose No. 2 after weeks of treatment, wherein for each single entity the amount will be increased in dose No. 2.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient receives a dose No 1 as initial dose for a certain period of time, wherein dose No. 1 comprises a small dose of each entity in the beginning and a dose No. 2 after weeks of treatment for a certain period of time, wherein for each single entity the amount will be increased in dose No. 2.

For example, a patient receives dose No. 1 for time period 1 in the form of a single pill, wherein said dose No. 1 comprises 2.5 mg betablocker, 5 mg ACE inhibitor, 10 mg MRA and 10 mg glifozine. Thereafter, said patient receives dose No.2 for time period 2 in the form of a single pill,, wherein said dose No. 2 comprises 5 mg betablocker, 10 mg ACE inhibitor, 20 mg MRA and 20 mg glifozine, wherein for betablocker, ACE inhibitor, MRA and glifozine in both single doses No. 1 and No.2 there are the identical chemical entities and these only differ each in their amounts for dose No. 1 and dose No.2

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient receives a dose No 1 of said combination medication for up to 2 to 4 weeks and thereafter a dose No 2 for at least 90 days.

In one particular embodiment of the present application, the duration of the second daily dose is at least one month, at elast 90 days or at least 6 months. The phase of the second dose may be unlimited or as long as the patient is in need thereof.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said patient receives a dose No 1 of said combination medication for up to 2 to 4 weeks and thereafter a dose No 2 for at least 90 days, wherein dose No 1 is one quarter dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

In the context of the present application, said patient receives said combination in an initial dose of 1 to 50% for up to 2 to 4 weeks (half dose) and thereafter a high full dose of 50 to 100% until 90 at least days, wherein the full doses comprises beta-blockers (100%, including 100% at optimal dose), ACE-inhibitors (100%, including 100% at optimal dose), MRAs (100%, including 100% at optimal dose), and/or canaglyfozine (30%).

In the context of the present application, the term dose is defined as a specified amount of medication taken at one time. The term dosage is the prescribed administration of a specific amount, number, and frequency of doses over a specific period of time.

As used herein, the term "full dose " relates to a dose of 50 to 100%, wherein the full dose is defined as the double amount of each each active ingredient in the later phase compared to the half dose in the initial phase of 2 to 4 weeks.

The term half dose relates to a dose of 1 to 50% and is also described as initial dose.

One specific embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said dose No 2 of said combination medication can comprise each single chemical entity of the following list of agents in a dosage range as specified for each entity.

For the avoidance of doubt for the present invention, these ranges for the amounts for the respective entities mean that these individual entities in the respective ranges may be part of the claimed combination medication individually.

### ACE-inhibitors

Captopril:
(2*S*)-1-[(2*S*)-2-methyl-3-sulfanylpropanoyl]pyrrolidine-2-carboxylic acid
Dosage range from 37.5 mg/day to 150 mg/day, preferable from 75 mg/day mg to 150 mg/day, more preferably from 112.5 mg/day to 150 mg/day

Enalapril:
(2*S*)-1-[(2*S*)-2-[[(2*S*)-1-ethoxy-1-oxo-4-phenylbutan-2yl]amino]propanoyl]pyrrolidine-2-carboxylic acid
Dosage range from 5-10 mg/day to 20-40 mg/day, preferable from 10-20 mg/day to 20-40 mg/day, more preferably from 15-30 mg/day to 20-40 mg/day

Lisinopril:
(2*S*)-1-[(2*S*)-6-amino-2-[[(1*S*)-1-carboxy-3 -phenylpropyl] amino] hexanoyl]pyrrolidine-2-carboxylic acid
Dosage range from 5-8.75 mg/day to 20-35 mg/day, preferable from 10-17.5 mg/day to 20-35 mg/day, more preferably from 15-26.25 mg/day to 20-35 mg/day

Ramipril:
(2*S*,3*aS*,6*aS*)-1-[(2*S*)-2-[[(2*S*)-1-ethoxy-1-oxo-4-phenylbutan-2yl]amino]propanoyl]-3,3*a*,4,5,6,6*a*-hexahydro-2*H*-cyclopenta[b]pyrrole-2-carboxylic acid
Dosage range from 2.5 mg/day to 10 mg/day, preferable from 5 mg/day to 10 mg/day, more preferably from 7.5 mg/day to 10 mg/day

Trandolapril:
(2*S*,3*aR*,7*aS*)-1-[(2*S*)-2-[[(2*S*)-1-ethoxy-1-oxo-4-phenylbutan-2-yl]amino]propanoyl]-2,3,3*a*,4,5,6,7,7*a*-octahydroindole-2-carboxylic acid
Dosage range from 1 mg/day to 4 mg/day, preferable from 2 mg/day to 4 mg/day, more preferably from 3 mg/day to 4 mg/day

Benazepril:
2-[(3S)-3-[[(2S)-1-ethoxy-1-oxo-4-phenylbutan-2-yl]amino]-2-oxo-4,5-dihydro-3H-1-benzazepin-1-yl]acetic acid
Dosage range from 2.5 mg/day to 10 mg/day, preferable from 5 mg/day to 10 mg/day, more preferably from 7.5 mg/day to 10 mg/day

Fosinopril:
(2S,4S)-4-cyclohexyl-1-[2-[(2-methyl-1-propanoyloxypropoxy)-(4-phenylbutyl)phosphoryl]acetyl]pyrrolidine-2-carboxylic acid
Dosage range from 10 mg/day to 40 mg/day, preferable from 20 mg/day to 40 mg/day, more preferably from 30 mg/day to 40 mg/day

Moexipril:
(3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid
Dosage range from 7.5 mg/day to 30 mg/day, preferable from 15 mg/day to 30 mg/day, more preferably from 22.5 mg/day to 30 mg/day

Perindopril *Arginine:*
(2S,3aS,7aS)-1-[(2S)-2-[[(2S)-1-ethoxy-1-oxopentan-2-yl]amino]propanoyl]-2, 3,3a,4,5,6,7,7a-octahydroindole-2-carboxylicacid;(2S)-2-amino-5-(diaminomethylideneamino) pentanoic acid
Dosage range from 2.5 mg/day to 10 mg/day, preferable from 5 mg/day to 10 mg/day, more preferably from 7.5 mg/day to 10 mg/day

Perindopril *tert-butylamine:*
(2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine
Dosage range from 2 mg/day to 8 mg/day, preferable from 4 mg/day to 8 mg/day, more preferably from 6 mg/day to 8 mg/day

Quinapril :
(3 S)-2-[(2 S)-2-[[(2 S)-1-ethoxy-1-oxo-4-phenylbutan-2-yl]amino]propanoyl]-3,4-dihydro-1H-isoquinoline-3-carboxylic acid
Dosage range from 10 mg/day to 40 mg/day, preferable from 20 mg/day to 40 mg/day, more preferably from 30 mg/day to 40 mg/day

Cilazapril :
(4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid
Dosage range from 1.25 mg/day to 5 mg/day, preferable from 2.5 mg/day to 5 mg/day, more preferably from 3.75 mg/day to 5 mg/day

### Beta-blockers

Bisoprolol:
1-(propan-2-ylamino)-3-[4-(2-propan-2-yloxyethoxymethyl)phenoxy]propan-2-ol
Dosage range from 2.5 mg/day to 10 mg/day, preferable from 5 mg/day to 10 mg/day, more preferably from 7.5 mg/day to 10 mg/day

Carvedilol:
1-(9*H*-carbazol-4-yloxy)-3-[2-(2-methoxyphenoxy)ethylamino]propan-2-ol
Dosage range from 12.5 mg/day to 50 mg/day, preferable from 25 mg/day to 50 mg/day , more preferably from 37.5 mg/day to 50 mg/day

Metoprolol succinate:
succinate of 1-[4-(2-methoxyethyl)phenoxy]-3-(propan-2-ylamino)propan-2-ol
Dosage range from 50 mg/day to 200 mg/day, preferable from 100 mg/day to 200 mg/day, more preferably from 150 mg/day to 200 mg/day

Nebivolol:
1-(6-fluoro-3,4-dihydro-2*H*-chromen-2-yl)-2-[[2-(6-fluoro-3,4-dihydro-2*H*-chromen-2-yl)-2-hydroxyethyl]amino] ethanol
Dosage range from 2.5 mg/day to 10 mg/day, preferable from 5 mg/day to 10 mg/day, more preferably from 7.5 mg/day to 10 mg/day

Nadolol:
(2R,3S)-5-[3-(tert-butylamino)-2-hydroxypropoxy]-1,2,3,4tetrahydronaphthalene-2,3-diol
Dosage range from 20 mg/day to 80 mg/day, preferable from 40 mg/day to 80 mg/day, more preferably from 60 mg/day to 80 mg/day

Acebutolol :
N-[3-acetyl-4-[2-hydroxy-3-(propan-2-ylamino)propoxy]phenyl]butanamide
Dosage range from 100 mg/day to 400 mg/day, preferable from 200 mg/day to 400 mg/day, more preferably from 300 mg/day to 400 mg/day

Labetolol :
2-hydroxy-5-[1-hydroxy-2-(4-phenylbutan-2-ylamino)ethyl]benzamide
Dosage range from 200 mg/day to 800 mg/day, preferable from 400 mg/day to 800 mg/day, more preferably from 600 mg/day to 800 mg/day

Celiprolol :
3-[3-acetyl-4-[3-(tert-butylamino)-2-hydroxypropoxy]phenyl]-1,1-diethylurea
Dosage range from 150 mg/day to 600 mg/day, preferable from 300 mg/day to 600 mg/day, more preferably from 450 mg/day to 600 mg/day

Atenolol :
2-[4-[2-hydroxy-3-(propan-2-ylamino)propoxy]phenyl] acetamide
Dosage range from 25 mg/day to 100 mg/day, preferable from 50 mg/day to 100 mg/day, more preferably from 75 mg/day to 100 mg/day

Propranolol :
1-naphthalen-1-yloxy-3 -(propan-2-ylamino)propan-2-ol
Dosage range from 40 mg/day to 160 mg/day, preferable from 80 mg/day to 160 mg/day, more preferably from 120 mg/day to 160 mg/day

### Angiotensin Receptor Blockers (ARBs)

Candesartan:
2-ethoxy-3-[[4-[2-(2*H*-tetrazol-5-yl)phenyl]phenyl]methyl]benzimidazole-4-carboxylic acid
Dosage range from 8 mg/day to 32 mg/day, preferable from 16 mg/day to 32 mg/day, more preferably from 24 mg/day to 32 mg/da

Valsartan:
(2*S*)-3-methyl-2-[pentanoyl-[[4-[2-(2*H*-tetrazol-5-yl)phenyl]phenyl]methyl] amino]butanoic acid
Dosage range from 80 mg/day to 320 mg/day, preferable from 160 mg/day to 320 mg/day, more preferably from 240 mg/day to 320 mg/day

Losartan:
[2-butyl-5-chloro-3-[[4-[2-(2*H*-tetrazol-5-yl)phenyl]phenyl]methyl]imidazol-4-yl]methanol Dosage range from 37.5 mg/day to 150 mg/day, preferable from 75 mg/day to 150 mg/day, more preferably from 112.5 mg/day to 150 mg/day

Azilsartan:
2-ethoxy-3-[[4-[2-(5-oxo-4H-1,2,4-oxadiazol-3-yl)phenyl]phenyl]methyl] benzimidazole-4-carboxylic acid
Dosage range from 20 mg/day to 80 mg/day, preferable from 40 mg/day to 80 mg/day, more preferably from 60 mg/day to 80 mg/day

Eprosartan:
4-[[2-butyl-5-[(E)-2-carboxy-3-thiophen-2-ylprop-1-enyl]imidazol-1-yl] methyl]benzoic acid Dosage range from 150 mg/day to 600 mg/day, preferable from 300 mg/day to 600 mg/day, more preferably from 450 mg/day to 600 mg/day

Irbesartan:
2-butyl-3-[[4-[2-(2H-tetrazol-5-yl)phenyl]phenyl]methyl]-1,3-diazaspiro[4.4]non-1-en-4-one Dosage range from 75 mg/day to 300 mg/day, preferable from 150 mg/day to 300 mg/day, more preferably from 225 mg/day to 300 mg/day

Olmesartan:
5-(2-hydroxypropan-2-yl)-2-propyl-3-[[4-[2-(2H-tetrazol-5-yl)phenyl]phenyl]methyl]imidazole-4-carboxylic acid
Dosage range from 10 mg/day to 40 mg/day, preferable from 20 mg/day to 40 mg/day, more preferably from 30 mg/day to 40 mg/day

Telmisartan:
2-[4-[[4-methyl-6-(1-methylbenzimidazol-2-yl)-2-propylbenzimidazol-1-yl]methyl]phenyl]benzoic acid
Dosage range from 20 mg/day to 80 mg/day, preferable from 40 mg/day to 80 mg/day, more preferably from 60 mg/day to 80 mg/day

### Mineraloreceptor antagonist (MRAs)

Eplerenone:
methyl(1*R*,2*S*,9*R*,10*R*,11*S*,14*R*,15*S*,17*R*)-2,15-dimethyl-5,5'-dioxospiro[18-oxapentacyclo[8.8.0.01,17.02,7.011,15]octadec-6-ene-14,2'-oxolane]-9-carboxylate
Dosage range from 12.5 mg/day to 50 mg/day, preferable from 25 mg/day to 50 mg/day, more preferably from 37.5 mg/day to 50 mg/day

Spironolactone:
S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate
Dosage range from 12.5 mg/day to 50 mg/day, preferable from 25 mg/day to 50 mg/day, more preferably from 37.5 mg/day to 50 mg/day

Finerenone:
(4*S*)-4-(4-cyano-2-methoxyphenyl)-5-ethoxy-2,8-dimethyl-1,4-dihydro-1,6-naphthyridine-3-carboxamide
Dosage range from 10 mg/day to 20 mg/day, preferable 15 mg/day to 20 mg/day

### ARNI

Sacubitril/valsartan:
4-[[(2*S*,4*R*)-5-ethoxy-4-methyl-5-oxo-1-(4-phenylphenyl)pentan-2-yl]amino]-4-oxobutanoic acid / (2*S*)-3-methyl-2-[pentanoyl-[[4-[2-(2*H*-tetrazol-5-yl)phenyl] phenyl] methyl] amino]butanoic acid
Dosage range from 48.5mg/51.5mg/day to 194mg/206mg/day, preferable from 97mg/103mg/day to 194mg/206mg/day, more preferably from 145.5mg/154.5mg/day to 194mg/206mg/day

### Loop-acting Diuretics

Furosemide:
4-chloro-2-(furan-2-ylmethylamino)-5-sulfamoylbenzoic acid
Dosage range from 10-60 mg/day to 40-240 mg/day, preferable from 20-120 mg/day to 40-240 mg/day, more preferably from 30-180 mg/day to 40-240 mg/day

Bumetanide:
3-(butylamino)-4-phenoxy-5-sulfamoylbenzoic acid
Dosage range from 0.25-1.25 mg/day to 1-5 mg/day, preferable from 0.5-2.5 mg/day to 1-5 mg/day, more preferably from 0.75-3.75 mg/day to 1-5 mg/day

Torasemide:
1-[4-(3-methylanilino)pyridin-3-yl]sulfonyl-3-propan-2-ylurea
Dosage range from 2.5-5 mg/day to 10-20 mg/day, preferable from 5-10 mg/day to 10-20 mg/day, more preferably from 7.5-15 mg/day to 10-20 mg/day

Bendroflumethiazide:
3-benzyl-1,1-dioxo-6-(trifluoromethyl)-3,4-dihydro-2H-1lambda6,2,4-benzothiadiazine-7-sulfonamide
Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day

Hydrochlorothiazide:
6-chloro-1,1-dioxo-3,4-dihydro-2H-1lambda6,2,4-benzothiadiazine-7-sulfonamide
Dosage range from 3.125-25 mg/day to 12.5-100 mg/day, preferable from 6.25-50 mg/day to 12.5-100 mg/day, more preferably from 9.375-75 mg/day to 12.5-100 mg/day

Metolazone:
7-chloro-2-methyl-3-(2-methylphenyl)-4-oxo-1,2-dihydroquinazoline-6-sulfonamide
Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day

Indapamide:
4-chloro-N-(2-methyl-2,3-dihydroindol-1-yl)-3-sulfamoylbenzamide
Dosage range from 0.625-1.25 mg/day to 2.5-5 mg/day, preferable from 1.25-2.5 mg/day to 2.5-5 mg/day, more preferably from 1.875-3.75 mg/day to 2.5-5 mg/day

### Gliflozine

Dapagliflozin:
(2S,3R,4R,5S,6R)-2-[4-chloro-3-[(4-ethoxyphenyl)methyl]phenyl]-6-(hydroxymethyl)oxane-3,4,5-triol
Dosage range from 2.5 mg/day to 10 mg/day, preferable from 5 mg/day to 10 mg/day, more preferably from 7.5 mg/day to 10 mg/day

Canagliflozin:
(2S,3R,4R,5S,6R)-2-[3-[[5-(4-fluorophenyl)thiophen-2-yl]methyl]-4-methylphenyl]-6-(hydroxymethyl)oxane-3,4,5-triol
Dosage range from 75 mg/day to 300 mg/day, preferable from 150 mg/day to 300 mg/day, more preferably from 225 mg/day to 300 mg/day

Empagliflozin :
(2S,3R,4R,5S,6R)-2-[4-chloro-3-[[4-[(3S)-oxolan-3-yl]oxyphenyl]methyl]phenyl]-6-(hydroxymethyl)oxane-3,4,5-triol
Dosage range from 6.25 mg/day to 25 mg/day, preferable from 10 mg/day to 25 mg/day, more preferably from 10 mg/day to 20 mg/day

Ertugliflozin :
(1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol
Dosage range from 3.75 mg/day to 15 mg/day, preferable from 7.5 mg/day to 15 mg/day, more preferably from 11.25 mg/day to 15 mg/day

Ipragliflozin
(2S,3R,4R,5S,6R)-2-[3-(1-benzothiophen-2-ylmethyl)-4-fluorophenyl]-6-(hydroxymethyl)oxane-3,4,5-triol
Dosage range from 25 mg/day to 100 mg/day, preferable from 50 mg/day to 100 mg/day, more preferably from 75 mg/day to 100 mg/day

Tofogliflozin
(3S,3'R,4'S,5'S,6'R)-5-[(4-ethylphenyl)methyl]-6'-(hydroxymethyl)spiro[1H-2-benzofuran-3,2'-oxane]-3',4',5'-triol
Dosage range from 5 mg/day to 20 mg/day, preferable from 10 mg/day to 20 mg/day, more preferably from 15 mg/day to 20 mg/day

Luseogliflozin
(2S,3R,4R,5S,6R)-2-[5-[(4-ethoxyphenyl)methyl]-2-methoxy-4-methylphenyl]-6-(hydroxymethyl)thiane-3,4,5-triol
Dosage range from 1.25 mg/day to 5 mg/day, preferable from 2.5 mg/day to 5m g/day, more preferably from 3.75 mg/day to 5 mg/day

Sotagliflozin :
(2S,3R,4R,5S,6R)-2-[4-chloro-3-[(4-ethoxyphenyl)methyl]phenyl]-6-methylsulfanyloxane-3,4,5-triol
Dosage range from 100 mg/day to 400 mg/day, preferable from 200 mg/day to 400 mg/day, more preferably from 300 mg/day to 400 mg/day.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, further comprising omecamtiv mecarbil (methyl 4-[[2-fluoro-3-[(6-methylpyridin-3-yl)carbamoylamino]phenyl]methyl]piperazine-1-carboxylate) with a dose range from 50 to 100 mg/day, more preferably 75 mg/day to 100 mg/day and/or vericiguat (methyl N-[4,6-diamino-2-[5-fluoro-1-[(2-fluorophenyl)methyl]pyrazolo[3,4-b]pyridin-3-yl]pyrimidin-5-yl]carbamate) with a dose range from 2.5 mg/day to 10 mg/day, preferably with a dose range from 5 mg/day to 10 mg/day, more preferably from 7.5 to 10 mg/day.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein said fixed or non-fixed combination medication is administered orally, preferably a fixed combination.

As used herein, the term "fixed combination" is defined as the combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the components to be administered are in a single administration form.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein a beta blocker is selected from the group comprising beta-1 adrenergic receptor antagonist 1-(propan-2-ylamino)-3-[4-(2-propan-2-yloxyethoxymethyl)phenoxy]propan-2-ol, beta-adrenoceptor blocking agent 1-(9*H*-carbazol-4-yloxy)-3-[2-(2-methoxyphenoxy)ethylamino]propan-2-ol, succinate of 1-[4-(2-methoxyethyl)phenoxy]-3-(propan-2-ylamino)propan-2-ol (CR/XL), the beta-1 adrenergic receptor antagonist 1-(6-fluoro-3,4-dihydro-2*H*-chromen-2-yl)-2-[[2-(6-fluoro-3,4-dihydro-2*H*-chromen-2-yl)-2-hydroxyethyl]amino]ethanol, (2R,3S)-5-[3-(tert-butylamino)-2-hydroxypropoxy]-1,2,3,4tetrahydronaphthalene-2,3-diol, N-[3-acetyl-4-[2-hydroxy-3-(propan-2-ylamino)propoxy]phenyl]butanamide, 2-hydroxy-5-[1-hydroxy-2-(4-phenylbutan-2-ylamino)ethyl]benzamide, 3-[3-acetyl-4-[3-(tert-butylamino)-2-hydroxypropoxy]phenyl]-1,1-diethylurea, 2-[4-[2-hydroxy-3 -(propan-2-ylamino)propoxy]phenyl]acetamide, 1-naphthalen-1-yloxy-3-(propan-2-ylamino)propan-2-ol.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the ACE inhibitor is selected from the group comprising the sulfhydryl-containing analog of proline (2*S*)-1-[(2*S*)-2-methyl-3-sulfanylpropanoyl]pyrrolidine-2-carboxylic acid, (2*S*)-1-[(2*S*)-2-[[(2,*S*)-1-ethoxy-1-oxo-4-phenylbutan-2-yl]amino]propanoyl]pyrrolidine-2-carboxylic acid, (2*S*)-1-[(2*S*)-6-amino-2-[[(1*S*)-1-carboxy-3-phenylpropyl]amino]hexanoyl]pyrrolidine-2-carboxylic acid, (2*S*,3*aS*,6*aS*)-1-[(2*S*)-2-[[(2*S*)-1-ethoxy-1-oxo-4-phenylbutan-2-yl]amino]propanoyl]-3,3*a*,4,5,6,6*a*-hexahydro-2*H*-cyclopenta[b]pyrrole-2-carboxylic acid, (2*S*,3*aR*,7*aS*)-1-[(2*S*)-2-[[(2*S*)-1-ethoxy-1-oxo-4-phenylbutan-2-yl]amino]propanoyl]-2,3,3*a*,4,5,6,7,7*a-*octahydroindole-2-carboxylic acid, 2-[(3S)-3-[[(2S)-1-ethoxy-1-oxo-4-phenylbutan-2-yl]amino]-2-oxo-4,5-dihydro-3H-1-benzazepin-1-yl] acetic acid, (2S,4S)-4-cyclohexyl-1-[2-[(2-methyl-1-propanoyloxypropoxy)-(4-phenylbutyl)phosphoryl]acetyl]pyrrolidine-2-carboxylic acid, (3 S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid, (2S,3aS,7aS)-1-[(2S)-2-[[(2S)-1-ethoxy-1-oxopentan-2-yl] amino]propanoyl]-2, 3,3a,4,5,6,7,7a-octahydroindole-2-carboxylicacid;(2S)-2-amino-5-(diaminomethylideneamino) pentanoic acid, (2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine, (3S)-2-[(2S)-2-[[(2S)-1-ethoxy-1-oxo-4-phenylbutan-2-yl]amino]propanoyl]-3,4-dihydro-1H-isoquinoline-3-carboxylic acid, (4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid.

Another specific embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the MRAs (mineralo receptor antagonist) is selected from the group comprising methyl (1*R*,2*S*,9*R*,10*R*,11*S*,14*R*,15*S*,17*R*)-2,15-dimethyl-5,5'-dioxospiro[18-oxapentacyclo[8.8.0.0^{1,17}.0^{2,7}.0^{11,15}]octadec-6-ene-14,2'-oxolane]-9-carboxylate, S-[(7R,8R,9S,10R,S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate or Finerenone: (4*S*)-4-(4-cyano-2-methoxyphenyl)-5-ethoxy-2,8-dimethyl-1,4-dihydro-1,6-naphthyridine-3-carboxamide.

Another embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the diurectic is selected from the group comprising loop-acting diuretics such as 3-(butylamino)-4-phenoxy-5-sulfamoylbenzoic acid, 2-[2,3-dichloro-4-(2-methylidenebutanoyl)phenoxy]acetic acid, 4-chloro-2-(furan-2-ylmethylamino)-5 - sulfamoylbenzoic acid, 1-[4-(3-methylanilino)pyridin-3-yl]sulfonyl-3-propan-2-ylurea, potassium-sparing diuretics such as 3,5-diamino-6-chloro-*N-*(diaminomethylidene)pyrazine-2-carboxamide, methyl (1*R*,2*S*,9*R*,10*R*,11*S*,14*R*,15*S*,17*R*)-2,15-dimethyl-5,5'-dioxospiro[18-oxapentacyclo[8.8.0.0 oxolane]-9-carboxylate, S-[(7R,8R,9S,10*R*,13*S*,14*S*,17*R*)-10,13-dimethyl-3,5'-dioxospiro[2,6,7,8,9,11,12,14,15,16-decahydro-1*H*-cyclopenta[a]phenanthrene-17,2'-oxolane]-7-yl] ethanethioate, 6-phenylpteridine-2,4,7-triamine , thiazide diuretics such as 6-chloro-1,1-dioxo-4*H*-1λ⁶,2,4-benzothiadiazine-7-sulfonamide, 2-chloro-5-(1-hydroxy-3-oxo-2*H*-isoindol-1-yl)benzenesulfonamide, 6-chloro-1,1-dioxo-3,4-dihydro-2H-1lambda6,2,4-benzothiadiazine-7-sulfonamide, 4-chloro-N-(2-methyl-2,3-dihydroindol-l-yl)-3-sulfamoylbenzamide, 7-chloro-2-methyl-3-(2-methylphenyl)-4-oxo-1,2-dihydroquinazoline-6-sulfonamide and/or mixtures thereof.

Another preferred embodiment of the present application relates to a combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea, wherein the gliflozine is selected from the group comprising (2S,3R,4R,5S,6R)-2-[3-[[5-(4-fluorophenyl)thiophen-2-yl]methyl]-4-methylphenyl]-6-(hydroxymethyl)oxane-3,4,5-triol, (2S,3R,4R,5S,6R)-2-[4-chloro-3-[(4-ethoxyphenyl)methyl]phenyl]-6-(hydroxymethyl)oxane-3,4,5-triol, (2S,3R,4R,5S,6R)-2-[4-chloro-3-[[4-[(3S)-oxolan-3-yl]oxyphenyl]methyl]phenyl]-6-(hydroxymethyl)oxane-3,4,5-triol, (1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol, Luseogliflozin, Remogliflozin etabonate (ethyl [(2R,3S,4S,5R,6S)-3,4,5-trihydroxy-6-[5-methyl-1-propan-2-yl-4-[(4-propan-2-yloxyphenyl)methyl]pyrazol-3-yl]oxyoxan-2-yl]methyl carbonate), sergliflozin etabonate (ethyl [(2R,3S,4S,5R,6S)-3,4,5-trihydroxy-6-[2-[(4-methoxyphenyl)methyl]phenoxy]oxan-2-yl]methyl carbonate), (2S,3R,4R,5S,6R)-2-[3-(1-benzothiophen-2-ylmethyl)-4-fluorophenyl]-6-(hydroxymethyl)oxane-3,4,5-triol, (3S,3'R,4'S,5'S,6'R)-5-[(4-ethylphenyl)methyl]-6'-(hydroxymethyl)spiro[1H-2-benzofuran-3,2'-oxane]-3',4',5'-triol, (2S,3R,4R,5S,6R)-2-[5-[(4-ethoxyphenyl)methyl]-2-methoxy-4-methylphenyl]-6-(hydroxymethyl)thiane-3,4,5-triol, (2S,3R,4R,5S,6R)-2-[4-chloro-3-[(4-ethoxyphenyl)methyl]phenyl]-6-methylsulfanyloxane-3,4,5-triol, and/or mixtures thereof.

Subject-matter of the present application is also a pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination medication, wherein a sample of bodily fluid of said patient has a level of BNP > 200 pg/mL and/or has a level NT-proBNP > 600 pg/mL, and wherein said sample of bodily fluid is selected from the group comprising blood, plasma and serum.

Subject-matter of the present application is also a pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination medication, wherein a sample of bodily fluid of said patient has a level of BNP > 300 pg/mL and/or has a level NT-proBNP > 800 pg/mL, and wherein said sample of bodily fluid is selected from the group comprising blood, plasma and serum.

Another embodiment of the present application relates to a pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination, wherein said patient has no heart failure (HF).

Another preferred embodiment of the present application relates to a pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination medication, wherein said patient has no history of heart failure (HF).

One embodiment of the present application relates to a pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination medication, wherein the patient has no clinical signs of congestion.

One preferred embodiment of the present application relates to a pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination medication, wherein the patient has no cardiovascular or pulmonary disease.

Another embodiment of the present application relates to a pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination medication, wherein said combination is a fixed combination in one administration form.

It is to be understood that a pharmaceutical composition comprising the present combination medication is for use to be administered to a human patient. The term "administering" in all of its grammatical forms means administration of a therapeutic agent or combination medication. In the context of the present invention "administration of a" or "administering a" or any other grammatical form thereof means that the present combination medication is administered in the form of a pharmaceutical composition, optionally comprising a pharmaceutically acceptable carrier.

Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO₂), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti-oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc), humectants (e.g. propylene, glycol, glycerol, sorbitol). Other suitable pharmaceutically acceptable excipients are *inter alia* described in Remington's Pharmaceutical Sciences, 15th Ed., Mack Publishing Co., New Jersey (1991) and Bauer et al., Pharmazeutische Technologie, 5th Ed., Govi-Verlag Frankfurt (1997). Another preferred embodiment of the present application relates to a pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination medication, wherein said combination is a fixed combination in one administration form, wherein said administration form is administered orally.

One embodiment of the present application relates to a pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination medication, wherein said combination is a fixed combination in one administration form, wherein the formulation is selected from the group comprising a pill, a tablet, a liqiuid medicine, a capsule, a film tablet, a dragee.

One specific embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the formulation is a tablet, pill or capsule.

Solid formulations may comprise excipients and other ingredients in suitable amounts. Such solid formulations may contain e.g. cellulose, cellulose microcrystalline, polyvidon in particular FB polyvidon, magnesium stearate and the like.

The person skilled in the art will readily be able to choose suitable pharmaceutically acceptable carriers or excipients, depending, e.g., on the formulation and administration route of the pharmaceutical composition.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said combination medication comprises at least threeof the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), e.g. dapagliflozin or empagliflozin, and wherein said inhibitor of the sodium glucose co-transporter-2, e.g. dapagliflozine or empagliflozin, is used for restoring the restoration rate and/or maintaining (a normal) respiration rate and/or restoring the oxygen saturation and/or for ameliorating the symptoms of dyspnea;
e. a diuretic to reduce or prevent occurrence of signs and/or symptoms of congestion in patients,
and wherein a sample of bodily fluid of said patient has a level of BNP > 200 pg/mL and/or has a level NT-proBNP > 600 pg/mL, and/or the patient is congestive and/or has residual congestion, and wherein said sample of bodily fluid is selected from the group comprising blood, plasma and serum,
and wherein said patient is either
- a patient with heart failure with left ventricular ejection fraction (LVEF), greater and equal to 40 %, preferably 50 % or
- a patient with no heart failure.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said formulation comprises a combination medication comprising at least four of the following compounds wherein each of said chemical compounds is a chemical entity, wherein each chemical entity is distinct from the other two :
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), e.g. dapagliflozin or empagliflozin, and wherein said inhibitor of the sodium glucose co-transporter-2, e.g. dapagliflozin or empagliflozin, is used for restoring the restoration rate and/or maintaining (a normal) respiration rate and/or restoring the oxygen saturation and/or for ameliorating the symptoms of dyspnea;
e. a diuretic to reduce or prevent occurrence of signs and/or symptoms of congestion in patients,
and wherein a sample of bodily fluid of said patient has a level of BNP > 200 pg/mL and/or has a level of NT-proBNP > 600 pg/mL and/or the patient is congestive and/or has residual congestion, and wherein said sample of bodily fluid is selected from the group comprising blood, plasma and serum;
and wherein said patient is either
- a patient with heart failure with left ventricular ejection fraction (LVEF), greater and equal to 40 %, preferably 50 % or
- a patient with no heart failure, and wherein the treatment is a daily administration of the combination medication, preferably once a day, and wherein said combination is a fixed combination and wherein said combination is a fixed combination in one administration form,
   wherein said administration form is selected from the group comprising a pill, a tablet, a liqiuid medicine, a capsule, a film tablet, a dragee, and
   wherein the level of proADM and/or fragments thereof having at least 5 amino acids in a sample of bodily fluid of said patient is above a certain threshold, and wherein said sample of bodily fluid is selected from the group comprising plasma, serum, blood, and wherein said patient receives the combination medication in two different doses for a certain period of time, which are named dose No 1 and dose No 2, and
   wherein said patient receives a dose No 1 of said combination medication for up to 2 to 4 weeks and thereafter a dose No 2 for at least 90 days, and
   wherein dose No 1 is one quarter dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said formulation comprises a combination medication comprising at least two of the following compounds wherein each of said chemical compounds is a chemical entity, wherein each chemical entity is distinct from the other :
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozine or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea,
e. a diuretic to reduce or prevent occurrence of signs and/or symptoms of congestion in patients,
and wherein a sample of bodily fluid of said patient has a level of BNP > 300 pg/mL and/or has a level NT-proBNP > 800 pg/mL, and/or the patient is congestive and/or has residual congestion and wherein said sample of bodily fluid is selected from the group comprising blood, plasma and serum,
wherein said patient is either
   - a patient with heart failure with left ventricular ejection fraction (LVEF) greater and equal to 40 %, preferably 50 % or
   - a patient with no heart failure, and
wherein the treatment is a daily administration of the combination medication, preferably once a day, and wherein said combination is a fixed combination and wherein said combination is a fixed combination in one single administration form, wherein said administration form is selected from the group comprising a pill, a tablet, a liqiuid medicine, a capsule, a film tablet, a dragee, and
wherein the level of proADM and/or fragments thereof having at least 5 amino acids in a sample of bodily fluid of said patient is above a certain threshold, and wherein said sample of bodily fluid is selected from the group comprising plasma, serum, blood, and wherein said patient receives the combination medication in two different doses for a certain period of time, respectively, which are named dose No 1 and dose No 2, and wherein said patient receives a dose No 1 of said combination medication for up to 2 to 4 weeks and thereafter a dose No 2 for at least 90 days, and
wherein dose No 1 is one quarter dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said formulation comprises a combination medication comprising at least two of the following compounds wherein each of said chemical compounds is a chemical entity, wherein each chemical entity is distinct from the other :
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozine or empagliflozin, and wherein said inhibitor of the, e.g. dapagliflozin or empagliflozin, is used for restoring the restoration rate and/or maintaining respiration rate and/or restoring and/or maintaining the oxygen saturation and/or for ameliorating the symptoms of dyspnea;
a diuretic to reduce or prevent occurrence of signs and/or symptoms of congestion in patients, and wherein a sample of bodily fluid of said patient has a level of BNP > 200 pg/mL and/or has a level NT-proBNP > 600 pg/mL, and/or the patient is congestive and/or has residual congestion and wherein said sample of bodily fluid is selected from the group comprising blood, plasma and serum, and
wherein the treatment is a daily administration of the combination medication, preferably once a day, and
wherein the patient has no heart failure but has another cardiovascular disease and/or another disease with non cardiac cause which maybe selected from the group comprising diabetes and kidney disease, and
wherein said combination is a fixed combination and wherein said combination is a fixed combination in one single administration form, wherein said administration form is selected from the group comprising a pill, a tablet, a liqiuid medicine, a capsule, a film tablet, a dragee, and
wherein the level of proADM and/or fragments thereof having at least 5 amino acids in a sample of bodily fluid of said patient is above a certain threshold, wherein said sample of bodily fluid is selected from the group comprising plasma, serum, blood, and wherein said patient receives a dose No 1 of said combination medication for up to 2 to 4 weeks and thereafter a dose No 2 for at least 90 days, wherein dose No 1 is one quarter dose (25%) of dose No 2, more preferably .dose No 1 is 35% of dose No 2, more preferably .dose No 1 is 40% of dose No 2, more preferably .dose No 1 is 45% of dose No 2, more preferably dose No 1 is 50% of dose No 2.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said formulation comprises a combination medication comprising at least two of the following compounds wherein each of said chemical compounds is a chemical entity, wherein each chemical entity is distinct from the other :
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozine or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea,
e. a diuretic to reduce or prevent occurrence of signs and/or symptoms of congestion in patients,
and wherein a sample of bodily fluid of said patient has a level of BNP > 300 pg/mL and/or has a level NT-proBNP > 800 pg/mL, and/or the patient is congestive and/or has residual congestion and wherein said sample of bodily fluid is selected from the group comprising blood, plasma and serum, and
wherein the treatment is a daily administration of the combination medication,preferably once a day, and
wherein the patient has no heart failure but has another cardiovascular disease and/or another disease with non cardiac cause which maybe selected from the group comprising diabetes and kidney disease, and
wherein said combination is a fixed combination and wherein said combination is a fixed combination in one single administration form, wherein said administration form is selected from the group comprising a pill, a tablet, a liqiuid medicine, a capsule, a film tablet, a dragee, and
wherein the level of proADM and/or fragments thereof having at least 5 amino acids in a sample of bodily fluid of said patient is above a certain threshold, wherein said sample of bodily fluid is selected from the group comprising plasma, serum, blood, and wherein said patient receives a dose No 1 of said combination medication for up to 2 to 4 weeks and thereafter a dose No 2 for at least 90 days, wherein dose No 1 is one quarter dose (25%) of dose No 2, more preferably .dose No 1 is 35% of dose No 2, more preferably .dose No 1 is 40% of dose No 2, more preferably .dose No 1 is 45% of dose No 2, more preferably dose No 1 is 50% of dose No 2.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said combination medication comprises at least two of the following compounds wherein each of said chemical compounds is a chemical entity, wherein each chemical entity is distinct from the other two :
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozine or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea,
e. a diuretic to reduce or prevent occurrence of signs and/or symptoms of congestion in patients,
   and wherein a sample of bodily fluid of said patient has a level of BNP > 300 pg/mL
   and/or has a level NT-proBNP > 800 pg/mL, and/or the patient is congestive and/or has residual congestion and wherein said sample of bodily fluid is selected from the group comprising blood, plasma and serum, and wherein said patient is either
   - a patient with heart failure with left ventricular ejection fraction (LVEF) greater and equal to 40 %, preferably 50 % or
   - a patient with no heart failure.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said combination medication comprises at least two of said components which are RAASi and BB, preferably said combination medication comprises at least three of said components which are RAASi, BB and MRAs, more preferably said combination medication comprises at least three of said components and gliflozine.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said patient is a patient with heart failure with left-ventricular ejection fraction (LVEF) or a patient with no heart-failure.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said patient
- is a patient with heart failure with left-ventricular ejection fraction (LVEF) greater and equal to 40 %, preferably 50 % or
- a patient with no heart failure.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the treatment is a daily administration of the combination medication, preferably once a day.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said patient is a patient with heart failure.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said patient is a patient with heart failure and wherein said patient has a mildly reduced left-ventricular ejection fraction (LVEF) with more than or equal to 40%.

2. Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the patient has no heart failure, but has another cardiovascular disease and/or another disease with non cardiac cause which maybe selected from the group comprising diabetes and kidney disease, and wherein a sample of bodily fluid of said patient has a level of BNP > 200 pg/mL and/or has a level NT-proBNP > 600 pg/mL, wherein the bodily fluid is a plasma sample, preferably a fasting plasma sample.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the patient has no heart failure, but has another cardiovascular disease and/or another disease with non cardiac cause which maybe selected from the group comprising diabetes and kidney disease, and wherein a sample of bodily fluid of said patient has a level of BNP > 300 pg/mL and/or has a level NT-proBNP > 800 pg/mL

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the agent according to group b. (iii) of said combination medication is a combination of angiotensin II inhibitor and neprilysin inhibitor (ARNi), wherein in particular the ARNi is a combination of
- the neprilysin inhibitor 4-[[(2*S*,4*R*)-5-ethoxy-4-methyl-5-oxo-1-(4-phenylphenyl)pentan-2-yl] amino]-4-oxobutanoic acid, and
- the nonpeptide triazole-derived antagonist of angiotensin (AT) II (2*S*)-3-methyl-2-[pentanoyl-[[4-[2-(2*H*-tetrazol-5-yl)phenyl]phenyl]methyl]amino]butanoic acid II, preferably a fixed combination.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said combination medication comprises
a. a beta blocker, and
b. one agent of the group consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA), and
d. gliflozine (canagliflozine),
preferably in a fixed combination

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said combination medication comprises in addition diuretics, preferably in a fixed combination..

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said patient receives the combination medication in two different doses for a certain period of time, respectively, which are named dose No 1 and dose No 2.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein said patient receives a dose No 1 of said combination medication for up to 2 to 4 weeks and thereafter a dose No 2 for at least 90 days, wherein dose No 1 is one quarter dose (25%) of dose No 2, more preferably dose No 1 is 30% of dose No 2, more preferably dose No 1 is 35% of dose No 2, more preferably .dose No 1 is 40% of dose No 2, more preferably dose No 1 is 45% of dose No 2, more preferably dose No 1 is 50% of dose No 2.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein a beta blocker is selected from the group comprising beta-1 adrenergic receptor antagonist 1-(propan-2-ylamino)-3-[4-(2-propan-2-yloxyethoxymethyl)phenoxy]propan-2-ol, beta-adrenoceptor blocking agent 1-(9*H*-carbazol-4-yloxy)-3-[2-(2-methoxyphenoxy)ethylamino]propan-2-ol, succinate of 1-[4-(2-methoxyethyl)phenoxy]-3-(propan-2-ylamino)propan-2-ol (CR/XL), the beta-1 adrenergic receptor antagonist 1-(6-fluoro-3,4-dihydro-2*H*-chromen-2-yl)-2-[[2-(6-fluoro-3,4-dihydro-2*H*-chromen-2-yl)-2-hydroxyethyl]amino]ethanol, (2R,3S)-5-[3-(tert-butylamino)-2-hydroxypropoxy] - 1,2,3,4tetrahydronaphthalene-2,3-diol, N-[3-acetyl-4-[2-hydroxy-3-(propan-2-ylamino)propoxy]phenyl]butanamide, 2-hydroxy-5-[1-hydroxy-2-(4-phenylbutan-2-ylamino)ethyl]benzamide, 3-[3-acetyl-4-[3-(tert-butylamino)-2-hydroxypropoxy]phenyl]-1,1-diethylurea, 2-[4-[2-hydroxy-3-(propan-2-ylamino)propoxy]phenyl]acetamide, 1-naphthalen-l-yloxy-3 -(propan-2-ylamino)propan-2-ol.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the ACE inhibitor is selected from the group comprising the sulfhydryl-containing analog of proline (2*S*)-1-[(2*S*)-2-methyl-3-sulfanylpropanoyl]pyrrolidine-2-carboxylic acid, (2*S*)-1-[(2*S*)-2-[[(2*S*)-1-ethoxy-1-oxo-4-phenylbutan-2-yl]amino]propanoyl]pyrrolidine-2-carboxylic acid, (2*S*)-1-[(2*S*)-6-amino-2-[[(1*S*)-1-carboxy-3-phenylpropyl]amino]hexanoyl]pyrrolidine-2-carboxylic acid, (2*S*,3*aS*,6*aS*)-1-[(2*S*)-2-[[(2*S*)-1-ethoxy-1-oxo-4-phenylbutan-2-yl]amino]propanoyl]-3,3*a*,4,5,6,6*a*-hexahydro-2*H*-cyclopenta[b]pyrrole-2-carboxylic acid, (2*S*,3*aR*,7*aS*)-1-[(2*S*)-2-[[(2*S*)-1-ethoxy-1-oxo-4-phenylbutan-2-yl]amino]propanoyl] - 2,3,3*a*,4,5,6,7,7*a*-octahydroindole-2-carboxylic acid, 2-[(3S)-3-[[(2S)-1-ethoxy-1-oxo-4-phenylbutan-2-yl] amino] -2-oxo-4,5 -dihydro-3H-1-benzazepin-1-yl] acetic acid, (2S,4S)-4-cyclohexyl-1-[2-[(2-methyl-1-propanoyloxypropoxy)-(4-phenylbutyl)phosphoryl]acetyl]pyrrolidine-2-carboxylic acid, (3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid, (2S,3aS,7aS)-1-[(2S)-2-[[(2S)-1-ethoxy-1-oxopentan-2-yl]amino]propanoyl]-2, 3,3a,4,5,6,7,7a-octahydroindole-2-carboxylicacid;(2S)-2-amino-5-(diaminomethylideneamino) pentanoic acid, (2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine, (3S)-2-[(2S)-2-[[(2S)-1-ethoxy-1-oxo-4-phenylbutan-2-yl] amino]propanoyl]-3,4-dihydro-1H-isoquinoline-3 -carboxylic acid, (4S, 7S)-7-[[(2S)-1-ethoxy-1-oxo-4-phenylbutan-2-yl]amino]-6-oxo-1,2,3,4,7,8,9, 10-octahydropyridazino[1,2-a]diazepine-4-carboxylic acid.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the MRAs (mineralo receptor antagonist) is selected from the group comprising methyl (1*R*,2*S*,9*R*, 10*R*,11*S*,14*R*,15*S*,17*R*)-2,15-dimethyl-5,5'-dioxospiro[18-oxapentacyclo[8.8.0.0^{1,17}.0^{2,7}.0^{11,15}]octadec-6-ene-14,2'-oxolane]-9-carboxylate, S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the diurectic is selected from the group comprising loop-acting diuretics such as 3-(butylamino)-4-phenoxy-5-sulfamoylbenzoic acid, 2-[2,3-dichloro-4-(2-methylidenebutanoyl)phenoxy]acetic acid, 4-chloro-2-(furan-2-ylmethylamino)-5-sulfamoylbenzoic acid, 1-[4-(3-methylanilino)pyridin-3-yl]sulfonyl-3-propan-2-ylurea, potassium-sparing diuretics such as 3,5-diamino-6-chloro-*N*-(diaminomethylidene)pyrazine-2-carboxamide,, methyl (1*R*,2*S*,9*R*,10*R*,11*S*,14*R*,15*S*,17*R*)-2,15-dimethyl-5,5'-dioxospiro[18-oxapentacyclo[8.8.0.0^{1,17}.0^{2,7}.0^{11,15}]octadec-6-ene-14,2'-oxolane]-9-carboxylate, *S-*[(7*R*,8*R*,9*S*,10*R*,13*S*,14*S*,17*R*)-10,13-dimethyl-3,5'-dioxospiro[2,6,7,8,9,11,12,14,15,16-decahydro-1*H*-cyclopenta[a]phenanthrene-17,2'-oxolane]-7-yl] ethanethioate , 6-phenylpteridine-2,4,7-triamine , thiazide diuretics such as 6-chloro-1,1-dioxo-4*H*-1λ⁶,2,4-benzothiadiazine-7-sulfonamide, 2-chloro-5-(1-hydroxy-3-oxo-2*H*-isoindol-1-yl)benzenesulfonamide, 6-chloro-1,1-dioxo-3,4-dihydro-2H-1lambda6,2,4-benzothiadiazine-7-sulfonamide, 4-chloro-N-(2-methyl-2,3-dihydroindol-1-yl)-3-sulfamoylbenzamide, 7-chloro-2-methyl-3-(2-methylphenyl)-4-oxo-1,2-dihydroquinazoline-6-sulfonamide, 7-chloro-2-methyl-3-(2-methylphenyl)-4-oxo-1,2-dihydroquinazoline-6-sulfonamide and/or mixtures thereof.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the gliflozine is selected from the group comprising (2S,3R,4R,5S,6R)-2-[3-[[5-(4-fluorophenyl)thiophen-2-yl]methyl]-4-methylphenyl]-6-(hydroxymethyl)oxane-3,4,5-triol, (2S,3R,4R,5S,6R)-2-[4-chloro-3-[(4-ethoxyphenyl)methyl]phenyl]-6-(hydroxymethyl)oxane-3,4,5-triol, (2S,3R,4R,5S,6R)-2-[4-chloro-3-[[4-[(3S)-oxolan-3-yl]oxyphenyl]methyl]phenyl]-6-(hydroxymethyl)oxane-3,4,5-triol, (1S,2S,3S,4R,5S)-5-[4-chloro-3-[(4-ethoxyphenyl)methyl]phenyl]-1-(hydroxymethyl)-6, 8-dioxabicyclo[3.2.1]octane-2,3,4-triol, Luseogliflozin, Remogliflozin etabonate (ethyl [(2R,3S,4S,5R,6S)-3,4,5-trihydroxy-6-[5-methyl-1-propan-2-yl-4-[(4-propan-2-yloxyphenyl)methyl]pyrazol-3-yl]oxyoxan-2-yl]methyl carbonate), sergliflozin etabonate (ethyl [(2R,3S,4S,5R,6S)-3,4,5-trihydroxy-6-[2-[(4-methoxyphenyl)methyl]phenoxy]oxan-2-yl]methyl carbonate), (2S,3R,4R,5S,6R)-2-[3-(1-benzothiophen-2-ylmethyl)-4-fluorophenyl]-6-(hydroxymethyl)oxane-3,4,5-triol,(3S,3'R,4'S,5'S,6'R)-5-[(4-ethylphenyl)methyl]-6'-(hydroxymethyl)spiro[1H-2-benzofuran-3,2'-oxane]-3',4',5'-triol, (2S,3R,4R,5S,6R)-2-[5-[(4-ethoxyphenyl)methyl]-2-methoxy-4-methylphenyl]-6-(hydroxymethyl)thiane-3,4,5-triol, (2S,3R,4R,5S,6R)-2-[4-chloro-3-[(4-ethoxyphenyl)methyl]phenyl]-6-methylsulfanyloxane-3,4,5-triol and/or mixtures thereof.

Another embodiment of the present application relates to a pharmaceutical oral formulation for use in the treatment of acute and/or persistent dyspnea for restoring the respiration rate and/or maintaining the respiration rate and/or restoring and/or maintaining the oxygen saturation and/or for ameliorating the symptoms of dyspnea in a patient, wherein wherein the ranges of each of the chemical entities of the combination for dose No. 2 of the following agentsare as follows:

### ACE-inhibitors

Captopril:
(2*S*)-1-[(2*S*)-2-methyl-3-sulfanylpropanoyl]pyrrolidine-2-carboxylic acid
Dosage range from 37.5 mg/day to 150 mg/day, preferable from 75 mg/day mg to 150 mg/day, more preferably from 112.5 mg/day to 150 mg/day

Enalapril:
(2*S*)-1-[(2*S*)-2-[[(2*S*)-1-ethoxy-1-oxo-4-phenylbutan-2yl]amino]propanoyl]pyrrolidine-2-carboxylic acid
Dosage range from 5-10 mg/day to 20-40 mg/day, preferable from 10-20 mg/day to 20-40 mg/day, more preferably from 15-30 mg/day to 20-40 mg/day

Lisinopril:
(2*S*)-1-[(2*S*)-6-amino-2-[[(1*S*)-1-carboxy-3-phenylpropyl]amino]hexanoyl]pyrrolidine-2-carboxylic acid
Dosage range from 5-8.75 mg/day to 20-35 mg/day, preferable from 10-17.5 mg/day to 20-35 mg/day, more preferably from 15-26.25 mg/day to 20-35 mg/day

Ramipril:
(2*S*,3*aS*,6*aS*)-1-[(2*S*)-2-[[(2*S*)-1-ethoxy-1-oxo-4-phenylbutan-2yl]amino]propanoyl]-3,3*a*,4,5,6,6*a*-hexahydro-2*H*-cyclopenta[b]pyrrole-2-carboxylic acid
Dosage range from 2.5 mg/day to 10 mg/day, preferable from 5 mg/day to 10 mg/day, more preferably from 7.5 mg/day to 10 mg/day

Trandolapril:
(2*S*,3*aR*,7*aS*)-1-[(2*S*)-2-[[(2*S*)-1-ethoxy-1-oxo-4-phenylbutan-2-yl]amino]propanoyl]-2,3,3*a*,4,5,6,7,7*a*-octahydroindole-2-carboxylic acid
Dosage range from 1 mg/day to 4 mg/day, preferable from 2 mg/day to 4 mg/day, more preferably from 3 mg/day to 4 mg/day

Benazepril:
2-[(3S)-3-[[(2S)-1-ethoxy-1-oxo-4-phenylbutan-2-yl]amino]-2-oxo-4,5-dihydro-3H-1-benzazepin-1-yl]acetic acid
Dosage range from 2.5 mg/day to 10 mg/day, preferable from 5 mg/day to 10 mg/day, more preferably from 7.5 mg/day to 10 mg/day

Fosinopril:
(2S,4S)-4-cyclohexyl-1-[2-[(2-methyl-1-propanoyloxypropoxy)-(4-phenylbutyl)phosphoryl]acetyl]pyrrolidine-2-carboxylic acid
Dosage range from 10 mg/day to 40 mg/day, preferable from 20 mg/day to 40 mg/day, more preferably from 30 mg/day to 40 mg/day

Moexipril:
(3 S)-2-[(2 S)-2-[[(2S)-1-ethoxy-1-oxo-4-phenylbutan-2yl]amino]propanoyl]-6,7-dimethoxy-3,4-dihydro-1H-isoquinoline-3-carboxylic acid
Dosage range from 7.5 mg/day to 30 mg/day, preferable from 15 mg/day to 30 mg/day, more preferably from 22.5 mg/day to 30 mg/day

Perindopril *Arginine:*
(2S,3aS,7aS)-1-[(2S)-2-[[(2S)-1-ethoxy-1-oxopentan-2-yl]amino]propanoyl]-2, 3,3a,4,5,6,7,7a-octahydroindole-2-carboxylicacid;(2S)-2-amino-5-(diaminomethylideneamino) pentanoic acid
Dosage range from 2.5 mg/day to 10 mg/day, preferable from 5 mg/day to 10 mg/day, more preferably from 7.5 mg/day to 10 mg/day

Perindopril *tert-butylamine:*
(2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine
Dosage range from 2 mg/day to 8 mg/day, preferable from 4 mg/day to 8 mg/day, more preferably from 6 mg/day to 8 mg/day

Quinapril :
(3S)-2-[(2S)-2-[[(2S)-1-ethoxy-1-oxo-4-phenylbutan-2-yl]amino]propanoyl]-3,4-dihydro-1H-isoquinoline-3-carboxylic acid
Dosage range from 10 mg/day to 40 mg/day, preferable from 20 mg/day to 40 mg/day, more preferably from 30 mg/day to 40 mg/day

Cilazapril :
(4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid
Dosage range from 1.25 mg/day to 5 mg/day, preferable from 2.5 mg/day to 5 mg/day, more preferably from 3.75 mg/day to 5 mg/day

### Beta-blockers

Bisoprolol:
1-(propan-2-ylamino)-3-[4-(2-propan-2-yloxyethoxymethyl)phenoxy]propan-2-ol
Dosage range from 2.5 mg/day to 10 mg/day, preferable from 5 mg/day to 10 mg/day, more preferably from 7.5 mg/day to 10 mg/day

Carvedilol:
1-(9*H*-carbazol-4-yloxy)-3-[2-(2-methoxyphenoxy)ethylamino]propan-2-ol
Dosage range from 12.5 mg/day to 50 mg/day, preferable from 25 mg/day to 50 mg/day , more preferably from 37.5 mg/day to 50 mg/day

Metoprolol succinate:
succinate of 1-[4-(2-methoxyethyl)phenoxy]-3-(propan-2-ylamino)propan-2-ol
Dosage range from 50 mg/day to 200 mg/day, preferable from 100 mg/day to 200 mg/day, more preferably from 150 mg/day to 200 mg/day

Nebivolol:
1-(6-fluoro-3,4-dihydro-2*H*-chromen-2-yl)-2-[[2-(6-fluoro-3,4-dihydro-2*H*-chromen-2-yl)-2-hydroxyethyl]amino] ethanol
Dosage range from 2.5 mg/day to 10 mg/day, preferable from 5 mg/day to 10 mg/day, more preferably from 7.5 mg/day to 10 mg/day

Nadolol:
(2R,3S)-5-[3-(tert-butylamino)-2-hydroxypropoxy]-1,2,3,4tetrahydronaphthalene-2,3-diol Dosage range from 20 mg/day to 80 mg/day, preferable from 40 mg/day to 80 mg/day, more preferably from 60 mg/day to 80 mg/day

Acebutolol :
N-[3-acetyl-4-[2-hydroxy-3-(propan-2-ylamino)propoxy]phenyl]butanamide
Dosage range from 100 mg/day to 400 mg/day, preferable from 200 mg/day to 400 mg/day, more preferably from 300 mg/day to 400 mg/day

Labetolol :
2-hydroxy-5-[1-hydroxy-2-(4-phenylbutan-2-ylamino)ethyl]benzamide
Dosage range from 200 mg/day to 800 mg/day, preferable from 400 mg/day to 800 mg/day, more preferably from 600 mg/day to 800 mg/day

Celiprolol :
3-[3-acetyl-4-[3-(tert-butylamino)-2-hydroxypropoxy]phenyl]-1,1-diethylurea
Dosage range from 150 mg/day to 600 mg/day, preferable from 300 mg/day to 600 mg/day, more preferably from 450 mg/day to 600 mg/day

Atenolol :
2-[4-[2-hydroxy-3 -(propan-2-ylamino)propoxy]phenyl] acetamide
Dosage range from 25 mg/day to 100 mg/day, preferable from 50 mg/day to 100 mg/day, more preferably from 75 mg/day to 100 mg/day

Propranolol :
1-naphthalen-1-yloxy-3-(propan-2-ylamino)propan-2-ol
Dosage range from 40 mg/day to 160 mg/day, preferable from 80 mg/day to 160 mg/day, more preferably from 120 mg/day to 160 mg/day

### Angiotensin Receptor Blockers (ARBs)

Candesartan:
2-ethoxy-3-[[4-[2-(2*H*-tetrazol-5-yl)phenyl]phenyl]methyl]benzimidazole-4-carboxylic acid Dosage range from 8 mg/day to 32 mg/day, preferable from 16 mg/day to 32 mg/day, more preferably from 24 mg/day to 32 mg/da

Valsartan:
(2*S*)-3-methyl-2-[pentanoyl-[[4-[2-(2*H*-tetrazol-5-yl)phenyl]phenyl]methyl]amino]butanoic acid
Dosage range from 80 mg/day to 320 mg/day, preferable from 160 mg/day to 320 mg/day, more preferably from 240 mg/day to 320 mg/day

Losartan:
[2-butyl-5-chloro-3-[[4-[2-(2*H*-tetrazol-5-yl)phenyl]phenyl]methyl]imidazol-4-yl]methanol Dosage range from 37.5 mg/day to 150 mg/day, preferable from 75 mg/day to 150 mg/day, more preferably from 112.5 mg/day to 150 mg/day

Azilsartan:
2-ethoxy-3-[[4-[2-(5-oxo-4H-1,2,4-oxadiazol-3-yl)phenyl]phenyl]methyl] benzimidazole-4-carboxylic acid
Dosage range from 20 mg/day to 80 mg/day, preferable from 40 mg/day to 80 mg/day, more preferably from 60 mg/day to 80 mg/day

Eprosartan:
4-[[2-butyl-5-[(E)-2-carboxy-3-thiophen-2-ylprop-1-enyl]imidazol-1-yl] methyl]benzoic acid Dosage range from 150 mg/day to 600 mg/day, preferable from 300 mg/day to 600 mg/day, more preferably from 450 mg/day to 600 mg/day

Irbesartan:
2-butyl-3-[[4-[2-(2H-tetrazol-5-yl)phenyl]phenyl]methyl]-1,3-diazaspiro[4.4]non-1-en-4-one Dosage range from 75 mg/day to 300 mg/day, preferable from 150 mg/day to 300 mg/day, more preferably from 225 mg/day to 300 mg/day

Olmesartan:
5-(2-hydroxypropan-2-yl)-2-propyl-3-[[4-[2-(2H-tetrazol-5-yl)phenyl]phenyl]methyl]imidazole-4-carboxylic acid
Dosage range from 10 mg/day to 40 mg/day, preferable from 20 mg/day to 40 mg/day, more preferably from 30 mg/day to 40 mg/day

Telmisartan:
2-[4-[[4-methyl-6-(1-methylbenzimidazol-2-yl)-2-propylbenzimidazol-1-yl]methyl]phenyl]benzoic acid
Dosage range from 20 mg/day to 80 mg/day, preferable from 40 mg/day to 80 mg/day, more preferably from 60 mg/day to 80 mg/day

### Mineraloreceptor antagonist (MRAs)

Eplerenone:
methyl(1*R*,2*S*,9*R*,10*R*,11*S*,14*R*,15*S*,17*R*)-2,15-dimethyl-5,5'-dioxospiro[18-oxapentacyclo[8.8.0.01,17.02,7.011,15]octadec-6-ene-14,2'-oxolane]-9-carboxylate
Dosage range from 12.5 mg/day to 50 mg/day, preferable from 25 mg/day to 50 mg/day, more preferably from 37.5 mg/day to 50 mg/day

Spironolactone:
S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate
Dosage range from 12.5 mg/day to 50 mg/day, preferable from 25 mg/day to 50 mg/day, more preferably from 37.5 mg/day to 50 mg/day

Finerenone:
(4*S*)-4-(4-cyano-2-methoxyphenyl)-5-ethoxy-2,8-dimethyl-1,4-dihydro-1,6-naphthyridine-3-carboxamide
Dosage range from 10 mg/day to 20 mg/day, preferable 15 mg/day to 20 mg/day

### ARNI

Sacubitril/valsartan:
4-[[(2*S*,4*R*)-5-ethoxy-4-methyl-5-oxo-1-(4-phenylphenyl)pentan-2-yl]amino]-4-oxobutanoic acid / (2*S*)-3-methyl-2-[pentanoyl-[[4-[2-(2*H*-tetrazol-5-yl)phenyl] phenyl] methyl] amino]butanoic acid
Dosage range from 48.5mg/51.5mg/day to 194mg/206mg/day, preferable from 97mg/103mg/day to 194mg/206mg/day, more preferably from 145.5mg/154.5mg/day to 194mg/206mg/day

### Loop-acting Diuretics

Furosemide:
4-chloro-2-(furan-2-ylmethylamino)-5-sulfamoylbenzoic acid
Dosage range from 10-60 mg/day to 40-240 mg/day, preferable from 20-120 mg/day to 40-240 mg/day, more preferably from 30-180 mg/day to 40-240 mg/day

Bumetanide:
3-(butylamino)-4-phenoxy-5-sulfamoylbenzoic acid
Dosage range from 0.25-1.25 mg/day to 1-5 mg/day, preferable from 0.5-2.5 mg/day to 1-5 mg/day, more preferably from 0.75-3.75 mg/day to 1-5 mg/day

Torasemide:
1-[4-(3-methylanilino)pyridin-3-yl]sulfonyl-3-propan-2-ylurea
Dosage range from 2.5-5 mg/day to 10-20 mg/day, preferable from 5-10 mg/day to 10-20 mg/day, more preferably from 7.5-15 mg/day to 10-20 mg/day

Bendroflumethiazide:
3-benzyl-1,1-dioxo-6-(trifluoromethyl)-3,4-dihydro-2H-1lambda6,2,4-benzothiadiazine-7-sulfonamide
Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day

Hydrochlorothiazide:
6-chloro-1,1-dioxo-3,4-dihydro-2H-1lambda6,2,4-benzothiadiazine-7-sulfonamide
Dosage range from 3.125-25 mg/day to 12.5-100 mg/day, preferable from 6.25-50 mg/day to 12.5-100 mg/day, more preferably from 9.375-75 mg/day to 12.5-100 mg/day

Metolazone:
7-chloro-2-methyl-3-(2-methylphenyl)-4-oxo-1,2-dihydroquinazoline-6-sulfonamide
Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day

Indapamide:
4-chloro-N-(2-methyl-2,3-dihydroindol-1-yl)-3-sulfamoylbenzamide
Dosage range from 0.625-1.25 mg/day to 2.5-5 mg/day, preferable from 1.25-2.5 mg/day to 2.5-5 mg/day, more preferably from 1.875-3.75 mg/day to 2.5-5 mg/day

### Gliflozine

Dapagliflozin:
(2S,3R,4R,5S,6R)-2-[4-chloro-3-[(4-ethoxyphenyl)methyl]phenyl]-6-(hydroxymethyl)oxane-3,4,5-triol
Dosage range from 2.5 mg/day to 10 mg/day, preferable from 5 mg/day to 10 mg/day, more preferably from 7.5 mg/day to 10 mg/day

Canagliflozin:
(2S,3R,4R,5S,6R)-2-[3-[[5-(4-fluorophenyl)thiophen-2-yl]methyl]-4-methylphenyl]-6-(hydroxymethyl)oxane-3,4,5-triol
Dosage range from 75 mg/day to 300 mg/day, preferable from 150 mg/day to 300 mg/day, more preferably from 225 mg/day to 300 mg/day

Empagliflozin :
(2S,3R,4R,5S,6R)-2-[4-chloro-3-[[4-[(3S)-oxolan-3-yl]oxyphenyl]methyl]phenyl]-6-(hydroxymethyl)oxane-3,4,5-triol
Dosage range from 6.25 mg/day to 25 mg/day, preferable from 10 mg/day to 25 mg/day, more preferably from 10 mg/day to 20 mg/day

Ertugliflozin :
(1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol
Dosage range from 3.75 mg/day to 15 mg/day, preferable from 7.5 mg/day to 15 mg/day, more preferably from 11.25 mg/day to 15 mg/day

Ipragliflozin
(2S,3R,4R,5S,6R)-2-[3-(1-benzothiophen-2-ylmethyl)-4-fluorophenyl]-6-(hydroxymethyl)oxane-3,4,5-triol
Dosage range from 25 mg/day to 100 mg/day, preferable from 50 mg/day to 100 mg/day, more preferably from 75 mg/day to 100 mg/day

Tofogliflozin
(3S,3'R,4'S,5'S,6'R)-5-[(4-ethylphenyl)methyl]-6'-(hydroxymethyl)spiro[1H-2-benzofuran-3,2'-oxane]-3',4',5'-triol
Dosage range from 5 mg/day to 20 mg/day, preferable from 10 mg/day to 20 mg/day, more preferably from 15 mg/day to 20 mg/day

Luseogliflozin
(2S,3R,4R,5S,6R)-2-[5-[(4-ethoxyphenyl)methyl]-2-methoxy-4-methylphenyl]-6-(hydroxymethyl)thiane-3,4,5-triol
Dosage range from 1.25 mg/day to 5 mg/day, preferable from 2.5 mg/day to 5m g/day, more preferably from 3.75 mg/day to 5 mg/day

Sotagliflozin :
(2S,3R,4R,5S,6R)-2-[4-chloro-3-[(4-ethoxyphenyl)methyl]phenyl]-6-methylsulfanyloxane-3,4,5-triol
Dosage range from 100 mg/day to 400 mg/day, preferable from 200mg/day to 400 mg/day, more preferably from 300 mg/day to 400 mg/day.

Subject-matter of the present application is also a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication comprising at least two of the following compounds wherein each of said chemical compounds is a chemical entity, wherein each chemical entity is distinct from the other :
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozine or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea,
e. a diuretic to reduce or prevent occurrence of signs and/or symptoms of congestion in patients,
   and wherein a sample of bodily fluid of said patient has a level of BNP > 200 pg/mL
   and/or has a level NT-proBNP > 600 pg/mL, and/or the patient is congestive and/or has residual congestion and wherein said sample of bodily fluid is selected from the group comprising blood, plasma and serum, and wherein said patient is either
   - a patient with heart failure with left ventricular ejection fraction (LVEF) greater and equal to 40 %, preferably 50 % or
   - a patient with no heart failure.

Subject-matter of the present application is also a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication comprising at least two of the following components:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozine or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea,
e. a diuretic to reduce or prevent occurrence of signs and/or symptoms of congestion in patients
   and wherein a sample of bodily fluid of said patient has a level of BNP > 300 pg/mL and/or has a level NT-proBNP > 800 pg/mL, and/or the patient is congestive and/or has residual congestion and wherein said sample of bodily fluid is selected from the group comprising blood, plasma and serum, and wherein said patient is either
   - a patient with heart failure with left ventricular ejection fraction (LVEF) greater and equal to 40 %, preferably 50 % or
   - a patient with no heart failure.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said combination medication comprises at least two of said components which are RAASi and BB, preferably said combination medication comprises at least three of said components which are RAASi, BB and MRAs.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said combination medication comprises at least two of said components which are RAASi and BB, preferably said combination medication comprises at least three of said components which are RAASi, BB and MRAs, more preferably said combination medication comprises at least three of said components and gliflozine.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient
- is a patient with heart failure with either mildly reduced, from 40 to 49%, left-ventricular ejection fraction (LVEF) or preserved, greater and equal to 50 %, LVEF or
- a patient with no heart failure.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein the treatment is a daily administration of the combination medication, preferably once a day.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient is a patient with heart failure with mildly reduced or preserved left-ventricular ejection fraction (LVEF) or a patient with with no heart failure.

Another preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient has a preserved left-ventricular ejection fraction (LVEF) with more than or equal to 50%.

Another preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient has a mildly reduced left-ventricular ejection fraction (LVEF) with more than or equal to 40%.

One embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient is a patient with heart failure and/or wherein said patient has a reduced left-ventricular ejection fraction (LVEF) with less than 40%.

One embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient has a reduced preserved left-ventricular ejection fraction (LVEF) with less than to 40%.

One embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the patient has no heart failure, but has another cardiovascular disease and/or another disease with non cardiac cause which maybe selected from the group comprising diabetes and kidney disease, and wherein a sample of bodily fluid of said patient has a level of BNP > 200 pg/mL and/or has a level NT-proBNP > 600 pg/mL

One embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, wherein the patient has no heart failure, but has another cardiovascular disease and/or another disease with non cardiac cause which maybe selected from the group comprising diabetes and kidney disease, and wherein a sample of bodily fluid of said patient has a level of BNP > 300 pg/mL and/or has a level NT-proBNP > 800 pg/mL, wherein the bodily fluid is a plasma sample, preferably a fasting plasma sample.

One preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient has no heart failure (HF).

One specific embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient has no history of heart failure.

One further embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient patient has no clinical signs of congestion.

One further embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient patient has clinical signs of congestion.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein the patient has no cardiovascular or pulmonary disease.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said combination is a fixed combination in one single administration form.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said combination is a fixed combination, and wherein said combination is a fixed combination in one single administration form, wherein said administration form is selected from the group comprising a pill, a tablet, a liqiuid medicine, a capsule, a film tablet, a dragee.

Another preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein the level of proADM and/or fragments thereof having at least 5 amino acids in a sample of bodily fluid of said patient is above a certain threshold.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein the level of proADM and/or fragments thereof having at least 5 amino acids in a sample of bodily fluid of said patient is above a certain threshold, wherein said sample of bodily fluid is selected from the group comprising plasma, serum, blood.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein Pro-Adrenomedullin or fragments thereof of at least 5 amino acids is used as a marker for congestion comprising:
- Determining the level of Pro-Adrenomedullin or fragments thereof of at least 5 amino acids in a bodily fluid obtained from said subject; and
- Correlating said level of Pro-Adrenomedullin or fragments thereof with the extent of congestion in said subject or diagnosing congestion, wherein an elevated level above a certain threshold is indicative of congestion or the extent of congestion,
wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH₂ according to SEQ ID No.: 4 or ADM-Gly according to SEQ ID No.: 5 or CT-proADM according to SEQ ID No.: 6, and wherein said patient has no congestion, i.e. the level of proADM and/or fragments thereof having at least 5 amino acids in a sample of bodily fluid of said patient is above a certain threshold.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said threshold is within a threshold range that is a threshold range for plasma mature ADM-NH₂ according to SEQ ID No.: 4 between 50 and 100 pg/ml and for plasma MR-proADM between 0.5 and 1.5 nmol/L, and for plasma CT-proADM between 85 and 350 pmol/L.

Another specific embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein an assay is used for determining the level of proADM and/or fragments thereof in a sample of bodily fluid of said patient, wherein the assay sensitivity of said assay is able to quantify the proADM of healthy subjects and is < 70 pg/ml, preferably < 40 pg/ml and more preferably < 10 pg/ml.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein in a sample of bodily fluid of said patient the level of mature ADM-NH2 and/or fragments thereof having at least 5 amino acids is determined.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein in a sample of bodily fluid of said patient the level of mature ADM-NH2 and/or fragments thereof having at least 5 amino acids is determined, and wherein said level of mature ADM-NH2 and/or fragments thereof is correlated with the extent of congestion in said subject or diagnosing congestion, wherein the threshold is within a threshold range between 50 and 100 pg/ml.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein an assay is used for determining the level of mature ADM-NH2 and/or fragments thereof having at least 5 amino acids in a sample of bodily fluid of said patient.

Another specific embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein an assay is used for determining the level of mature ADM-NH2 and/or fragments thereof having at least 5 amino acids in a sample of bodily fluid of said patient, wherein the assay sensitivity of said assay is able to quantify the mature ADM-NH2 of healthy subjects and is < 70 pg/ml, preferably < 40 pg/ml and more preferably < 10 pg/ml.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein in a sample of bodily fluid of said patient the level of CT-proADM and/or fragments thereof having at least 5 amino acids is determined.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein in a sample of bodily fluid of said patient the level of CT-proADM and/or fragments thereof having at least 5 amino acids is determined, and wherein said level of CT-proADM and/or fragments thereof is correlated with the extent of congestion in said subject or diagnosing congestion, wherein the threshold is within a threshold range between 85 and 350 pmol/L.

Another preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein an assay is used for determining the level of CT-proADM and/or fragments thereof having at least 5 amino acids in a sample of bodily fluid of said patient.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein an assay is used for determining the level of CT-proADM and/or fragments thereof having at least 5 amino acids in a sample of bodily fluid of said patient, wherein the assay sensitivity of said assay is able to quantify the CT-proADM of healthy subjects and is < 100 pmol/L, preferably < 75 pmol/L and more preferably < 50 pmol/L.

Another preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein in a sample of bodily fluid of said patient the level of MR-proADM and/or fragments thereof having at least 5 amino acids is determined.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein in a sample of bodily fluid of said patient the level of MR-proADM and/or fragments thereof having at least 5 amino acids is determined, and wherein said level of MR-proADM and/or fragments thereof is correlated with the extent of congestion in said subject or diagnosing congestion, wherein the threshold is within a threshold range between 0.5 and 1.5 nmol/L.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein an assay is used for determining the level of MR-proADM and/or fragments thereof having at least 5 amino acids in a sample of bodily fluid of said patient.

Another preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein an assay is used for determining the level of MR-proADM and/or fragments thereof having at least 5 amino acids in a sample of bodily fluid of said patient, wherein the assay sensitivity of said assay is able to quantify the MR-proADM of healthy subjects and is < 0,5 nmol/L, preferably < 0,4npmol/L and more preferably < 0,2 nmol/L.

One embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein the agent according to group b. (iii) of said combination medication is a combination of angiotensin II inhibitor and neprilysin inhibitor (ARNi), and wherein in particular the ARNi is a combination of the
neprilysin inhibitor 4-[[(2*S*,4*R*)-5-ethoxy-4-methyl-5-oxo-1-(4-phenylphenyl)pentan-2-yl]amino]-4-oxobutanoic acid, and
the nonpeptide triazole-derived antagonist of angiotensin (AT) II (2*S*)-3-methyl-2-[pentanoyl-[[4-[2-(2*H*-tetrazol-5-yl)phenyl]phenyl]methyl]amino]butanoic acidII, preferably a fixed combination.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said combination medication comprises
- a beta blocker, and
- one agent of the group consisting of (i) an angiotensin-converting enzyme (ACE) inhibitor, (ii) an angiotensin II inhibitor and (iii) a combination of angiotensin II inhibitor and angiotensin receptor-neprilysin inhibitor (ARNi)
- and mineralo receptor antagonist (MRA), and
gliflozine (canagliflozine),
preferably in a fixed combination.

Another preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said combination medication comprises in addition diuretics, preferably in a fixed combination.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient receives the combination medication in two different doses for a certain period of time, respectively,, which are named dose No 1 and dose No 2.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient receives a dose No 1 of said combination medication for up to 2 to 4 weeks and thereafter a dose No 2 for at least 90 days, wherein dose No 1 is one qaurter dose of dose No 2.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said patient receives a dose No 1 of said combination medication for up to 2 to 4 weeks and thereafter a dose No 2 for at least 90 days, wherein dose No 1 is one quarter dose (25%) of dose No 2, more preferably dose No 1 is 30% of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

In the context of the present application, this means that dose No. 2 contains the double amount of each active ingredient compared to dose No.1. Dose No.2 is used in the second phase of the study.

One embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein said fixed or non-fixed combination medication is administered orally, preferably a fixed combination.

One preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein a beta blocker is selected from the group comprising beta-1 adrenergic receptor antagonist 1-(propan-2-ylamino)-3-[4-(2-propan-2-yloxyethoxymethyl)phenoxy]propan-2-ol, beta-adrenoceptor blocking agent 1-(9*H*-carbazol-4-yloxy)-3-[2-(2-methoxyphenoxy)ethylamino]propan-2-ol, succinate of 1-[4-(2-methoxyethyl)phenoxy]-3-(propan-2-ylamino)propan-2-ol (CR/XL), the beta-1 adrenergic receptor antagonist 1-(6-fluoro-3,4-dihydro-2*H*-chromen-2-yl)-2-[[2-(6-fluoro-3,4-dihydro-2*H*-chromen-2-yl)-2-hydroxyethyl]amino]ethanol, (2R,3S)-5-[3-(tert-butylamino)-2-hydroxypropoxy]-1,2,3,4tetrahydronaphthalene-2,3-diol, N-[3-acetyl-4-[2-hydroxy-3-(propan-2-ylamino)propoxy]phenyl]butanamide, 2-hydroxy-5-[1-hydroxy-2-(4-phenylbutan-2-ylamino)ethyl]benzamide, 3-[3-acetyl-4-[3-(tert-butylamino)-2-hydroxypropoxy]phenyl]-1, 1-diethylurea, 2-[4-[2-hydroxy-3-(propan-2-ylamino)propoxy]phenyl]acetamide, 1-naphthalen-1-yloxy-3-(propan-2-ylamino)propan-2-ol.

One specific embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein the ACE inhibitor is selected from the group comprising the sulfhydryl-containing analog of proline (2*S*)-1-[(2*S*)-2-methyl-3-sulfanylpropanoyl]pyrrolidine-2-carboxylic acid, (2*S*)-1-[(2*S*)-2-[[(2*S*)-1-ethoxy-1-oxo-4-phenylbutan-2-yl]amino]propanoyl]pyrrolidine-2-carboxylic acid, (2*S*)-1-[(2*S*)-6-amino-2-[[(1*S*)-1-carboxy-3-phenylpropyl]amino]hexanoyl]pyrrolidine-2-carboxylic acid, (2*S*,3*aS*,6*aS*)-1-[(2*S*)-2-[[(2*S*)-1-ethoxy-1-oxo-4-phenylbutan-2-yl]amino]propanoyl]-3,3*a*,4,5,6,6*a*-hexahydro-2*H-*cyclopenta[b]pyrrole-2-carboxylic acid, (2*S*,3*aR*,7*aS*)-1-[(2*S*)-2-[[(2*S*)-1-ethoxy-1-oxo-4-phenylbutan-2-yl]amino]propanoyl]-2,3,3*a*,4,5,6,7,7*a*-octahydroindole-2-carboxylic acid, 2-[(3S)-3-[[(2S)-1-ethoxy-1-oxo-4-phenylbutan-2-yl]amino]-2-oxo-4,5-dihydro-3H-1-benzazepin-1-yl] acetic acid, (2S,4S)-4-cyclohexyl-1-[2-[(2-methyl-1-propanoyloxypropoxy)-(4-phenylbutyl)phosphoryl]acetyl]pyrrolidine-2-carboxylic acid, (3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid, (2S,3aS,7aS)-1-[(2S)-2-[[(2S)-1-ethoxy-1-oxopentan-2-yl]amino]propanoyl]-2, 3,3a,4,5,6,7,7a-octahydroindole-2-carboxylicacid;(2S)-2-amino-5-(diaminomethylideneamino) pentanoic acid, (2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine, (3S)-2-[(2S)-2-[[(2S)-1-ethoxy-1-oxo-4-phenylbutan-2-yl]amino]propanoyl]-3,4-dihydro-1H-isoquinoline-3-carboxylic acid, (4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid.

One further embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein the MRAs (mineralo receptor antagonist) is selected from the group comprising methyl (1*R*,2*S*,9*R*,10*R*,11*S*,14*R*,15*S*,17*R*)-2,15-dimethyl-5,5'-dioxospiro[18-oxapentacyclo[8.8.0.0^{1,17}.0^{2,7}.0^{11,15}]octadec-6-ene-14,2'-oxolane]-9-carboxylate, S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein the diurectic is selected from the group comprising loop-acting diuretics such as 3-(butylamino)-4-phenoxy-5-sulfamoylbenzoic acid, 2-[2,3-dichloro-4-(2-methylidenebutanoyl)phenoxy]acetic acid, 4-chloro-2-(furan-2-ylmethylamino)-5-sulfamoylbenzoic acid, 1-[4-(3-methylanilino)pyridin-3-yl]sulfonyl-3-propan-2-ylurea, potassium-sparing diuretics such as 3,5-diamino-6-chloro-N-(diaminomethylidene)pyrazine-2-carboxamide,, methyl (1*R*,2*S*,9*R*,10*R*,11*S*,14*R*,15*S*,17*R*)-2,15-dimethyl-5,5'-dioxospiro[18-oxapentacyclo[8.8.0.0^{1,17}.0^{2,7}.0^{11,15}]octadec-6-ene-14,2'-oxolane]-9-carboxylate, *S-*[(7*R*,8*R*,9*S*,10*R*,13*S*,14*S*,17*R*)-10,13-dimethyl-3,5'-dioxospiro[2,6,7,8,9,11,12,14,15,16-decahydro-1H-cyclopenta[a]phenanthrene-17,2'-oxolane]-7-yl] ethanethioate , 6-phenylpteridine-2,4,7-triamine , thiazide diuretics such as 6-chloro-1,1-dioxo-4*H*-1λ⁶,2,4-benzothiadiazine-7-sulfonamide, 2-chloro-5-(1-hydroxy-3-oxo-2*H*-isoindol-1-yl)benzenesulfonamide, 6-chloro-1,1-dioxo-3,4-dihydro-2H-1lambda6,2,4-benzothiadiazine-7-sulfonamide, 4-chloro-N-(2-methyl-2,3-dihydroindol-1-yl)-3-sulfamoylbenzamide, 7-chloro-2-methyl-3-(2-methylphenyl)-4-oxo-1,2-dihydroquinazoline-6-sulfonamide, 7-chloro-2-methyl-3-(2-methylphenyl)-4-oxo-1,2-dihydroquinazoline-6-sulfonamide and/or mixtures thereof.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein the gliflozine is selected from the group comprising (2S,3R,4R,5S,6R)-2-[3-[[5-(4-fluorophenyl)thiophen-2-yl]methyl]-4-methylphenyl]-6-(hydroxymethyl)oxane-3,4,5-triol, (2S,3R,4R,5S,6R)-2-[4-chloro-3-[(4-ethoxyphenyl)methyl]phenyl]-6-(hydroxymethyl)oxane-3,4,5-triol, (2S,3R,4R,5S,6R)-2-[4-chloro-3-[[4-[(3S)-oxolan-3-yl]oxyphenyl]methyl]phenyl]-6-(hydroxymethyl)oxane-3,4,5-triol, (1S,2S,3S,4R,5S)-5-[4-chloro-3-[(4-ethoxyphenyl)methyl]phenyl]-1-(hydroxymethyl)-6, 8-dioxabicyclo[3.2.1]octane-2,3,4-triol, Luseogliflozin, Remogliflozin etabonate (ethyl [(2R,3S,4S,5R,6S)-3,4,5-trihydroxy-6-[5-methyl-1-propan-2-yl-4-[(4-propan-2-yloxyphenyl)methyl]pyrazol-3-yl]oxyoxan-2-yl]methyl carbonate), sergliflozin etabonate (ethyl [(2R,3S,4S,5R,6S)-3,4,5-trihydroxy-6-[2-[(4-methoxyphenyl)methyl]phenoxy]oxan-2-yl]methyl carbonate), (2S,3R,4R,5S,6R)-2-[3-(1-benzothiophen-2-ylmethyl)-4-fluorophenyl]-6-(hydroxymethyl)oxane-3,4,5-triol, (3S,3'R,4'S,5'S,6'R)-5-[(4-ethylphenyl)methyl]-6'-(hydroxymethyl)spiro[1H-2-benzofuran-3,2'-oxane]-3',4',5'-triol, (2S,3R,4R,5S,6R)-2-[5-[(4-ethoxyphenyl)methyl]-2-methoxy-4-methylphenyl]-6-(hydroxymethyl)thiane-3,4,5-triol, (2S,3R,4R,5S,6R)-2-[4-chloro-3-[(4-ethoxyphenyl)methyl]phenyl]-6-methylsulfanyloxane-3,4,5-triol and/or mixtures thereof.

Another preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering a combination medication, wherein the dose No. 2 of the following agents are as follows:

### ACE-inhibitors

Captopril:
(2S)-1-[(2S)-2-methyl-3-sulfanylpropanoyl]pyrrolidine-2-carboxylic acid
Dosage range from 37.5 mg/day to 150 mg/day, preferable from 75 mg/day mg to 150 mg/day, more preferably from 112.5 mg/day to 150 mg/day

Enalapril:
(2S)-1-[(2S)-2-[[(2S)-1-ethoxy-1-oxo-4-phenylbutan-2yl]amino]propanoyl]pyrrolidine-2-carboxylic acid
Dosage range from 5-10 mg/day to 20-40 mg/day, preferable from 10-20 mg/day to 20-40 mg/day, more preferably from 15-30 mg/day to 20-40 mg/day

Lisinopril:
(2S)-1-[(2S)-6-amino-2-[[(1S)-1-carboxy-3-phenylpropyl]amino]hexanoyl]pyrrolidine-2-carboxylic acid
Dosage range from 5-8.75 mg/day to 20-35 mg/day, preferable from 10-17.5 mg/day to 20-35 mg/day, more preferably from 15-26.25 mg/day to 20-35 mg/day

Ramipril:
(2S,3aS,6aS)-1-[(2S)-2-[[(2S)-1-ethoxy-1-oxo-4-phenylbutan-2yl] amino]propanoyl]-3,3a,4,5,6,6a-hexahydro-2H-cyclopenta[b]pyrrole-2-carboxylic acid
Dosage range from 2.5 mg/day to 10 mg/day, preferable from 5 mg/day to 10 mg/day, more preferably from 7.5 mg/day to 10 mg/day

Trandolapril:
(2S,3aR,7aS)-1-[(2S)-2-[[(2S)-1-ethoxy-1-oxo-4-phenylbutan-2-yl]amino]propanoyl]-2,3,3a,4,5,6,7,7a-octahydroindole-2-carboxylic acid
Dosage range from 1 mg/day to 4 mg/day, preferable from 2 mg/day to 4 mg/day, more preferably from 3 mg/day to 4 mg/day

Benazepril:
2-[(3S)-3-[[(2S)-1-ethoxy-1-oxo-4-phenylbutan-2-yl]amino]-2-oxo-4,5-dihydro-3H-1-benzazepin-1-yl]acetic acid
Dosage range from 2.5 mg/day to 10 mg/day, preferable from 5 mg/day to 10 mg/day, more preferably from 7.5 mg/day to 10 mg/day

Fosinopril:
(2S,4S)-4-cyclohexyl-1-[2-[(2-methyl-1-propanoyloxypropoxy)-(4-phenylbutyl)phosphoryl]acetyl]pyrrolidine-2-carboxylic acid
Dosage range from 10 mg/day to 40 mg/day, preferable from 20 mg/day to 40 mg/day, more preferably from 30 mg/day to 40 mg/day

Moexipril:
(3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid
Dosage range from 7.5 mg/day to 30 mg/day, preferable from 15 mg/day to 30 mg/day, more preferably from 22.5 mg/day to 30 mg/day

Perindopril Arginine:
(2S,3aS,7aS)-1-[(2S)-2-[[(2S)-1-ethoxy-1-oxopentan-2-yl]amino]propanoyl]-2, 3,3a,4,5,6,7,7a-octahydroindole-2-carboxylicacid;(2S)-2-amino-5-(diaminomethylideneamino) pentanoic acid
Dosage range from 2.5 mg/day to 10 mg/day, preferable from 5 mg/day to 10 mg/day, more preferably from 7.5 mg/day to 10 mg/day

Perindopril tert-butylamine:
(2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine Dosage range from 2 mg/day to 8 mg/day, preferable from 4 mg/day to 8 mg/day, more preferably from 6 mg/day to 8 mg/day

Quinapril :
(3S)-2-[(2S)-2-[[(2S)-1-ethoxy-1-oxo-4-phenylbutan-2-yl]amino]propanoyl]-3,4-dihydro-1H-isoquinoline-3-carboxylic acid
Dosage range from 10 mg/day to 40 mg/day, preferable from 20 mg/day to 40 mg/day, more preferably from 30 mg/day to 40 mg/day

Cilazapril :
(4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid
Dosage range from 1.25 mg/day to 5 mg/day, preferable from 2.5 mg/day to 5 mg/day, more preferably from 3.75 mg/day to 5 mg/day

### Beta-blockers

Bisoprolol:
1-(propan-2-ylamino)-3-[4-(2-propan-2-yloxyethoxymethyl)phenoxy]propan-2-ol Dosage range from 2.5 mg/day to 10 mg/day, preferable from 5 mg/day to 10 mg/day, more preferably from 7.5 mg/day to 10 mg/day

Carvedilol:
1-(9H-carbazol-4-yloxy)-3-[2-(2-methoxyphenoxy)ethylamino]propan-2-ol Dosage range from 12.5 mg/day to 50 mg/day, preferable from 25 mg/day to 50 mg/day , more preferably from 37.5 mg/day to 50 mg/day

Metoprolol succinate:
succinate of 1-[4-(2-methoxyethyl)phenoxy]-3-(propan-2-ylamino)propan-2-ol Dosage range from 50 mg/day to 200 mg/day, preferable from 100 mg/day to 200 mg/day, more preferably from 150 mg/day to 200 mg/day

Nebivolol:
1-(6-fluoro-3,4-dihydro-2H-chromen-2-yl)-2-[[2-(6-fluoro-3,4-dihydro-2H-chromen-2-yl)-2-hydroxyethyl]amino]ethanol
Dosage range from 2.5 mg/day to 10 mg/day, preferable from 5 mg/day to 10 mg/day, more preferably from 7.5 mg/day to 10 mg/day

Nadolol:
(2R,3S)-5-[3-(tert-butylamino)-2-hydroxypropoxy]-1,2,3,4tetrahydronaphthalene-2,3-diol Dosage range from 20 mg/day to 80 mg/day, preferable from 40 mg/day to 80 mg/day, more preferably from 60 mg/day to 80 mg/day

Acebutolol :
N-[3-acetyl-4-[2-hydroxy-3-(propan-2-ylamino)propoxy]phenyl]butanamide Dosage range from 100 mg/day to 400 mg/day, preferable from 200 mg/day to 400 mg/day, more preferably from 300 mg/day to 400 mg/day

Labetolol :
2-hydroxy-5-[1-hydroxy-2-(4-phenylbutan-2-ylamino)ethyl]benzamide Dosage range from 200 mg/day to 800 mg/day, preferable from 400 mg/day to 800 mg/day, more preferably from 600 mg/day to 800 mg/day

Celiprolol :
3-[3-acetyl-4-[3-(tert-butylamino)-2-hydroxypropoxy]phenyl]-1,1-diethylurea Dosage range from 150 mg/day to 600 mg/day, preferable from 300 mg/day to 600 mg/day, more preferably from 450 mg/day to 600 mg/day

Atenolol :
2-[4-[2-hydroxy-3-(propan-2-ylamino)propoxy]phenyl] acetamide
Dosage range from 25 mg/day to 100 mg/day, preferable from 50 mg/day to 100 mg/day, more preferably from 75 mg/day to 100 mg/day

Propranolol :
1-naphthalen-1-yloxy-3 -(propan-2-ylamino)propan-2-ol
Dosage range from 40 mg/day to 160 mg/day, preferable from 80 mg/day to 160 mg/day, more preferably from 120 mg/day to 160 mg/day

### Angiotensin Receptor Blockers (ARBs)

Candesartan:
2-ethoxy-3-[[4-[2-(2H-tetrazol-5-yl)phenyl]phenyl]methyl]benzimidazole-4-carboxylic acid Dosage range from 8 mg/day to 32 mg/day, preferable from 16 mg/day to 32 mg/day, more preferably from 24 mg/day to 32 mg/da

Valsartan:
(2S)-3-methyl-2-[pentanoyl-[[4-[2-(2H-tetrazol-5-yl)phenyl]phenyl]methyl] amino]butanoic acid Dosage range from 80 mg/day to 320 mg/day, preferable from 160 mg/day to 320 mg/day, more preferably from 240 mg/day to 320 mg/day

Losartan:
[2-butyl-5-chloro-3-[[4-[2-(2H-tetrazol-5-yl)phenyl]phenyl]methyl]imidazol-4-yl]methanol Dosage range from 37.5 mg/day to 150 mg/day, preferable from 75 mg/day to 150 mg/day, more preferably from 112.5 mg/day to 150 mg/day

### Angiotensin-Receptor-Antagonists

Azilsartan:
2-ethoxy-3-[[4-[2-(5-oxo-4H-1,2,4-oxadiazol-3-yl)phenyl]phenyl]methyl] benzimidazole-4-carboxylic acid
Dosage range from 20 mg/day to 80 mg/day, preferable from 40 mg/day to 80 mg/day, more preferably from 60 mg/day to 80 mg/day

Eprosartan:
4-[[2-butyl-5-[(E)-2-carboxy-3-thiophen-2-ylprop-1-enyl]imidazol-1-yl] methyl]benzoic acid Dosage range from 150 mg/day to 600 mg/day, preferable from 300 mg/day to 600 mg/day, more preferably from 450 mg/day to 600 mg/day

Irbesartan:
2-butyl-3-[[4-[2-(2H-tetrazol-5-yl)phenyl]phenyl]methyl]-1,3-diazaspiro[4.4]non-1-en-4-one Dosage range from 75 mg/day to 300 mg/day, preferable from 150 mg/day to 300 mg/day, more preferably from 225 mg/day to 300 mg/day

Olmesartan:
5-(2-hydroxypropan-2-yl)-2-propyl-3-[[4-[2-(2H-tetrazol-5-yl)phenyl]phenyl]methyl]imidazole-4-carboxylic acid
Dosage range from 10 mg/day to 40 mg/day, preferable from 20 mg/day to 40 mg/day, more preferably from 30 mg/day to 40 mg/day

Telmisartan:
2-[4-[[4-methyl-6-(1-methylbenzimidazol-2-yl)-2-propylbenzimidazol-1-yl]methyl]phenyl]benzoic acid
Dosage range from 20 mg/day to 80 mg/day, preferable from 40 mg/day to 80 mg/day, more preferably from 60 mg/day to 80 mg/day

### Mineraloreceptor antagonist (MRAs)

Eplerenone:
methyl(1R,2S,9R,10R,11S,14R,15S,17R)-2,15-dimethyl-5,5'-dioxospiro[18-oxapentacyclo[8.8.0.01,17.02,7.011,15]octadec-6-ene-14,2'-oxolane]-9-carboxylate Dosage range from 12.5 mg/day to 50 mg/day, preferable from 25 mg/day to 50 mg/day, more preferably from 37.5 mg/day to 50 mg/day

Spironolactone:
S-[(7R,8R,9S,10R,13S,14S,17R)-10,13-dimethyl-3,5'-dioxospiro[2,6,7,8,9,11,12,14,15,16-decahydro-1H-cyclopenta[a]phenanthrene-17,2'-oxolane]-7-yl]ethanethioate Dosage range from 12.5 mg/day to 50 mg/day, preferable from 25 mg/day to 50 mg/day, more preferably from 37.5 mg/day to 50 mg/day

### ARNI

Sacubitril/valsartan:
4-[[(2S,4R)-5-ethoxy-4-methyl-5-oxo-1-(4-phenylphenyl)pentan-2-yl]amino]-4-oxobutanoic acid / (2S)-3-methyl-2-[pentanoyl-[[4-[2-(2H-tetrazol-5-yl)phenyl] phenyl] methyl] amino]butanoic acidII
Dosage range from 48.5mg/51.5mg/day to 194mg/206mg/day, preferable from 97mg/103mg/day to 194mg/206mg/day, more preferably from 145.5mg/154.5mg/day to 194mg/206mg/day

Finerenone:
(4*S*)-4-(4-cyano-2-methoxyphenyl)-5-ethoxy-2,8-dimethyl-1,4-dihydro-1,6-naphthyridine-3-carboxamide
Dosage range from 10 mg/day to 20 mg/day, preferable 15 mg/day to 20 mg/day

### Loop-acting Diuretics

Furosemide:
4-chloro-2-(furan-2-ylmethylamino)-5-sulfamoylbenzoic acid
Dosage range from 10-60 mg/day to 40-240 mg/day, preferable from 20-120 mg/day to 40-240 mg/day, more preferably from 30-180 mg/day to 40-240 mg/day

Bumetanide:
3-(butylamino)-4-phenoxy-5-sulfamoylbenzoic acid
Dosage range from 0.25-1.25 mg/day to 1-5 mg/day, preferable from 0.5-2.5 mg/day to 1-5 mg/day, more preferably from 0.75-3.75 mg/day to 1-5 mg/day

Torasemide:
1-[4-(3-methylanilino)pyridin-3-yl]sulfonyl-3-propan-2-ylurea
Dosage range from 2.5-5 mg/day to 10-20 mg/day, preferable from 5-10 mg/day to 10-20 mg/day, more preferably from 7.5-15 mg/day to 10-20 mg/day

Bendroflumethiazide:
3-benzyl-1,1-dioxo-6-(trifluoromethyl)-3,4-dihydro-2H-1lambda6,2,4-benzothiadiazine-7-sulfonamide
Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day

Hydrochlorothiazide:
6-chloro-1,1-dioxo-3,4-dihydro-2H-1lambda6,2,4-benzothiadiazine-7-sulfonamide Dosage range from 3.125-25 mg/day to 12.5-100 mg/day, preferable from 6.25-50 mg/day to 12.5-100 mg/day, more preferably from 9.375-75 mg/day to 12.5-100 mg/day

Metolazone:
7-chloro-2-methyl-3-(2-methylphenyl)-4-oxo-1,2-dihydroquinazoline-6-sulfonamide Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day

Indapamide:
4-chloro-N-(2-methyl-2,3 -dihydroindol-1-yl)-3-sulfamoylbenzamide Dosage range from 0.625-1.25 mg/day to 2.5-5 mg/day, preferable from 1.25-2.5 mg/day to 2.5-5 mg/day, more preferably from 1.875-3.75 mg/day to 2.5-5 mg/day

### Gliflozine

Dapagliflozin:
(2S,3R,4R,5S,6R)-2-[4-chloro-3-[(4-ethoxyphenyl)methyl]phenyl]-6-(hydroxymethyl)oxane-3,4,5-triol
Dosage range from 2.5 mg/day to 10 mg/day, preferable from 5 mg/day to 10 mg/day, more preferably from 7.5 mg/day to 10 mg/day

Canagliflozin:
(2S,3R,4R,5S,6R)-2-[3-[[5-(4-fluorophenyl)thiophen-2-yl]methyl]-4-methylphenyl]-6-(hydroxymethyl)oxane-3,4,5-triol
Dosage range from 75 mg/day to 300 mg/day, preferable from 150 mg/day to 300 mg/day, more preferably from 225 mg/day to 300 mg/day

Empagliflozin :
(2S,3R,4R,5S,6R)-2-[4-chloro-3-[[4-[(3S)-oxolan-3-yl]oxyphenyl]methyl]phenyl]-6-(hydroxymethyl)oxane-3,4,5-triol
Dosage range from 6.25 mg/day to 25 mg/day, preferable from 10 mg/day to 25 mg/day, more preferably from 10 mg/day to 20 mg/day

Ertugliflozin :
(1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol
Dosage range from 3.75 mg/day to 15 mg/day, preferable from 7.5 mg/day to 15 mg/day, more preferably from 11.25 mg/day to 15 mg/day

Ipragliflozin
(2S,3R,4R,5S,6R)-2-[3-(1-benzothiophen-2-ylmethyl)-4-fluorophenyl]-6-(hydroxymethyl)oxane-3,4,5-triol
Dosage range from 25 mg/day to 100 mg/day, preferable from 50 mg/day to 100 mg/day, more preferably from 75 mg/day to 100 mg/day

Tofogliflozin
(3S,3'R,4'S,5'S,6'R)-5-[(4-ethylphenyl)methyl]-6'-(hydroxymethyl)spiro[1H-2-benzofuran-3,2'-oxane]-3',4',5'-triol
Dosage range from 5 mg/day to 20 mg/day, preferable from 10 mg/day to 20 mg/day, more preferably from 15 mg/day to 20 mg/day

Luseogliflozin
(2S,3R,4R,5S,6R)-2-[5-[(4-ethoxyphenyl)methyl]-2-methoxy-4-methylphenyl]-6-(hydroxymethyl)thiane-3,4,5-triol
Dosage range from 1.25 mg/day to 5 mg/day, preferable from 2.5 mg/day to 5m g/day, more preferably from 3.75 mg/day to 5 mg/day

Sotagliflozin :
(2S,3R,4R,5S,6R)-2-[4-chloro-3-[(4-ethoxyphenyl)methyl]phenyl]-6-methylsulfanyloxane-3,4,5-triol
Dosage range from 100 mg/day to 400 mg/day, preferable from 200mg/day to 400 mg/day, more preferably from 300 mg/day to 400 mg/day.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein a sample of bodily fluid of said patient has a level of BNP > 200 pg/mL and/or has a level NT-proBNP > 600 pg/mL, and wherein said sample of bodily fluid is selected from the group comprising blood, plasma and serum.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein a sample of bodily fluid of said patient has a level of BNP > 300 pg/mL and/or has a level NT-proBNP > 800 pg/mL, and wherein said sample of bodily fluid is selected from the group comprising blood, plasma and serum.

Another specific embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein said patient has no heart failure (HF).

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein said patient has no history of heart failure (HF).

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein the patient has no clinical signs of congestion.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein the patient has clinical signs of congestion.

Another preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein the patient has no cardiovascular or pulmonary disease.

Another preferred embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein said combination is a fixed combination, and wherein said combination is a fixed combination in one single administration form.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein the formulation is is selected from the group comprising a pill, a tablet, a liqiuid medicine, a capsule, a film tablet, a dragee.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein said formulation comprises a combination medication comprising at least two of the following compounds wherein each of said chemical compounds is a chemical entity, wherein each chemical entity is distinct from the other:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozine or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea,
e. a diuretic to reduce or prevent occurrence of signs and/or symptoms of congestion in patients,
and wherein a sample of bodily fluid of said patient has a level of BNP > 200 pg/mL and/or has a level NT-proBNP > 600 pg/mL, and/or the patient is congestive and/or has residual congestion, and wherein said sample of bodily fluid is selected from the group comprising blood, plasma and serum
wherein said patient is either
   - a patient with heart failure with left-ventricular ejection fraction (LVEF), greater and equal to 40 %, preferably 50 % or
   - a patient with no heartfailure, and
wherein the treatment is a daily administration of the combination medication, preferably once a day, and
wherein said combination is a fixed combination and wherein said combination is a fixed combination in one administration form, wherein said administration form is selected from the group comprising a pill, a tablet, a liqiuid medicine, a capsule, a film tablet, a dragee, and wherein the level of proADM and/or fragments thereof having at least 5 amino acids in a sample of bodily fluid of said patient is above a certain threshold, and wherein said sample of bodily fluid is selected from the group comprising plasma, serum, blood, and wherein said patient receives the combination medication in two different doses for a certain period of time, respectively,, which are named dose No 1 and dose No 2, and wherein said patient receives a dose No 1 of said combination medication for up to 2 to 4 weeks and thereafter a dose No 2 for at least 90 days, and
wherein dose No 1 is one quarter dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein said formulation comprises a combination medication comprising at least two of the following compounds wherein each of said chemical compounds is a chemical entity, wherein each chemical entity is distinct from the other:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozine or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea,
e. a diuretic to reduce or prevent occurrence of signs and/or symptoms of congestion in patients,
and wherein a sample of bodily fluid of said patient has a level of BNP > 300 pg/mL and/or has a level NT-proBNP > 800 pg/mL, and/or the patient is congestive and/or has residual congestion, and wherein said sample of bodily fluid is selected from the group comprising blood, plasma and serum,
wherein said patient is
   - a patient with heart failure with left-ventricular ejection fraction (LVEF) greater or equal to 40 %, preferably 50 % or
   - a patient with no heart failure, and
wherein the treatment is a daily administration of the combination medication, preferably once a day, and
wherein said combination is a fixed combination and wherein said combination is a fixed combination in one single administration form, wherein said administration form is selected from the group comprising a pill, a tablet, a liqiuid medicine, a capsule, a film tablet, a dragee, and
wherein the level of proADM and/or fragments thereof having at least 5 amino acids in a sample of bodily fluid of said patient is above a certain threshold, and wherein said sample of bodily fluid is selected from the group comprising plasma, serum, blood, and wherein said patient receives the combination medication in two different doses for a certain period of time, respectively, which are named dose No 1 and dose No 2, and wherein said patient receives a dose No 1 of said combination medication for up to 2 to 4 weeks and thereafter a dose No 2 for at least 90 days, and
wherein dose No 1 is one quarter dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein said formulation comprises a combination medication comprising at least two of the following compounds wherein each of said chemical compounds is a chemical entity, wherein each chemical entity is distinct from the other two :
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozine or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea,
e. a diuretic,
and wherein a sample of bodily fluid of said patient has a level of BNP > 200 pg/mL and/or has a level NT-proBNP > 600 pg/mL, and/or the patient is congestive and/or has residual congestion and wherein said sample of bodily fluid is selected from the group comprising blood, plasma and serum, and
wherein the treatment is a daily administration of the combination medication,preferably once a day, and
wherein the patient has no heart failure but has another cardiovascular disease and/or another disease with non cardiac cause which maybe selected from the group comprising diabetes and kidney disease, and
wherein said combination is a fixed combination and wherein said combination is a fixed combination in one administration form, wherein said administration form is selected from the group comprising a pill, a tablet, a liqiuid medicine, a capsule, a film tablet, a dragee, and wherein the level of proADM and/or fragments thereof having at least 5 amino acids in a sample of bodily fluid of said patient is above a certain threshold, wherein said sample of bodily fluid is selected from the group comprising plasma, serum, blood, and wherein said patient receives a dose No 1 of said combination medication for up to 2 to 4 weeks and thereafter a dose No 2 for at least 90 days, wherein dose No 1 is half dose (25%) of dose No 2, more preferably .dose No 1 is 35% of dose No 2, more preferably .dose No 1 is 40% of dose No 2, more preferably .dose No 1 is 45% of dose No 2, more preferably dose No 1 is 50% of dose No 2.

Another embodiment of the present application relates to a method of treatment of acute and/or persistent dyspnea for restoring the respiration rate and/or maintaining the respiration rate and/or restoring and/or maintaining the oxygen saturation and/or for ameliorating the symptoms of dyspnea in a patient, the method comprising administering an oral pharmaceutical formulation, wherein said formulation comprises a combination medication comprising at least two of the following compounds wherein each of said chemical compounds is a chemical entity, wherein each chemical entity is distinct from the other two :
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozine or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea,
e. a diuretic to reduce or prevent occurrence of signs and/or symptoms of congestion in patients,
and wherein a sample of bodily fluid of said patient has a level of BNP > 300 pg/mL and/or has a level NT-proBNP > 800 pg/mL, and/or the patient is congestive and/or has residual congestion and wherein said sample of bodily fluid is selected from the group comprising blood, plasma and serum, and
wherein the treatment is a daily administration of the combination medication,preferably once a day, and
wherein the patient has no heart failure but has another cardiovascular disease and/or another disease with non cardiac cause which maybe selected from the group comprising diabetes and kidney disease, and
wherein said combination is a fixed combination and wherein said combination is a fixed combination in one single administration form, wherein said administration form is selected from the group comprising a pill, a tablet, a liqiuid medicine, a capsule, a film tablet, a dragee, and
wherein the level of proADM and/or fragments thereof having at least 5 amino acids in a sample of bodily fluid of said patient is above a certain threshold, wherein said sample of bodily fluid is selected from the group comprising plasma, serum, blood, and wherein said patient receives a dose No 1 of said combination medication for up to 2 to 4 weeks and thereafter a dose No 2 for at least 90 days, wherein dose No 1 is one quarter dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

Subject matter of the present application is also combination medication for use in the treatment of a patient having acute and/or persistent dyspnea for restoring the respiration rate and/or oxygen saturation toward normal values and/or maintaining the respiration rate and/or restoring and/or maintaining the oxygen saturation and/or for ameliorating the symptoms of dyspnea comprising at least three of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozin or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea;
e. a diuretic to reduce or prevent occurrence of signs and/or symptoms of congestion in patients,
   and wherein a sample of bodily fluid of said patient has a level of Brain Natriuretic peptide (BNP) > 200 pg/mL and/or has a level of N-terminal (NT)-pro hormone BNP (NT-proBNP) > 600 pg/mL and/or the patient is congestive and/or has residual congestion, and wherein said sample of bodily fluid is selected from the group comprising blood, plasma and serum;
   and wherein said patient is either
      - a patient with heart failure with left ventricular ejection fraction (LVEF) with less than 40 %, or
      - a patient with no heart failure.

With the above context, the following consecutively numbered embodiments provide further specific aspects of the invention:
1. Combination medication for use in the treatment of a patient having acute and/or persistent dyspnea comprising at least two of the following components:
   a. a beta blocker (BB),
   b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and angiotensin receptor-neprilysin inhibitor (ARNi)
   c. and mineralo receptor antagonist (MRA),
   d. gliflozine,
   e. a diuretic
   and wherein a sample of bodily fluid of said patient has a level of BNP > 200 pg/mL and/or has a level NT-proBNP > 600 pg/mL, and wherein said sample of bodily fluid is selected from the group comprising blood, plasma and serum.
2. Combination medication for use in the treatment of a patient having acute and/or persistent dyspnea according to embodiment 1, wherein said combination medication comprises at least two of said components which are RAASi and BB, preferably said combination medication comprises at least three of said components which are RAASi, BB and MRAs, more preferably said combination medication comprises at least three of said components and gliflozine.
3. Combination medication for use in the treatment of a patient having acute and/or persistent dyspnea in a patient according to embodiment 1 or 2, wherein said patient is a patient with heart failure with mildly reduced or preserved left-ventricular ejection fraction (LVEF) or a patient with no heart-failure.
4. Combination medication for use in the treatment of a patient having acute and/or persistent dyspnea in a patient according to embodiments 1 to 3, wherein said patient is a patient with heart failure and wherein said patient has a mildly reduced with more than or equal to 40%.
5. Combination medication for use in the treatment of a patient having acute and/or persistent dyspnea in a patient according any of embodiments 1-4, wherein said patient has no heart failure (HF).
6. Combination medication for use in the treatment of a patient having acute and/or persistent dyspnea in a patient according any of embodiments 1 to 5, wherein said patient has no history of heart failure.
7. Combination medication for use in the treatment of a patient having acute and/or persistent dyspnea in a patient according any of embodiments 1 to 6, wherein the patient has no clinical signs of congestion.
8. Combination medication for use in the treatment of a patient having acute and/or persistent dyspnea in a patient according any of embodiments 1 to 7, wherein the patient has no cardiovascular or pulmonary disease.
9. Combination medication for use in the treatment of a patient having acute and/or persistent dyspnea in a patient according any of embodiments 1 to 8, wherein said combination is a fixed combination in one administration form.
10. Combination medication for use in the treatment of a patient having acute and/or persistent dyspnea in a patient according any of embodiments 1 to 9, wherein said combination is a fixed combination and wherein said combination is a fixed combination in one administration form, wherein said administration form is selected from the group comprising a pill, a tablet, a liqiuid medicine, a capsule, a film tablet, a dragee.
11. Combination medication for use in the treatment of a patient having acute and/or persistent dyspnea in a patient according any of embodiments 1 to 10, wherein the level of proADM and/or fragments thereof having at least 5 amino acids in a sample of bodily fluid of said patient is above a certain threshold.
12. Combination medication for use in the treatment of a patient having acute and/or persistent dyspnea in a patient according any of embodiments 1 to 11, wherein the level of proADM and/or fragments thereof having at least 5 amino acids in a sample of bodily fluid of said patient is above a certain threshold, wherein said sample of bodily fluid is selected from the group comprising plasma, serum, blood.
13. Combination medication for use in the treatment of a patient having acute and/or persistent dyspnea in a patient according embodiment 11 to 12, wherein Pro-Adrenomedullin or fragments thereof of at least 5 amino acids is used as a marker for congestion comprising:
   ∘ Determining the level of Pro-Adrenomedullin or fragments thereof of at least 5 amino acids in a bodily fluid obtained from said subject; and
   ∘ Correlating said level of Pro-Adrenomedullin or fragments thereof with the extent of congestion in said subject or diagnosing congestion, wherein an elevated level above a certain threshold is indicative of congestion or the extent of congestion,
   wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH2 according to SEQ ID No.: 4 or ADM-Gly according to SEQ ID No.: 5 or CT-proADM according to SEQ ID No.: 6, and wherein said patient has no congestion, i.e. the level of proADM and/or fragments thereof having at least 5 amino acids in a sample of bodily fluid of said patient is above a certain threshold.
14. Combination medication for use in the treatment of a patient having acute and/or persistent dyspnea in a patient according to embodiment 13, wherein said threshold is within a threshold range that is a threshold range for plasma mature ADM-NH2 according to SEQ ID No.: 4 between 50 and 100 pg/ml and for plasma MR-proADM between 0.5 and 1.5 nmol/L, and for plasma CT-proADM between 85 and 350 pmol/L.
15. Combination medication for use in the treatment of a patient having acute and/or persistent dyspnea in a patient according any of embodiments 11 to 14, wherein an assay is used for determining the level of proADM and/or fragments thereof in a sample of bodily fluid of said patient, wherein the assay sensitivity of said assay is able to quantify the proADM of healthy subjects and is < 70 pg/ml, preferably < 40 pg/ml and more preferably < 10 pg/ml.
16. Combination medication for use in the treatment of a patient having acute and/or persistent dyspnea in a patient according any of embodiments 11 - 15 wherein in a sample of bodily fluid of said patient the level of mature ADM-NH2 and/or fragments thereof having at least 5 amino acids is determined, and wherein said level of mature ADM-NH2 and/or fragments thereof is correlated with the extent of congestion in said subject or diagnosing congestion, wherein the threshold is within a threshold range between 50 and 100 pg/ml.
17. Combination medication for use in the treatment of a patient having acute and/or persistent dyspnea in a patient according any of embodiments 11 - 16, wherein an assay is used for determining the level of mature ADM-NH2 and/or fragments thereof having at least 5 amino acids in a sample of bodily fluid of said patient.
18. Combination medication for use in the treatment of a patient having acute and/or persistent dyspnea in a patient according any of embodiments 11 - 17, wherein an assay is used for determining the level of mature ADM-NH2 and/or fragments thereof having at least 5 amino acids in a sample of bodily fluid of said patient, wherein the assay sensitivity of said assay is able to quantify the mature ADM-NH2 of healthy subjects and is < 70 pg/ml, preferably < 40 pg/ml and more preferably < 10 pg/ml.
19. Combination medication for use in the treatment of a patient having acute and/or persistent dyspnea in a patient according any of embodiments 11 - 18, wherein in a sample of bodily fluid of said patient the level of CT-proADM and/or fragments thereof having at least 5 amino acids is determined, and wherein said level of CT-proADM and/or fragments thereof is correlated with the extent of congestion in said subject or diagnosing congestion, wherein the threshold is within a threshold range between 85 and 350 pmol/L.
20. Combination medication for use in the treatment of a patient having acute and/or persistent dyspnea in a patient according any of embodiments 11 - 19, wherein an assay is used for determining the level of CT-proADM and/or fragments thereof having at least 5 amino acids in a sample of bodily fluid of said patient.
21. Combination medication for use in the treatment of a patient having acute and/or persistent dyspnea in a patient according any of embodiments 11 - 20, wherein an assay is used for determining the level of CT-proADM and/or fragments thereof having at least 5 amino acids in a sample of bodily fluid of said patient, wherein the assay sensitivity of said assay is able to quantify the CT-proADM of healthy subjects and is < 100 pmol/L, preferably < 75 pmol/L and more preferably < 50 pmol/L.
22. Combination medication for use in the treatment of a patient having acute and/or persistent dyspnea in a patient according any of embodiments 11 - 21, wherein in a sample of bodily fluid of said patient the level of MR-proADM and/or fragments thereof having at least 5 amino acids is determined, and wherein said level of MR-proADM and/or fragments thereof is correlated with the extent of congestion in said subject or diagnosing congestion, wherein the threshold is within a threshold range between 0.5 and 1.5 nmol/L.
23. Combination medication for use in the treatment of a patient having acute and/or persistent dyspnea according any of embodiments 11 - 22, wherein an assay is used for determining the level of MR-proADM and/or fragments thereof having at least 5 amino acids in a sample of bodily fluid of said patient.
24. Combination medication for use in the treatment of a patient having acute and/or persistent dyspnea according any of embodiments 11 - 23, wherein an assay is used for determining the level of MR-proADM and/or fragments thereof having at least 5 amino acids in a sample of bodily fluid of said patient, wherein the assay sensitivity of said assay is able to quantify the MR-proADM of healthy subjects and is < 0,5 nmol/L, preferably < 0,4npmol/L and more preferably < 0,2 nmol/L.
25. Combination medication for use in the treatment of a patient having acute and/or persistent dyspnea according any of embodiments 11 to 24, wherein the agent according to group b. (iii) of said combination medication according to embodiment 1 is a combination of angiotensin II inhibitor and neprilysin inhibitor (ARNi), wherein in particular the ARNi is a combination of
   ∘ the neprilysin inhibitor 4-[[(2S,4R)-5-ethoxy-4-methyl-5-oxo-1-(4-phenylphenyl)pentan-2-yl]amino]-4-oxobutanoic acid, and
   ∘ the nonpeptide triazole-derived antagonist of angiotensin (AT) II (2S)-3-methyl-2-[pentanoyl-[[4-[2-(2H-tetrazol-5-yl)phenyl]phenyl]methyl]amino]butanoic acid II,
   ▪ preferably a fixed combination.
26. Combination medication for use in the treatment of a patient having acute and/or persistent dyspnea according any of embodiments 1 to 25, wherein said combination medication comprises
   a. a beta blocker, and
   b. one agent of the group consisting of (i) an angiotensin-converting enzyme (ACE) inhibitor, (ii) an angiotensin II inhibitor and (iii) a combination of angiotensin II inhibitor and neprilysin inhibitor (ARNi)
   c. and mineralo receptor antagonist (MRA), and
   d. gliflozine (e.g canagliflozine, empaglifozine, dapaglifozine),
   preferably in a fixed combination
27. Combination medication for use in the treatment of a patient having acute and/or persistent dyspnea according any of embodiments 1 to 26, wherein said combination medication comprises in addition diuretics, preferably in a fixed combination.
28. Combination medication for use in the treatment of a patient having acute and/or persistent dyspnea according any of embodiments 1 to 27, wherein said patient receives the combination medication in two different doses, which are named dose No 1 and dose No 2.
29. Combination medication for use in the treatment of a patient having acute and/or persistent dyspnea according any of embodiments 1 to 28, wherein said patient receives a dose No 1 of said combination medication for up to 2 to 4 weeks and thereafter a dose No 2 for at least 90 days, wherein dose No 1 is half dose (250%) of dose No 2, more preferably .dose No 1 is 30% of dose No 2, more preferably .dose No 1 is 35% of dose No 2, more preferably .dose No 1 is 40% of dose No 2, more preferably .dose No 1 is 45% of dose No 2, more preferably .dose No 1 is 50% of dose No 2..
30. Combination medication for use in the treatment of a patient having acute and/or persistent dyspnea according any of embodiments 1 - 29, wherein said fixed or non-fixed combination medication is administered orally, preferably a fixed combination.
31. Combination medication for use in the treatment of a patient having acute and/or persistent dyspnea according any of embodiments 1 to 30, wherein a beta blocker is selected from the group comprising beta-1 adrenergic receptor antagonist 1-(propan-2-ylamino)-3-[4-(2-propan-2-yloxyethoxymethyl)phenoxy]propan-2-ol, beta-adrenoceptor blocking agent 1-(9*H*-carbazol-4-yloxy)-3-[2-(2-methoxyphenoxy)ethylamino]propan-2-ol, succinate of 1-[4-(2-methoxyethyl)phenoxy]-3-(propan-2-ylamino)propan-2-ol (CR/XL), the beta-1 adrenergic receptor antagonist 1-(6-fluoro-3,4-dihydro-2*H*-chromen-2-yl)-2-[[2-(6-fluoro-3,4-dihydro-2*H*-chromen-2-yl)-2-hydroxyethyl]amino]ethanol, (2R,3S)-5-[3-(tert-butylamino)-2-hydroxypropoxy]-1,2,3,4tetrahydronaphthalene-2,3-diol, N-[3-acetyl-4-[2-hydroxy-3-(propan-2-ylamino)propoxy]phenyl]butanamide, 2-hydroxy-5-[1-hydroxy-2-(4-phenylbutan-2-ylamino)ethyl]benzamide, 3-[3-acetyl-4-[3-(tert-butylamino)-2-hydroxypropoxy]phenyl]-1,1-diethylurea, 2-[4-[2-hydroxy-3-(propan-2-ylamino)propoxy]phenyl]acetamide, 1-naphthalen-1-yloxy-3-(propan-2-ylamino)propan-2-ol.
32. Combination medication for use in the treatment of a patient having acute and/or persistent dyspnea according any of embodiments 1 to 31, wherein the ACE inhibitor is selected from the group comprising the sulfhydryl-containing analog of proline (2*S*)-1-[(2*S*)-2-methyl-3-sulfanylpropanoyl]pyrrolidine-2-carboxylic acid, (2*S*)-1-[(2*S*)-2-[[(2*S*)-1-ethoxy-1-oxo-4-phenylbutan-2-yl]amino]propanoyl]pyrrolidine-2-carboxylic acid, (2*S*)-1-[(2*S*)-6-amino-2-[[(1*S*)-1-carboxy-3-phenylpropyl]amino]hexanoyl]pyrrolidine-2-carboxylic acid, (2*S*,3*aS*,6*aS*)-1-[(2*S*)-2-[[(2*S*)-1-ethoxy-1-oxo-4-phenylbutan-2-yl]amino]propanoyl]-3,3*a*,4,5,6,6*a-*hexahydro-2*H*-cyclopenta[b]pyrrole-2-carboxylic acid, (2*S*,3*aR*,7*aS*)-1-[(2*S*)-2-[[(2*S*)-1-ethoxy-1-oxo-4-phenylbutan-2-yl]amino]propanoyl]-2,3,3*a*,4,5,6,7,7*a-*octahydroindole-2-carboxylic acid, 2-[(3S)-3-[[(2S)-1-ethoxy-1-oxo-4-phenylbutan-2-yl]amino]-2-oxo-4,5-dihydro-3H-1-benzazepin-1-yl]acetic acid, (2S,4S)-4-cyclohexyl-1-[2-[(2-methyl-1-propanoyloxypropoxy)-(4-phenylbutyl)phosphoryl]acetyl]pyrrolidine-2-carboxylic acid, (3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid, (2S,3aS,7aS)-1-[(2S)-2-[[(2S)-1-ethoxy-1-oxopentan-2-yl]amino]propanoyl]-2, 3,3a,4,5,6,7,7a-octahydroindole-2-carboxylicacid;(2S)-2-amino-5-(diaminomethylideneamino) pentanoic acid, (2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine, (3S)-2-[(2S)-2-[[(2S)-1-ethoxy-1-oxo-4-phenylbutan-2-yl]amino]propanoyl]-3,4-dihydro-1H-isoquinoline-3-carboxylic acid, (4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid.
33. Combination medication for use in the treatment of a patient having acute and/or persistent dyspnea according any of embodiments 1 to 32, wherein the MRAs (mineralo receptor antagonist) is selected from the group comprising methyl (1R,2S,9R,10R,11S,14R,15S,17R)-2,15-dimethyl-5,5'-dioxospiro[18-oxapentacyclo[8.8.0.01,17.02,7.011,15]octadec-6-ene-14,2'-oxolane]-9-carboxylate, S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate.
34. Combination medication for use in the treatment of a patient having acute and/or persistent dyspnea according any of embodiments 1 to 33, wherein the diurectic is selected from the group comprising loop-acting diuretics such as 3-(butylamino)-4-phenoxy-5-sulfamoylbenzoic acid, 2-[2,3-dichloro-4-(2-methylidenebutanoyl)phenoxy] acetic acid, 4-chloro-2-(furan-2-ylmethylamino)-5- sulfamoylbenzoic acid, 1-[4-(3-methylanilino)pyridin-3-yl]sulfonyl-3-propan-2-ylurea, potassium-sparing diuretics such as 3,5-diamino-6-chloro-*N-*(diaminomethylidene)pyrazine-2-carboxamide,, methyl (1*R*,2*S*,9*R*,10*R*,11*S*,14*R*,15*S*,17*R*)-2,15-dimethyl-5,5'-dioxospiro[18-oxapentacyclo[8.8.0.0^{1,17}.0^{2,7}.0^{11,15}]octadec-6-ene-14,2'-oxolane]-9-carboxylate, *S-*[(7*R*,8*R*,9*S*,10*R*,13*S*,14*S*,17*R*)-10,13-dimethyl-3,5'-dioxospiro[2,6,7,8,9,11,12,14,15,16-decahydro-1*H*-cyclopenta[a]phenanthrene-17,2'-oxolane]-7-yl]ethanethioate, 6-phenylpteridine-2,4,7-triamine, thiazide diuretics such as 6-chloro-1,1-dioxo-4*H*-1λ⁶,2,4-benzothiadiazine-7-sulfonamide, 2-chloro-5-(1-hydroxy-3-oxo-2*H*-isoindol-1-yl)benzenesulfonamide, 6-chloro-1,1-dioxo-3,4-dihydro-2H-1lambda6,2,4-benzothiadiazine-7-sulfonamide, 4-chloro-N-(2-methyl-2,3-dihydroindol-1-yl)-3-sulfamoylbenzamide, 7-chloro-2-methyl-3-(2-methylphenyl)-4-oxo-1,2-dihydroquinazoline-6-sulfonamide, 7-chloro-2-methyl-3-(2-methylphenyl)-4-oxo-1,2-dihydroquinazoline-6-sulfonamide and/or mixtures thereof.
35. Combination medication for use in the treatment of a patient having acute and/or persistent dyspnea according any of embodiments 1 to 34, wherein the gliflozine is selected from the group comprising (2S,3R,4R,5S,6R)-2-[3-[[5-(4-fluorophenyl)thiophen-2-yl]methyl]-4-methylphenyl]-6-(hydroxymethyl)oxane-3,4,5-triol, (2S,3R,4R,5S,6R)-2-[4-chloro-3-[(4-ethoxyphenyl)methyl]phenyl]-6-(hydroxymethyl)oxane-3,4,5-triol, (2S,3R,4R,5S,6R)-2-[4-chloro-3-[[4-[(3S)-oxolan-3-yl]oxyphenyl]methyl]phenyl]-6-(hydroxymethyl)oxane-3,4,5-triol, (1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol, Luseogliflozin, Remogliflozin etabonate (ethyl[(2R,3S,4S,5R,6S)-3,4,5-trihydroxy-6-[5-methyl-1-propan-2-yl-4-[(4-propan-2-yloxyphenyl)methyl]pyrazol-3-yl]oxyoxan-2-yl]methyl carbonate), sergliflozin etabonate (ethyl [(2R,3S,4S,5R,6S)-3,4,5-trihydroxy-6-[2-[(4-methoxyphenyl)methyl]phenoxy]oxan-2-yl]methyl carbonate), (2S,3R,4R,5S,6R)-2-[3-(1-benzothiophen-2-ylmethyl)-4-fluorophenyl]-6-(hydroxymethyl)oxane-3,4,5-triol, (3S,3'R,4'S,5'S,6'R)-5-[(4-ethylphenyl)methyl]-6'-(hydroxymethyl)spiro[1H-2-benzofuran-3,2'-oxane]-3',4',5'-triol, (2S,3R,4R,5S,6R)-2-[5-[(4-ethoxyphenyl)methyl]-2-methoxy-4-methylphenyl]-6-(hydroxymethyl)thiane-3,4,5-triol, (2S,3R,4R,5S,6R)-2-[4-chloro-3-[(4-ethoxyphenyl
36. Combination medication for use in the treatment of a patient having acute and/or persistent dyspnea according any of embodiments 1-35, wherein the dose No. 2 of the following agents are as follows:

### ACE-inhibitors

Captopril:
(2S)-1-[(2S)-2-methyl-3-sulfanylpropanoyl]pyrrolidine-2-carboxylic acid Dosage range from 37.5 mg/day to 150 mg/day, preferable from 75 mg/day mg to 150 mg/day, more preferably from 112.5 mg/day to 150 mg/day

Enalapril:
(2S)-1-[(2S)-2-[[(2S)-1-ethoxy-1-oxo-4-phenylbutan-2yl]amino]propanoyl] pyrrolidine-2-carboxylic acid
Dosage range from 5-10 mg/day to 20-40 mg/day, preferable from 10-20 mg/day to 20-40 mg/day, more preferably from 15-30 mg/day to 20-40 mg/day

Lisinopril:
(2S)-1-[(2S)-6-amino-2-[[(1S)-1-carboxy-3-phenylpropyl]amino] hexanoyl]pyrrolidine-2-carboxylic acid
Dosage range from 5-8.75 mg/day to 20-35 mg/day, preferable from 10-17.5 mg/day to 20-35 mg/day, more preferably from 15-26.25 mg/day to 20-35 mg/day

Ramipril:
(2S,3aS,6aS)-1-[(2S)-2-[[(2S)-1-ethoxy-1-oxo-4-phenylbutan-2yl]amino]propanoyl]-3,3a,4,5,6,6a-hexahydro-2H-cyclopenta[b]pyrrole-2-carboxylic acid Dosage range from 2.5 mg/day to 10 mg/day, preferable from 5 mg/day to 10 mg/day, more preferably from 7.5 mg/day to 10 mg/day

Trandolapril:
(2S,3aR,7aS)-1-[(2S)-2-[[(2S)-1-ethoxy-1-oxo-4-phenylbutan-2-yl]amino]propanoyl]-2,3,3a,4,5,6,7,7a-octahydroindole-2-carboxylic acid
Dosage range from 1 mg/day to 4 mg/day, preferable from 2 mg/day to 4 mg/day, more preferably from 3 mg/day to 4 mg/day

Benazepril:
2-[(3S)-3-[[(2S)-1-ethoxy-1-oxo-4-phenylbutan-2-yl]amino]-2-oxo-4,5-dihydro-3H-1-benzazepin-1-yl]acetic acid
Dosage range from 2.5 mg/day to 10 mg/day, preferable from 5 mg/day to 10 mg/day, more preferably from 7.5 mg/day to 10 mg/day

Fosinopril:
(2S,4S)-4-cyclohexyl-1-[2-[(2-methyl-1-propanoyloxypropoxy)-(4-phenylbutyl)phosphoryl]acetyl]pyrrolidine-2-carboxylic acid
Dosage range from 10 mg/day to 40 mg/day, preferable from 20 mg/day to 40 mg/day, more preferably from 30 mg/day to 40 mg/day

Moexipril:
(3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid
Dosage range from 7.5 mg/day to 30 mg/day, preferable from 15 mg/day to 30 mg/day, more preferably from 22.5 mg/day to 30 mg/day

Perindopril Arginine:
(2S,3aS,7aS)-1-[(2S)-2-[[(2S)-1-ethoxy-1-oxopentan-2-yl]amino]propanoyl]-2, 3,3a,4,5,6,7,7a-octahydroindole-2-carboxylicacid;(2S)-2-amino-5-(diaminomethylideneamino) pentanoic acid
Dosage range from 2.5 mg/day to 10 mg/day, preferable from 5 mg/day to 10 mg/day, more preferably from 7.5 mg/day to 10 mg/day

Perindopril tert-butylamine:
(2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine Dosage range from 2 mg/day to 8 mg/day, preferable from 4 mg/day to 8 mg/day, more preferably from 6 mg/day to 8 mg/day

Quinapril :
(3S)-2-[(2S)-2-[[(2S)-1-ethoxy-1-oxo-4-phenylbutan-2-yl]amino]propanoyl]-3,4-dihydro-1H-isoquinoline-3-carboxylic acid
Dosage range from 10 mg/day to 40 mg/day, preferable from 20 mg/day to 40 mg/day, more preferably from 30 mg/day to 40 mg/day

Cilazapril :
(4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid
Dosage range from 1.25 mg/day to 5 mg/day, preferable from 2.5 mg/day to 5 mg/day, more preferably from 3.75 mg/day to 5 mg/day

### Beta-blockers

Bisoprolol:
1-(propan-2-ylamino)-3-[4-(2-propan-2-yloxyethoxymethyl)phenoxy]propan-2-ol Dosage range from 2.5 mg/day to 10 mg/day, preferable from 5 mg/day to 10 mg/day, more preferably from 7.5 mg/day to 10 mg/day

Carvedilol:
1-(9H-carbazol-4-yloxy)-3-[2-(2-methoxyphenoxy)ethylamino]propan-2-ol Dosage range from 12.5 mg/day to 50 mg/day, preferable from 25 mg/day to 50 mg/day , more preferably from 37.5 mg/day to 50 mg/day

Metoprolol succinate:
succinate of 1-[4-(2-methoxyethyl)phenoxy]-3-(propan-2-ylamino)propan-2-ol Dosage range from 50 mg/day to 200 mg/day, preferable from 100 mg/day to 200 mg/day, more preferably from 150 mg/day to 200 mg/day

Nebivolol:
1-(6-fluoro-3,4-dihydro-2H-chromen-2-yl)-2-[[2-(6-fluoro-3,4-dihydro-2H-chromen-2-yl)-2-hydroxyethyl]amino]ethanol
Dosage range from 2.5 mg/day to 10 mg/day, preferable from 5 mg/day to 10 mg/day, more preferably from 7.5 mg/day to 10 mg/day

Nadolol:
(2R,3S)-5-[3-(tert-butylamino)-2-hydroxypropoxy]-1,2,3,4tetrahydronaphthalene-2,3-diol
Dosage range from 20 mg/day to 80 mg/day, preferable from 40 mg/day to 80 mg/day, more preferably from 60 mg/day to 80 mg/day

Acebutolol :
N-[3-acetyl-4-[2-hydroxy-3-(propan-2-ylamino)propoxy]phenyl]butanamide Dosage range from 100 mg/day to 400 mg/day, preferable from 200 mg/day to 400 mg/day, more preferably from 300 mg/day to 400 mg/day

Labetolol :
2-hydroxy-5-[1-hydroxy-2-(4-phenylbutan-2-ylamino)ethyl]benzamide Dosage range from 200 mg/day to 800 mg/day, preferable from 400 mg/day to 800 mg/day, more preferably from 600 mg/day to 800 mg/day

Celiprolol :
3-[3-acetyl-4-[3-(tert-butylamino)-2-hydroxypropoxy]phenyl]-1,1-diethylurea Dosage range from 150 mg/day to 600 mg/day, preferable from 300 mg/day to 600 mg/day, more preferably from 450 mg/day to 600 mg/day

Atenolol :
2-[4-[2-hydroxy-3-(propan-2-ylamino)propoxy]phenyl]acetamide Dosage range from 25 mg/day to 100 mg/day, preferable from 50 mg/day to 100 mg/day, more preferably from 75 mg/day to 100 mg/day

Propranolol :
1-naphthalen-1-yloxy-3-(propan-2-ylamino)propan-2-ol Dosage range from 40 mg/day to 160 mg/day, preferable from 80 mg/day to 160 mg/day, more preferably from 120 mg/day to 160 mg/day

### ARBs

Candesartan:
2-ethoxy-3-[[4-[2-(2H-tetrazol-5-yl)phenyl]phenyl]methyl]benzimidazole-4-carboxylic acid
Dosage range from 8 mg/day to 32 mg/day, preferable from 16 mg/day to 32 mg/day, more preferably from 24 mg/day to 32 mg/da

Valsartan:
(2S)-3-methyl-2-[pentanoyl-[[4-[2-(2H-tetrazol-5-yl)phenyl]phenyl]methyl] amino]butanoic acid
Dosage range from 80 mg/day to 320 mg/day, preferable from 160 mg/day to 320 mg/day, more preferably from 240 mg/day to 320 mg/day

Losartan:
[2-butyl-5-chloro-3-[[4-[2-(2H-tetrazol-5-yl)phenyl]phenyl]methyl]imidazol-4-yl]methanol
Dosage range from 37.5 mg/day to 150 mg/day, preferable from 75 mg/day to 150 mg/day, more preferably from 112.5 mg/day to 150 mg/day

### Angiotensin-Receptor-Antagonists

Azilsartan:
2-ethoxy-3-[[4-[2-(5-oxo-4H-1,2,4-oxadiazol-3-yl)phenyl]phenyl]methyl] benzimidazole-4-carboxylic acid
Dosage range from 20 mg/day to 80 mg/day, preferable from 40 mg/day to 80 mg/day, more preferably from 60 mg/day to 80 mg/day

Eprosartan:
4-[[2-butyl-5-[(E)-2-carboxy-3-thiophen-2-ylprop-1-enyl]imidazol-1-yl] methyl]benzoic acid
Dosage range from 150 mg/day to 600 mg/day, preferable from 300 mg/day to 600 mg/day, more preferably from 450 mg/day to 600 mg/day

Irbesartan:
2-butyl-3-[[4-[2-(2H-tetrazol-5-yl)phenyl]phenyl]methyl]-1,3-diazaspiro[4.4]non-1-en-4-one
Dosage range from 75 mg/day to 300 mg/day, preferable from 150 mg/day to 300 mg/day, more preferably from 225 mg/day to 300 mg/day

Olmesartan:
5-(2-hydroxypropan-2-yl)-2-propyl-3-[[4-[2-(2H-tetrazol-5-yl)phenyl]phenyl]methyl]imidazole-4-carboxylic acid
Dosage range from 10 mg/day to 40 mg/day, preferable from 20 mg/day to 40 mg/day, more preferably from 30 mg/day to 40 mg/day

Telmisartan:
2-[4-[[4-methyl-6-(1-methylbenzimidazol-2-yl)-2-propylbenzimidazol-1-yl]methyl]phenyl]benzoic acid
Dosage range from 20 mg/day to 80 mg/day, preferable from 40 mg/day to 80 mg/day, more preferably from 60 mg/day to 80 mg/day

### Mineraloreceptor antagonist (MRAs)

Eplerenone:
methyl(1R,2S,9R,10R,11S,14R,15S,17R)-2,15-dimethyl-5,5'-dioxospiro[18-oxapentacyclo[8.8.0.01,17.02,7.011,15]octadec-6-ene-14,2'-oxolane]-9-carboxylate Dosage range from 12.5 mg/day to 50 mg/day, preferable from 25 mg/day to 50 mg/day, more preferably from 37.5 mg/day to 50 mg/day

Spironolactone:
S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate
Dosage range from 12.5 mg/day to 50 mg/day, preferable from 25 mg/day to 50 mg/day, more preferably from 37.5 mg/day to 50 mg/day

### ARNI

Sacubitril/valsartan:
4-[[(2S,4R)-5-ethoxy-4-methyl-5-oxo-1-(4-phenylphenyl)pentan-2-yl]amino]-4-oxobutanoic acid / (2S)-3-methyl-2-[pentanoyl-[[4-[2-(2H-tetrazol-5-yl)phenyl] phenyl] methyl] amino]butanoic acidII
Dosage range from 48.5mg/51.5mg/day to 194mg/206mg/day, preferable from 97mg/103mg/day to 194mg/206mg/day, more preferably from 145.5mg/154.5mg/day to 194mg/206mg/day

### If-channel blocker

Ivabradine:
3-[3-[[(7S)-3,4-dimethoxy-7-bicyclo[4.2.0]octa-1,3,5-trienyl]methyl-methylamino]propyl]-7,8-dimethoxy-2,5-dihydro-1H-3-benzazepin-4-one Dosage range from 3.75 mg/day to 15 mg/day, preferable from 7.5 mg/day to 15 mg/day, more preferably from 11.25 mg/day to 15 mg/day

### Diuretics

Furosemide:
4-chloro-2-(furan-2-ylmethylamino)-5-sulfamoylbenzoic acid
Dosage range from 10-60 mg/day to 40-240 mg/day, preferable from 20-120 mg/day to 40-240 mg/day, more preferably from 30-180 mg/day to 40-240 mg/day

Bumetanide:
3-(butylamino)-4-phenoxy-5-sulfamoylbenzoic acid
Dosage range from 0.25-1.25 mg/day to 1-5 mg/day, preferable from 0.5-2.5 mg/day to 1-5 mg/day, more preferably from 0.75-3.75 mg/day to 1-5 mg/day

Torasemide:
1-[4-(3-methylanilino)pyridin-3-yl]sulfonyl-3-propan-2-ylurea
Dosage range from 2.5-5 mg/day to 10-20 mg/day, preferable from 5-10 mg/day to 10-20 mg/day, more preferably from 7.5-15 mg/day to 10-20 mg/day

B endroflumethiazide:
3-benzyl-1,1-dioxo-6-(trifluoromethyl)-3,4-dihydro-2H-1lambda6,2,4-benzothiadiazine-7-sulfonamide
Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day

Hydrochlorothiazide:
6-chloro-1,1-dioxo-3,4-dihydro-2H-1lambda6,2,4-benzothiadiazine-7-sulfonamide Dosage range from 3.125-25 mg/day to 12.5-100 mg/day, preferable from 6.25-50 mg/day to 12.5-100 mg/day, more preferably from 9.375-75 mg/day to 12.5-100 mg/day

Metolazone:
7-chloro-2-methyl-3-(2-methylphenyl)-4-oxo-1,2-dihydroquinazoline-6-sulfonamide Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day

Indapamide:
4-chloro-N-(2-methyl-2,3-dihydroindol-1-yl)-3-sulfamoylbenzamide Dosage range from 0.625-1.25 mg/day to 2.5-5 mg/day, preferable from 1.25-2.5 mg/day to 2.5-5 mg/day, more preferably from 1.875-3.75 mg/day to 2.5-5 mg/day Gliflozine

Dapagliflozin:
(2S,3R,4R,5S,6R)-2-[4-chloro-3-[(4-ethoxyphenyl)methyl]phenyl]-6-(hydroxymethyl)oxane-3,4,5-triol
Dosage range from 2.5 mg/day to 10 mg/day, preferable from 5 mg/day to 10 mg/day, more preferably from 7.5 mg/day to 10 mg/day

Canagliflozin:
(2S,3R,4R,5S,6R)-2-[3-[[5-(4-fluorophenyl)thiophen-2-yl]methyl]-4-methylphenyl]-6-(hydroxymethyl)oxane-3,4,5-triol
Dosage range from 75 mg/day to 300 mg/day, preferable from 150 mg/day to 300 mg/day, more preferably from 225 mg/day to 300 mg/day

Empagliflozin :
(2S,3R,4R,5S,6R)-2-[4-chloro-3-[[4-[(3 S)-oxolan-3-yl]oxyphenyl]methyl]phenyl]-6-(hydroxymethyl)oxane-3,4,5-triol
Dosage range from 6.25 mg/day to 25 mg/day, preferable from 10 mg/day to 25 mg/day, more preferably from 10 mg/day to 20 mg/day

Ertugliflozin :
(1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol
Dosage range from 3.75 mg/day to 15 mg/day, preferable from 7.5 mg/day to 15 mg/day, more preferably from 11.25 mg/day to 15 mg/day

Ipragliflozin
(2S,3R,4R,5S,6R)-2-[3-(1-benzothiophen-2-ylmethyl)-4-fluorophenyl]-6-(hydroxymethyl)oxane-3,4,5-triol
Dosage range from 25 mg/day to 100 mg/day, preferable from 50 mg/day to 100 mg/day, more preferably from 75 mg/day to 100 mg/day

Tofogliflozin
(3S,3'R,4'S,5'S,6'R)-5-[(4-ethylphenyl)methyl]-6'-(hydroxymethyl)spiro[1H-2-benzofuran-3,2'-oxane]-3',4',5'-triol
Dosage range from 5 mg/day to 20 mg/day, preferable from 10 mg/day to 20 mg/day, more preferably from 15 mg/day to 20 mg/day

Luseogliflozin
(2S,3R,4R,5S,6R)-2-[5-[(4-ethoxyphenyl)methyl]-2-methoxy-4-methylphenyl]-6-(hydroxymethyl)thiane-3,4,5-triol
Dosage range from 1.25 mg/day to 5 mg/day, preferable from 2.5 mg/day to 5m g/day, more preferably from 3.75 mg/day to 5 mg/day

Sotagliflozin :
(2S,3R,4R,5S,6R)-2-[4-chloro-3-[(4-ethoxyphenyl)methyl]phenyl]-6-methylsulfanyloxane-3,4,5-triol
Dosage range from 100 mg/day to 400 mg/day, preferable from 200mg/day to 400 mg/day, more preferably from 300 mg/day to 400 mg/day.

37. Pharmaceutical oral formulation for use in the treatment of a patient having acute and/or persistent dyspnea comprising a combination medication according to any of embodiments 1 - 36, and wherein a sample of bodily fluid of said patient has a level of BNP > 200 pg/mL and/or has a level NT-proBNP > 600 pg/mL, and wherein said sample of bodily fluid is selected from the group comprising blood, plasma and serum.

38. Pharmaceutical oral formulation for use in the treatment of a patient having acute and/or persistent dyspnea comprising a combination medication according to any of embodiments 1 - 37, wherein said patient has no heart failure (HF).

39. Pharmaceutical oral formulation for use in the treatment of a patient having acute and/or persistent dyspnea comprising a combination medication according to any of embodiments 1 - 38, wherein said patient has no history of heart failure (HF).

40. Pharmaceutical oral formulation for use in the treatment of a patient having acute and/or persistent dyspnea comprising a combination medication according to any of embodiments 1 - 39, wherein the patient has no clinical signs of congestion.

41. Pharmaceutical oral formulation for use in the treatment of a patient having acute and/or persistent dyspnea comprising a combination medication according to any of embodiments 1 - 40, wherein the patient has no cardiovascular or pulmonary disease.

42. Pharmaceutical oral formulation for use in the treatment of a patient having acute and/or persistent dyspnea comprising a combination medication according to any of embodiments 1 - 41, wherein said combination is a fixed combination in one administration form.

43. Pharmaceutical oral formulation for use in the treatment of a patient having acute and/or persistent dyspnea comprising a combination medication according to any of embodiments 1 - 42, wherein said combination is a fixed combination, and wherein said combination is a fixed combination in one administration form, wherein said administration form is administered orally.

44. Pharmaceutical oral formulation for use in the treatment of a patient having acute and/or persistent dyspnea comprising a combination medication according to any of embodiments 1 - 43, wherein said combination is a fixed combination in one administration form, wherein the formulation is selected from the group comprising a pill, a tablet, a liqiuid medicine, a capsule, a film tablet, a dragee.

45. Method of treatment of acute and/or persistent dyspnea in a patient, the method comprising administering a combination medication comprising at least two of the following components:
a. a beta blocker (BB),
b. one agent of the group of *renin-angiotensin-aldosterone system* (RAAS) inhibitors consisting of (i) an angiotensin-converting enzyme (ACE) inhibitor, (ii) an angiotensin II inhibitor and (iii) a combination of angiotensin II inhibitor and angiotensin receptor-neprilysin inhibitor (ARNi)
c. and mineralo receptor antagonist (MRA),
d. gliflozine,
e. a diurectic
and wherein a sample of bodily fluid of said patient has a level of BNP > 200 pg/mL and/or has a level NT-proBNP > 600 pg/mL, and wherein said sample of bodily fluid is selected from the group comprising blood, plasma and serum.

46. Method of treatment of acute and/or persistent dyspnea in a patient, the method comprising administering a combination medication according to embodiment 45, wherein said combination medication comprises at least two of said components which are BB and RAASi, preferably said combination medication comprises at least three of said components which are BB, RAASi and MRAs, more preferably said combination medication comprises at least three of said components and gliflozine.

47. Method of treatment of acute and/or persistent dyspnea in a patient, the method comprising administering a combination medication according to embodiments 45 to 46, wherein said patient is a patient with heart failure with mildly reduced or preserved left-ventricular ejection fraction (LVEF) or a patient with with no heart failure.

48. Method of treatment of acute and/or persistent dyspnea in a patient, the method comprising administering a combination medication according to embodiments 45 to 47, wherein said patient is a patient with heart failure, wherein said patient has a mildly reduced left-ventricular ejection fraction (LVEF) with more than or equal to 40%.

49. Method of treatment of acute and/or persistent dyspnea in a patient, the method comprising administering a combination medication according to embodiments 45 to 48, wherein said patient has no heart failure (HF).

50. Method of treatment of acute and/or persistent dyspnea in a patient, the method comprising administering a combination medication according to embodiments 45 to 49, wherein said patient has no history of heart failure.

51. Method of treatment of acute and/or persistent dyspnea in a patient, the method comprising administering a combination medication according to embodiments 45 to 50, wherein said patient has no clinical signs of congestion.

52. Method of treatment of acute and/or persistent dyspnea in a patient, the method comprising administering a combination medication according to embodiments 45 to 51, wherein the patient has no cardiovascular or pulmonary disease.

53. Method of treatment of acute and/or persistent dyspnea in a patient, the method comprising administering a combination medication according to embodiments 45 to 52, wherein said patient receives the combination medication in two different doses, which are named dose No 1 and dose No 2.

54. Method of treatment of acute and/or persistent dyspnea in a patient, the method comprising administering a combination medication according to embodiments 45 to 53, wherein said combination is a fixed combination in one administration form.

55. Method of treatment of acute and/or persistent dyspnea in a patient, the method comprising administering a combination medication according to embodiments 45 to 54, wherein said combination is a fixed combination in one administration form, wherein said administration form is selected from the group comprising a pill, a tablet, a liqiuid medicine, a capsule, a film tablet, a dragee.

56. Method of treatment of acute and/or persistent dyspnea in a patient, the method comprising administering a combination medication according to embodiments 45 to 55, wherein the level of proADM and/or fragments thereof having at least 5 amino acids in a sample of bodily fluid of said patient is above a certain threshold.

57. Method of treatment of acute and/or persistent dyspnea in a patient, the method comprising administering a combination medication according to embodiments 45 to 56, wherein the level of proADM and/or fragments thereof having at least 5 amino acids in a sample of bodily fluid of said patient is above a certain threshold, wherein said sample of bodily fluid is selected from the group comprising plasma, serum, blood.

58. Method of treatment of acute and/or persistent dyspnea in a patient, the method comprising administering a combination medication according to embodiments 45 to 57, wherein Pro-Adrenomedullin or fragments thereof of at least 5 amino acids is used as a marker for congestion comprising:
o Determining the level of Pro-Adrenomedullin or fragments thereof of at least 5 amino acids in a bodily fluid obtained from said subject; and
o Correlating said level of Pro-Adrenomedullin or fragments thereof with the extent of congestion in said subject or diagnosing congestion, wherein an elevated level above a certain threshold is indicative of congestion or the extent of congestion,
wherein said Pro-Adrenomedullin or fragment is selected from the group comprising Pro-Adrenomedullin according to SEQ ID No. 1 or PAMP according to SEQ ID No.: 2 or MR-proADM according to SEQ ID No.: 3 or mature ADM-NH2 according to SEQ ID No.: 4 or ADM-Gly according to SEQ ID No.: 5 or CT-proADM according to SEQ ID No.: 6, and wherein said patient has no congestion, i.e. the level of proADM and/or fragments thereof having at least 5 amino acids in a sample of bodily fluid of said patient is below a certain threshold.

59. Method of treatment of acute and/or persistent dyspnea in a patient, the method comprising administering a combination medication according to embodiments 45 to 58, wherein said threshold is within a threshold range that is a threshold range for plasma mature ADM-NH2 according to SEQ ID No.: 4 between 50 and 100 pg/ml and for plasma MR-proADM between 0.5 and 1.5 nmol/L, and for plasma CT-proADM between 85 and 350 pmol/L.

60. Method of treatment of acute and/or persistent dyspnea in a patient, the method comprising administering a combination medication according to embodiments 45 to 59, wherein an assay is used for determining the level of proADM and/or fragments thereof in a sample of bodily fluid of said patient, wherein the assay sensitivity of said assay is able to quantify the proADM of healthy subjects and is < 70 pg/ml, preferably < 40 pg/ml and more preferably < 10 pg/ml.

61. Method of treatment of acute and/or persistent dyspnea in a patient, the method comprising administering a combination medication according to embodiments 45 to 60, wherein in a sample of bodily fluid of said patient the level of mature ADM-NH2 and/or fragments thereof having at least 5 amino acids is determined.

62. Method of treatment of acute and/or persistent dyspnea in a patient, the method comprising administering a combination medication according to embodiments 45 to 61, wherein in a sample of bodily fluid of said patient the level of mature ADM-NH2 and/or fragments thereof having at least 5 amino acids is determined, and wherein said level of mature ADM-NH2 and/or fragments thereof is correlated with the extent of congestion in said subject or diagnosing congestion, wherein the threshold is within a threshold range between 50 and 100 pg/ml.

63. Method of treatment of acute and/or persistent dyspnea in a patient, the method comprising administering a combination medication according to embodiments 45 to 62, wherein an assay is used for determining the level of mature ADM-NH2 and/or fragments thereof having at least 5 amino acids in a sample of bodily fluid of said patient.

64. Method of treatment of acute and/or persistent dyspnea in a patient, the method comprising administering a combination medication according to embodiments 45 to 63, wherein an assay is used for determining the level of mature ADM-NH2 and/or fragments thereof having at least 5 amino acids in a sample of bodily fluid of said patient, wherein the assay sensitivity of said assay is able to quantify the mature ADM-NH2 of healthy subjects and is < 70 pg/ml, preferably < 40 pg/ml and more preferably < 10 pg/ml.

65. Method of treatment of acute and/or persistent dyspnea in a patient, the method comprising administering a combination medication according to embodiments 45 to 64, wherein in a sample of bodily fluid of said patient the level of CT-proADM and/or fragments thereof having at least 5 amino acids is determined, and wherein said level of CT-proADM and/or fragments thereof is correlated with the extent of congestion in said subject or diagnosing congestion, wherein the threshold is within a threshold range between 85 and 350 pmol/L.

66. Method of treatment of acute and/or persistent dyspnea in a patient, the method comprising administering a combination medication according to embodiments 45 to 65, wherein an assay is used for determining the level of CT-proADM and/or fragments thereof having at least 5 amino acids in a sample of bodily fluid of said patient.

67. Method of treatment of acute and/or persistent dyspnea in a patient, the method comprising administering a combination medication according to embodiments 45 to 66, wherein an assay is used for determining the level of CT-proADM and/or fragments thereof having at least 5 amino acids in a sample of bodily fluid of said patient, wherein the assay sensitivity of said assay is able to quantify the CT-proADM of healthy subjects and is < 100 pmol/L, preferably < 75 pmol/L and more preferably < 50 pmol/L.

68. Method of treatment of acute and/or persistent dyspnea in a patient, the method comprising administering a combination medication according to embodiments 45 to 67, wherein in a sample of bodily fluid of said patient the level of MR-proADM and/or fragments thereof having at least 5 amino acids is determined, and wherein said level of MR-proADM and/or fragments thereof is correlated with the extent of congestion in said subject or diagnosing congestion, wherein the threshold is within a threshold range between 0.5 and 1.5 nmol/L.

69. Method of treatment of acute and/or persistent dyspnea in a patient, the method comprising administering a combination medication according to embodiments 45 to 68, wherein an assay is used for determining the level of MR-proADM and/or fragments thereof having at least 5 amino acids in a sample of bodily fluid of said patient.

70. Method of treatment of acute and/or persistent dyspnea in a patient, the method comprising administering a combination medication according to embodiments 45 to 69,
wherein an assay is used for determining the level of MR-proADM and/or fragments thereof having at least 5 amino acids in a sample of bodily fluid of said patient, wherein the assay sensitivity of said assay is able to quantify the MR-proADM of healthy subjects and is < 0,5 nmol/L, preferably < 0,4npmol/L and more preferably < 0,2 nmol/L.

71. Method of treatment of acute and/or persistent dyspnea in a patient, the method comprising administering a combination medication according to embodiments 45 to 70, wherein the agent according to group b. (iii) of said combination medication is a combination of angiotensin II inhibitor and angiotensin receptor-neprilysin inhibitor (ARNi), wherein in particular the ARNi is a combination of the neprilysin inhibitor 4-[[(2S,4R)-5-ethoxy-4-methyl-5-oxo-1-(4-phenylphenyl)pentan-2-yl]amino]-4-oxobutanoic acid and the nonpeptide triazole-derived antagonist of angiotensin (AT) II (2S)-3-methyl-2-[pentanoyl-[[4-[2-(2H-tetrazol-5-yl)phenyl]phenyl]methyl]amino]butanoic acidII.

72. Method of treatment of acute and/or persistent dyspnea in a patient, the method comprising administering a combination medication according to embodiments 45 to 71, wherein said combination medication comprises
a. a beta blocker,
b. one agent of the group consisting of (i) an angiotensin-converting enzyme (ACE) inhibitor, (ii) an angiotensin II inhibitor and (iii) a combination of angiotensin II inhibitor and angiotensin receptor-neprilysin inhibitor (ARNi)
c. and mineralo receptor antagonist (MRA), and
d. gliflozine (canagliflozine).
e. Diuretic

73. Method of treatment of acute and/or persistent dyspnea in a patient, the method comprising administering a combination medication according to embodiments 45 to 72, wherein said combination medication comprises in addition diuretics.

74. Method of treatment of acute and/or persistent dyspnea in a patient, the method comprising administering a combination medication according to embodiments 45 to 73, wherein said patient receives a dose No 1 of said combination medication for up to 2 to 4 weeks and thereafter a dose No 2 for at least 90 days, wherein dose No 1 is half dose (25%) of dose No 2, more preferably .dose No 1 is 30% of dose No 2, more preferably .dose No 1 is 35% of dose No 2, more preferably dose No 1 is 40% of dose No 2, more preferably .dose No 1 is 45% of dose No 2, more preferably .dose No 1 is 50% of dose No 2.

75. Method of treatment of acute and/or persistent dyspnea in a patient, the method comprising administering a combination medication according to embodiments 45 to 74, wherein said fixed or non-fixed combination medication is administered orally.

76. Method of treatment of acute and/or persistent dyspnea in a patient, the method comprising administering a combination medication according to embodiments 45 to 75, wherein a beta blocker is selected from the group beta-1 adrenergic receptor antagonist 1-(propan-2-ylamino)-3-[4-(2-propan-2-yloxyethoxymethyl)phenoxy]propan-2-ol, beta-adrenoceptor blocking agent 1-(9H-carbazol-4-yloxy)-3-[2-(2-methoxyphenoxy)ethylamino]propan-2-ol, succinate of 1-[4-(2-methoxyethyl)phenoxy]-3-(propan-2-ylamino)propan-2-ol (CR/XL), the beta-1 adrenergic receptor antagonist 1-(6-fluoro-3,4-dihydro-2*H*-chromen-2-yl)-2-[[2-(6-fluoro-3,4-dihydro-2*H*-chromen-2-yl)-2-hydroxyethyl]amino]ethanol, (2R,3S)-5-[3-(tert-butylamino)-2-hydroxypropoxy]-1,2,3,4tetrahydronaphthalene-2,3-diol, N-[3-acetyl-4-[2-hydroxy-3-(propan-2-ylamino)propoxy]phenyl]butanamide, 2-hydroxy-5-[1-hydroxy-2-(4-phenylbutan-2-ylamino)ethyl]benzamide, 3-[3-acetyl-4-[3-(tert-butylamino)-2-hydroxypropoxy]phenyl]-1,1-diethylurea, 2-[4-[2-hydroxy-3-(propan-2-ylamino)propoxy]phenyl]acetamide, 1-naphthalen-1-yloxy-3-(propan-2-ylamino)propan-2-ol.

77. Method of treatment of acute and/or persistent dyspnea in a patient, the method comprising administering a combination medication according to embodiments 45 to 76, wherein the ACE inhibitor is selected from the group comprising the sulfhydryl-containing analog of proline (2*S*)-1-[(2*S*)-2-methyl-3-sulfanylpropanoyl]pyrrolidine-2-carboxylic acid, (2*S*)-1-[(2*S*)-2-[[(2*S*)-1-ethoxy-1-oxo-4-phenylbutan-2-yl]amino]propanoyl]pyrrolidine-2-carboxylic acid, (2*S*)-1-[(2*S*)-6-amino-2-[[(1*S*)-1-carboxy-3-phenylpropyl]amino]hexanoyl]pyrrolidine-2-carboxylic acid, (2*S*,3*aS*,6*aS*)-1-[(2*S*)-2-[[(2*S*)-1-ethoxy-1-oxo-4-phenylbutan-2-yl]amino]propanoyl]-3,3*a*,4,5,6,6*a-*hexahydro-2*H*-cyclopenta[b]pyrrole-2-carboxylic acid, (2*S*,3*aR*,7*aS*)-1-[(2*S*)-2-[[(2*S*)-1-ethoxy-1-oxo-4-phenylbutan-2-yl]amino]propanoyl]-2,3,3*a*,4,5,6,7,7*a-*octahydroindole-2-carboxylic acid, 2-[(3S)-3-[[(2S)-1-ethoxy-1-oxo-4-phenylbutan-2-yl]amino]-2-oxo-4,5-dihydro-3H-1-benzazepin-1-yl] acetic acid, (2S,4S)-4-cyclohexyl-1-[2-[(2-methyl-1-propanoyloxypropoxy)-(4-phenylbutyl)phosphoryl]acetyl]pyrrolidine-2-carboxylic acid, (3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid, (2S,3aS,7aS)-1-[(2S)-2-[[(2S)-1-ethoxy-1-oxopentan-2-yl]amino]propanoyl]-2, 3,3a,4,5,6,7,7a-octahydroindole-2-carboxylicacid;(2S)-2-amino-5-(diaminomethylideneamino) pentanoic acid, (2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine, (3S)-2-[(2S)-2-[[(2S)-1-ethoxy-1-oxo-4-phenylbutan-2-yl]amino]propanoyl]-3,4-dihydro-1H-isoquinoline-3-carboxylic acid, (4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid.

78. Method of treatment of acute and/or persistent dyspnea in a patient, the method comprising administering a combination medication according to embodiments 45 to 77, wherein the MRAs(mineralo receptor antagonist) is selected from the group comprising methyl (1R,2S,9R,10R,11S,14R,15S,17R)-2,15-dimethyl-5,5'-dioxospiro[18-oxapentacyclo[8.8.0.01,17.02,7.011,15]octadec-6-ene-14,2'-oxolane]-9-carboxylate, S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate.

79. Method of treatment of acute and/or persistent dyspnea in a patient, the method comprising administering a combination medication according to embodiments 45 to 78, wherein the diurectic is selected from the group comprising loop-acting diuretics such as 3-(butylamino)-4-phenoxy-5-sulfamoylbenzoic acid, 2-[2,3-dichloro-4-(2-methylidenebutanoyl)phenoxy]acetic acid, 4-chloro-2-(furan-2-ylmethylamino)-5-sulfamoylbenzoic acid, 1-[4-(3-methylanilino)pyridin-3-yl]sulfonyl-3-propan-2-ylurea, potassium-sparing diuretics such as 3,5-diamino-6-chloro-*N-*(diaminomethylidene)pyrazine-2-carboxamide,, methyl (1*R*,2*S*,9*R*,10*R*,11*S*,14*R*,15*S*,17*R*)-2,15-dimethyl-5,5'-dioxospiro[18-oxapentacyclo[8.8.0.0^{1,17}.0^{2,7}.0^{11,15}]octadec-6-ene-14,2'-oxolane]-9-carboxylate, *S*-[(7*R*,8*R*,9*S*,10*R*,13*S*,14*S*,17*R*)-10,13-dimethyl-3,5'-dioxospiro[2,6,7,8,9,11,12,14,15,16-decahydro-1*H*-cyclopenta[a]phenanthrene-17,2'-oxolane]-7-yl]ethanethioate, 6-phenylpteridine-2,4,7-triamine, thiazide diuretics such as 6-chloro-1,1-dioxo-4*H*-1λ⁶,2,4-benzothiadiazine-7-sulfonamide, 2-chloro-5-(1-hydroxy-3-oxo-2*H*-isoindol-1-yl)benzenesulfonamide, 6-chloro-1,1-dioxo-3,4-dihydro-2H-1lambda6,2,4-benzothiadiazine-7-sulfonamide, 4-chloro-N-(2-methyl-2,3-dihydroindol-1-yl)-3-sulfamoylbenzamide, 7-chloro-2-methyl-3-(2-methylphenyl)-4-oxo-1,2-dihydroquinazoline-6-sulfonamide, 7-chloro-2-methyl-3-(2-methylphenyl)-4-oxo-1,2-dihydroquinazoline-6-sulfonamide and/or mixtures thereof.

80. Method of treatment of acute and/or persistent dyspnea in a patient, the method comprising administering a combination medication according to embodiments 45 to 79, wherein the gliflozine is selected from the group comprising (2S,3R,4R,5S,6R)-2-[3-[[5-(4-fluorophenyl)thiophen-2-yl]methyl]-4-methylphenyl]-6-(hydroxymethyl)oxane-3,4,5-triol, (2S,3R,4R,5S,6R)-2-[4-chloro-3-[(4-ethoxyphenyl)methyl]phenyl]-6-(hydroxymethyl)oxane-3,4,5-triol, (2S,3R,4R,5S,6R)-2-[4-chloro-3-[[4-[(3S)-oxolan-3-yl]oxyphenyl]methyl]phenyl]-6-(hydroxymethyl)oxane-3,4,5-triol, (1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol, Luseogliflozin, Remogliflozin etabonate (ethyl [(2R,3S,4S,5R,6S)-3,4,5-trihydroxy-6-[5-methyl-1-propan-2-yl-4-[(4-propan-2-yloxyphenyl)methyl]pyrazol-3-yl]oxyoxan-2-yl]methyl carbonate), sergliflozin etabonate (ethyl [(2R,3S,4S,5R,6S)-3,4,5-trihydroxy-6-[2-[(4-methoxyphenyl)methyl]phenoxy]oxan-2-yl]methyl carbonate), (2S,3R,4R,5S,6R)-2-[3-(1-benzothiophen-2-ylmethyl)-4-fluorophenyl]-6-(hydroxymethyl)oxane-3,4,5-triol, (3S,3'R,4'S,5'S,6'R)-5-[(4-ethylphenyl)methyl]-6'-(hydroxymethyl)spiro[1H-2-benzofuran-3,2'-oxane]-3',4',5'-triol, (2S,3R,4R,5S,6R)-2-[5-[(4-ethoxyphenyl)methyl]-2-methoxy-4-methylphenyl]-6-(hydroxymethyl)thiane-3,4,5-triol, (2S,3R,4R,5S,6R)-2-[4-chloro-3-[(4-ethoxyphenyl)methyl]phenyl]-6-methylsulfanyloxane-3,4,5-triol and/or mixtures thereof.

81. Method of treatment of acute and/or persistent dyspnea in a patient according to embodiments 45 to 80, the method comprising administering an oral pharmaceutical formulation, wherein a sample of bodily fluid of said patient has a level of BNP > 200 pg/mL and/or has a level NT-proBNP > 600 pg/mL, and wherein said sample of bodily fluid is selected from the group comprising blood, plasma and serum.

82. Method of treatment of acute and/or persistent dyspnea in a patient, the method comprising administering an oral pharmaceutical formulation according to embodiment 81, wherein said patient has no heart failure (HF).

83. Method of treatment of acute and/or persistent dyspnea in a patient, the method comprising administering an oral pharmaceutical formulation according to any of embodiments 45-82, wherein said patient has no history of heart failure (HF).

84. Method of treatment of acute and/or persistent dyspnea in a patient, the method comprising administering an oral pharmaceutical formulation according to any of embodiments 45-83 wherein the patient has no clinical signs of congestion.

85. Method of treatment of acute and/or persistent dyspnea in a patient, the method comprising administering an oral pharmaceutical formulation according to any of embodiments 45-84, wherein the patient has no cardiovascular or pulmonary disease.

86. Method of treatment of acute and/or persistent dyspnea in a patient, the method comprising administering an oral pharmaceutical formulation according to any of embodiments 45-85, wherein the formulation is a tablet or capsule.

87. Combination medication for use in the treatment of a patient having acute and/or persistent dyspnea according to embodiment 1 and following embodiments comprising at least two of the following components:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and angiotensin receptor-neprilysin inhibitor (ARNi)
c. and mineralo receptor antagonist (MRA),
d. gliflozine,
e. a diuretic,
and wherein a sample of bodily fluid of said patient has a level of BNP > 200 pg/mL and/or has a level NT-proBNP > 600 pg/mL, and wherein said sample of bodily fluid is selected from the group comprising blood, plasma and serum,
wherein said patient is
   - a patient with heart failure with mildly reduced, from 40 to 49%, left-ventricular ejection fraction (LVEF) or preserved, greater and equal to 50 %, LVEF or
   - a patient with no heartfailure, and
wherein the treatment is a daily administration of the combination medication, preferably once a day, and
wherein said combination is a fixed combination and wherein said combination is a fixed combination in one administration form, wherein said administration form is selected from the group comprising a pill, a tablet, a liqiuid medicine, a capsule, a film tablet, a dragee, and
wherein the level of proADM and/or fragments thereof having at least 5 amino acids in a sample of bodily fluid of said patient is above a certain threshold, and wherein said sample of bodily fluid is selected from the group comprising plasma, serum, blood, and wherein said patient receives the combination medication in two different doses, which are named dose No 1 and dose No 2, and
wherein said patient receives a dose No 1 of said combination medication for up to 2 to 4 weeks and thereafter a dose No 2 for at least 90 days, and
wherein dose No 1 is half dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

88. Combination medication for use in the treatment of a patient having acute and/or persistent dyspnea according to embodiment 1 and following embodiments comprising at least two of the following components:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and angiotensin receptor-neprilysin inhibitor (ARNi)
c. and mineralo receptor antagonist (MRA),
d. gliflozine,
e. a diuretic,
and wherein a sample of bodily fluid of said patient has a level of BNP > 200 pg/mL and/or has a level NT-proBNP > 600 pg/mL, and wherein said sample of bodily fluid is selected from the group comprising blood, plasma and serum, and
wherein the treatment is a daily administration of the combination medication,preferably once a day, and
wherein the patient has no heart failure but has another cardiovascular disease and/or another disease with non cardiac cause which maybe selected from the group comprising diabetes and kidney disease, and
wherein said combination is a fixed combination and wherein said combination is a fixed combination in one administration form, wherein said administration form is selected from the group comprising a pill, a tablet, a liqiuid medicine, a capsule, a film tablet, a dragee, and wherein the level of proADM and/or fragments thereof having at least 5 amino acids in a sample of bodily fluid of said patient is above a certain threshold, wherein said sample of bodily fluid is selected from the group comprising plasma, serum, blood, and wherein said patient receives a dose No 1 of said combination medication for up to 2 to 4 weeks and thereafter a dose No 2 for at least 90 days, wherein dose No 1 is half dose (25%) of dose No 2, more preferably .dose No 1 is 35% of dose No 2, more preferably .dose No 1 is 40% of dose No 2, more preferably .dose No 1 is 45% of dose No 2, more preferably dose No 1 is 50% of dose No 2.

Moreover, the following consecutively numbered embodiments provide further specific aspects of the invention:
1. Combination medication for use in the treatment of a patient having acute and/or persistent dyspnea for restoring the respiration rate and/or oxygen saturation toward normal values and/or maintaining the respiration rate and/or restoring and/or maintaining the oxygen saturation and/or for ameliorating the symptoms of dyspnea comprising at least three of the following compounds wherein each of said chemical compounds is a chemical entity, wherein each chemical entity is distinct from the other two:
   a. a beta blocker (BB),
   b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
   c. and mineralo receptor antagonist (MRA),
   d. an inhibitor of the sodium glucose co-transporter-2 (SGLT2), also called glifozins, e.g. dapagliflozin or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea;
   e. a diuretic to reduce or prevent occurrence of signs and/or symptoms of congestion in patients,
      and wherein a sample of bodily fluid of said patient has a level of Brain Natriuretic peptide (BNP) > 200 pg/mL and/or has a level of N-terminal (NT)-pro hormone BNP(NT-proBNP) > 600 pg/mL and/or the patient is congestive and/or has residual congestion, and wherein said sample of bodily fluid is selected from the group comprising blood, plasma and serum;
      and wherein said patient is either
         - a patient with heart failure with left ventricular ejection fraction (LVEF) greater and equal to 40 %, preferably 50 % or
         - a patient with no heart failure.
2. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to embodiment 1, wherein said combination comprises an inhibitor of the sodium glucose co-transporter-2, e.g. dapagliflozin or empagliflozin that is used for restoring the respiration rate and/or maintaining the respiration rate and/or restoring and/or maintaining the oxygen saturation and/or for ameliorating the symptoms of dyspnea.
3. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to embodiment 1 to 2, wherein said patient does not have diabetes.
4. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 3, wherein said patient has a myocardial dysfunction.
5. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 4, wherein the treatment is a daily administration of the combination medication, preferably once a day.
6. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 5, wherein the patient has no heart failure, but has another cardiovascular disease and/or another disease with non cardiac cause which maybe selected from the group comprising diabetes and kidney disease, and wherein a sample of bodily fluid of said patient has a level of BNP > 200 pg/mL and/or has a level NT-proBNP > 600 pg/mL, wherein the bodily fluid is a plasma sample, preferably a fasting plasma sample.
7. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 6, wherein said combination is a fixed combination and wherein said combination is a fixed combination in one single administration form, wherein said administration form is selected from the group comprising a pill, a tablet, a liqiuid medicine, a capsule, a film tablet, a dragee.
8. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 7, wherein the level of proAdrenomedullin (proADM) and/or fragments thereof, preferably mature Adrenomedullin (ADM-NH₂), having at least 5 amino acids in a sample of bodily fluid of said patient is above a certain threshold, wherein said sample of bodily fluid is selected from the group comprising plasma, serum, blood.
9. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 8, wherein the agent according to group b. (iii) of said combination medication according to embodiment 1 is a combination of angiotensin II inhibitor and angiotensin receptor-neprilysin inhibitor (ARNi), wherein in particular the ARNi is a combination of
   - the neprilysin inhibitor 4-[[(2*S*,4*R*)-5-ethoxy-4-methyl-5-oxo-1-(4-phenylphenyl)pentan-2-yl]amino]-4-oxobutanoic acid, and
   - the nonpeptide triazole-derived antagonist of angiotensin (AT) II (2*S*)-3-methyl-2-[pentanoyl-[[4-[2-(2*H*-tetrazol-5-yl)phenyl]phenyl]methyl]amino]butanoic acid II;
   preferably a fixed combination.
10. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 9, wherein said patient receives the combination medication in two different doses for a certain period of time, respectively, wherein said doses are named dose No 1 and dose No 2., wherein each dose No. 1 and No.2 refers to the respective dose of each of the chemical entities of the combination, wherein dose No. 1 is initially administered to said patient in an initial daily dose of each of the chemical entities of the combination for a certain period of time and is thereafter administered in a subsequent dose No. 2 for a certain period of time that is 2 to 4-fold higher than the said initial daily dose.
11. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 10, wherein said patient receives a dose No 1 of said combination medication for up to 2 to 4 weeks and thereafter a dose No 2 for at least 90 days, wherein dose No 1 is one quarter dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.
12. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 11, wherein a beta blocker is selected from the group comprising beta-1 adrenergic receptor antagonist 1-(propan-2-ylamino)-3-[4-(2-propan-2-yloxyethoxymethyl)phenoxy]propan-2-ol, beta-adrenoceptor blocking agent 1-(9H-carbazol-4-yloxy)-3-[2-(2-methoxyphenoxy)ethylamino]propan-2-ol, succinate of 1-[4-(2-methoxyethyl)phenoxy]-3-(propan-2-ylamino)propan-2-ol (CR/XL), the beta-1 adrenergic receptor antagonist 1-(6-fluoro-3,4-dihydro-2H-chromen-2-yl)-2-[[2-(6-fluoro-3,4-dihydro-2H-chromen-2-yl)-2-hydroxyethyl]amino]ethanol, (2R,3S)-5-[3-(tert-butylamino)-2-hydroxypropoxy]-1,2,3,4tetrahydronaphthalene-2,3-diol, N-[3-acetyl-4-[2-hydroxy-3-(propan-2-ylamino)propoxy]phenyl]butanamide, 2-hydroxy-5-[1-hydroxy-2-(4-phenylbutan-2-ylamino)ethyl]benzamide, 3-[3-acetyl-4-[3-(tert-butylamino)-2-hydroxypropoxy]phenyl]-1,1-diethylurea, 2-[4-[2-hydroxy-3-(propan-2-ylamino)propoxy]phenyl]acetamide, 1-naphthalen-1-yloxy-3-(propan-2-ylamino)propan-2-ol.
13. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 11, wherein the ACE inhibitor is selected from the group comprising the sulfhydryl-containing analog of proline (2S)-1-[(2S)-2-methyl-3-sulfanylpropanoyl]pyrrolidine-2-carboxylic acid, (2S)-1-[(2S)-2-[[(2S)-1-ethoxy-1-oxo-4-phenylbutan-2-yl]amino]propanoyl]pyrrolidine-2-carboxylic acid, (2S)-1-[(2S)-6-amino-2-[[(1S)-1-carboxy-3-phenylpropyl]amino]hexanoyl]pyrrolidine-2-carboxylic acid, (2S,3aS,6aS)-1-[(2S)-2-[[(2S)-1-ethoxy-1-oxo-4-phenylbutan-2-yl]amino]propanoyl]-3,3a,4,5,6,6a-hexahydro-2H-cyclopenta[b]pyrrole-2-carboxylic acid, (2S,3aR,7aS)-1-[(2S)-2-[[(2S)-1-ethoxy-1-oxo-4-phenylbutan-2-yl]amino]propanoyl]-2,3,3a,4,5,6,7,7a-octahydroindole-2-carboxylic acid, 2-[(3S)-3-[[(2S)-1-ethoxy-1-oxo-4-phenylbutan-2-yl]amino]-2-oxo-4,5-dihydro-3H-1-benzazepin-1-yl] acetic acid, (2S,4S)-4-cyclohexyl-1-[2-[(2-methyl-1-propanoyloxypropoxy)-(4-phenylbutyl)phosphoryl]acetyl]pyrrolidine-2-carboxylic acid, (3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid, (2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylicacid;(2S)-2-amino-5-(diaminomethylideneamino) pentanoic acid, (2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine, (3S)-2-[(2S)-2-[[(2S)-1-ethoxy-1-oxo-4-phenylbutan-2-yl]amino]propanoyl]-3,4-dihydro-1H-isoquinoline-3-carboxylic acid, (4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid.
14. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 11, wherein the MRAs (mineralo receptor antagonist) is selected from the group comprising methyl (1R,2S,9R,10R,11S,14R,15S,17R)-2,15-dimethyl-5,5'-dioxospiro[18-oxapentacyclo[8.8.0.01,17.02,7.011,15]octadec-6-ene-14,2'-oxolane]-9-carboxylate, S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate and (4*S*)-4-(4-cyano-2-methoxyphenyl)-5-ethoxy-2,8-dimethyl-1,4-dihydro-1,6-naphthyridine-3 -carboxamide.
15. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 11, wherein the diurectic is selected from the group comprising loop-acting diuretics such as 3-(butylamino)-4-phenoxy-5-sulfamoylbenzoic acid, 2-[2,3-dichloro-4-(2-methylidenebutanoyl)phenoxy] acetic acid, 4-chloro-2-(furan-2-ylmethylamino)-5 - sulfamoylbenzoic acid, 1-[4-(3-methylanilino)pyridin-3-yl]sulfonyl-3-propan-2-ylurea, potassium-sparing diuretics such as 3,5-diamino-6-chloro-N-(diaminomethylidene)pyrazine-2-carboxamide,, methyl (1R,2S,9R,10R,11S,14R,15S,17R)-2,15-dimethyl-5,5'-dioxospiro[18-oxapentacyclo[8.8.0.01,17.02,7.011,15]octadec-6-ene-14,2'-oxolane]-9-carboxylate, S-[(7R,8R,9S,10R,13S,14S,17R)-10,13-dimethyl-3,5'-dioxospiro[2,6,7,8,9,11,12,14,15,16-decahydro-1H-cyclopenta[a]phenanthrene-17,2'-oxolane]-7-yl]ethanethioate, 6-phenylpteridine-2,4,7-triamine, thiazide diuretics such as 6-chloro-1,1-dioxo-4H-1λ6,2,4-benzothiadiazine-7-sulfonamide, 2-chloro-5-(1-hydroxy-3-oxo-2H-isoindol-1-yl)benzenesulfonamide, 6-chloro-1,1-dioxo-3,4-dihydro-2H-1lambda6,2,4-benzothiadiazine-7-sulfonamide, 4-chloro-N-(2-methyl-2,3-dihydroindol-1-yl)-3-sulfamoylbenzamide, 7-chloro-2-methyl-3-(2-methylphenyl)-4-oxo-1,2-dihydroquinazoline-6-sulfonamide, and/or mixtures thereof.
16. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1 to 11, wherein the gliflozine is selected from the group comprising(2S,3R,4R,5S,6R)-2-[3-[[5-(4-fluorophenyl)thiophen-2-yl]methyl]-4-methylphenyl]-6-(hydroxymethyl)oxane-3,4,5-triol, (2S,3R,4R,5S,6R)-2-[4-chloro-3-[(4-ethoxyphenyl)methyl]phenyl]-6-(hydroxymethyl)oxane-3,4,5-triol, (2S,3R,4R,5S,6R)-2-[4-chloro-3-[[4-[(3S)-oxolan-3-yl]oxyphenyl]methyl]phenyl]-6-(hydroxymethyl)oxane-3,4,5-triol, (1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol, Luseogliflozin, Remogliflozin etabonate (ethyl [(2R,3S,4S,5R,6S)-3,4,5-trihydroxy-6-[5-methyl-1-propan-2-yl-4-[(4-propan-2-yloxyphenyl)methyl]pyrazol-3-yl]oxyoxan-2-yl]methyl carbonate), sergliflozin etabonate (ethyl [(2R,3S,4S,5R,6S)-3,4,5-trihydroxy-6-[2-[(4-methoxyphenyl)methyl]phenoxy]oxan-2-yl]methyl carbonate), (2S,3R,4R,5S,6R)-2-[3-(1-benzothiophen-2-ylmethyl)-4-fluorophenyl]-6-(hydroxymethyl)oxane-3,4,5-triol, (3S,3'R,4'S,5'S,6'R)-5-[(4-ethylphenyl)methyl]-6'-(hydroxymethyl)spiro[1H-2-benzofuran-3,2'-oxane]-3',4',5'-triol, (2S,3R,4R,5S,6R)-2-[5-[(4-ethoxyphenyl)methyl]-2-methoxy-4-methylphenyl]-6-(hydroxymethyl)thiane-3,4,5-triol, (2S,3R,4R,5S,6R)-2-[4-chloro-3-[(4-ethoxyphenyl)methyl]phenyl]-6-methylsulfanyloxane-3,4,5-triol and/or mixtures thereof.
17. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of embodiments 1-16, wherein the ranges of each of the chemical entities of the combination for dose No. 2 of the following agents are as follows:

### ACE-inhibitors

Captopril:
(2S)-1-[(2S)-2-methyl-3-sulfanylpropanoyl]pyrrolidine-2-carboxylic acid Dosage range from 37.5 mg/day to 150 mg/day, preferable from 75 mg/day mg to 150 mg/day, more preferably from 112.5 mg/day to 150 mg/day,

Enalapril:
(2S)-1-[(2S)-2-[[(2S)-1-ethoxy-1-oxo-4-phenylbutan-2yl]amino]propanoyl]pyrrolidine-2-carboxylic acid
Dosage range from 5-10 mg/day to 20-40 mg/day, preferable from 10-20 mg/day to 20-40 mg/day, more preferably from 15-30 mg/day to 20-40 mg/day

Lisinopril:
(2S)-1-[(2S)-6-amino-2-[[(1S)-1-carboxy-3-phenylpropyl]amino]hexanoyl]pyrrolidine-2-carboxylic acid
Dosage range from 5-8.75 mg/day to 20-35 mg/day, preferable from 10-17.5 mg/day to 20-35 mg/day, more preferably from 15-26.25 mg/day to 20-35 mg/day

Ramipril:
(2S,3aS,6aS)-1-[(2S)-2-[[(2S)-1-ethoxy-1-oxo-4-phenylbutan-2yl]amino]propanoyl]-3,3a,4,5,6,6a-hexahydro-2H-cyclopenta[b]pyrrole-2-carboxylic acid
Dosage range from 2.5 mg/day to 10 mg/day, preferable from 5 mg/day to 10 mg/day, more preferably from 7.5 mg/day to 10 mg/day

Trandolapril:
(2S,3aR,7aS)-1-[(2S)-2-[[(2S)-1-ethoxy-1-oxo-4-phenylbutan-2-yl]amino]propanoyl]-2,3,3a,4,5,6,7,7a-octahydroindole-2-carboxylic acid
Dosage range from 1 mg/day to 4 mg/day, preferable from 2 mg/day to 4 mg/day, more preferably from 3 mg/day to 4 mg/day

Benazepril:
2-[(3S)-3-[[(2S)-1-ethoxy-1-oxo-4-phenylbutan-2-yl]amino]-2-oxo-4,5-dihydro-3H-1-benzazepin-1-yl]acetic acid
Dosage range from 2.5 mg/day to 10 mg/day, preferable from 5 mg/day to 10 mg/day, more preferably from 7.5 mg/day to 10 mg/day

Fosinopril:
(2S,4S)-4-cyclohexyl-1-[2-[(2-methyl-1-propanoyloxypropoxy)-(4-phenylbutyl)phosphoryl]acetyl]pyrrolidine-2-carboxylic acid
Dosage range from 10 mg/day to 40 mg/day, preferable from 20 mg/day to 40 mg/day, more preferably from 30 mg/day to 40 mg/day

Moexipril:
(3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid
Dosage range from 7.5 mg/day to 30 mg/day, preferable from 15 mg/day to 30 mg/day, more preferably from 22.5 mg/day to 30 mg/day

Perindopril Arginine:
(2S,3aS,7aS)-1-[(2S)-2-[[(2S)-1-ethoxy-1-oxopentan-2-yl]amino]propanoyl]-2, 3,3a,4,5,6,7,7a-octahydroindole-2-carboxylicacid;(2S)-2-amino-5-(diaminomethylideneamino) pentanoic acid
Dosage range from 2.5 mg/day to 10 mg/day, preferable from 5 mg/day to 10 mg/day, more preferably from 7.5 mg/day to 10 mg/day

Perindopril tert-butylamine:
(2S,3aS,7aS)-1-[(2S)-2-[[(2S)-1-ethoxy-1-oxopentan-2-yl]amino]propanoyl]-2,3,3 a,4,5,6,7,7a-octahydroindole-2-carboxylic acid;2-methylpropan-2-amine Dosage range from 2 mg/day to 8 mg/day, preferable from 4 mg/day to 8 mg/day, more preferably from 6 mg/day to 8 mg/day

Quinapril :
(3S)-2-[(2S)-2-[[(2S)-1-ethoxy-1-oxo-4-phenylbutan-2-yl]amino]propanoyl]-3,4-dihydro-1H-isoquinoline-3-carboxylic acid
Dosage range from 10 mg/day to 40 mg/day, preferable from 20 mg/day to 40 mg/day, more preferably from 30 mg/day to 40 mg/day

Cilazapril :
(4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid
Dosage range from 1.25 mg/day to 5 mg/day, preferable from 2.5 mg/day to 5 mg/day, more preferably from 3.75 mg/day to 5 mg/day

### Beta-blockers

Bisoprolol:
1-(propan-2-ylamino)-3-[4-(2-propan-2-yloxyethoxymethyl)phenoxy]propan-2-ol Dosage range from 2.5 mg/day to 10 mg/day, preferable from 5 mg/day to 10 mg/day, more preferably from 7.5 mg/day to 10 mg/day

Carvedilol:
1-(9H-carbazol-4-yloxy)-3-[2-(2-methoxyphenoxy)ethylamino]propan-2-ol Dosage range from 12.5 mg/day to 50 mg/day, preferable from 25 mg/day to 50 mg/day, more preferably from 37.5 mg/day to 50 mg/day

Metoprolol succinate:
succinate of 1-[4-(2-methoxyethyl)phenoxy]-3-(propan-2-ylamino)propan-2-ol Dosage range from 50 mg/day to 200 mg/day, preferable from 100 mg/day to 200 mg/day, more preferably from 150 mg/day to 200 mg/day

Nebivolol:
1-(6-fluoro-3,4-dihydro-2H-chromen-2-yl)-2-[[2-(6-fluoro-3,4-dihydro-2H-chromen-2-yl)-2-hydroxyethyl]amino]ethanol
Dosage range from 2.5 mg/day to 10 mg/day, preferable from 5 mg/day to 10 mg/day, more preferably from 7.5 mg/day to 10 mg/day

Nadolol:
(2R,3S)-5-[3-(tert-butylamino)-2-hydroxypropoxy]-1,2,3,4tetrahydronaphthalene-2,3-diol
Dosage range from 20 mg/day to 80 mg/day, preferable from 40 mg/day to 80 mg/day, more preferably from 60 mg/day to 80 mg/day

Acebutolol :
N-[3-acetyl-4-[2-hydroxy-3-(propan-2-ylamino)propoxy]phenyl]butanamide
Dosage range from 100 mg/day to 400 mg/day, preferable from 200 mg/day to 400 mg/day, more preferably from 300 mg/day to 400 mg/day

Labetolol :
2-hydroxy-5-[1-hydroxy-2-(4-phenylbutan-2-ylamino)ethyl]benzamide
Dosage range from 200 mg/day to 800 mg/day, preferable from 400 mg/day to 800 mg/day, more preferably from 600 mg/day to 800 mg/day

Celiprolol :
3-[3-acetyl-4-[3-(tert-butylamino)-2-hydroxypropoxy]phenyl]-1,1-diethylurea
Dosage range from 150 mg/day to 600 mg/day, preferable from 300 mg/day to 600 mg/day, more preferably from 450 mg/day to 600 mg/day

Atenolol :
2-[4-[2-hydroxy-3-(propan-2-ylamino)propoxy]phenyl]acetamide
Dosage range from 25 mg/day to 100 mg/day, preferable from 50 mg/day to 100 mg/day, more preferably from 75 mg/day to 100 mg/day

Propranolol :
1-naphthalen-1-yloxy-3 -(propan-2-ylamino)propan-2-ol
Dosage range from 40 mg/day to 160 mg/day, preferable from 80 mg/day to 160 mg/day, more preferably from 120 mg/day to 160 mg/day

### Angiotensin Receptor Blockers

Candesartan:
2-ethoxy-3-[[4-[2-(2H-tetrazol-5-yl)phenyl]phenyl]methyl]benzimidazole-4-carboxylic acid
Dosage range from 8 mg/day to 32 mg/day, preferable from 16 mg/day to 32 mg/day, more preferably from 24 mg/day to 32 mg/da

Valsartan:
(2S)-3-methyl-2-[pentanoyl-[[4-[2-(2H-tetrazol-5-yl)phenyl]phenyl]methyl] amino]butanoic acid
Dosage range from 80 mg/day to 320 mg/day, preferable from 160 mg/day to 320 mg/day, more preferably from 240 mg/day to 320 mg/day

Losartan:
[2-butyl-5-chloro-3-[[4-[2-(2H-tetrazol-5-yl)phenyl]phenyl]methyl]imidazol-4-yl]methanol
Dosage range from 37.5 mg/day to 150 mg/day, preferable from 75 mg/day to 150 mg/day, more preferably from 112.5 mg/day to 150 mg/day

Azilsartan:
2-ethoxy-3-[[4-[2-(5-oxo-4H-1,2,4-oxadiazol-3-yl)phenyl]phenyl]methyl] benzimidazole-4-carboxylic acid
Dosage range from 20 mg/day to 80 mg/day, preferable from 40 mg/day to 80 mg/day, more preferably from 60 mg/day to 80 mg/day

Eprosartan:
4-[[2-butyl-5-[(E)-2-carboxy-3-thiophen-2-ylprop-1-enyl]imidazol-1-yl] methyl]benzoic acid
Dosage range from 150 mg/day to 600 mg/day, preferable from 300 mg/day to 600 mg/day, more preferably from 450 mg/day to 600 mg/day

Irbesartan:
2-butyl-3-[[4-[2-(2H-tetrazol-5-yl)phenyl]phenyl]methyl]-1,3-diazaspiro[4.4]non-1-en-4-one
Dosage range from 75 mg/day to 300 mg/day, preferable from 150 mg/day to 300 mg/day, more preferably from 225 mg/day to 300 mg/day

Olmesartan:
5-(2-hydroxypropan-2-yl)-2-propyl-3-[[4-[2-(2H-tetrazol-5-yl)phenyl]phenyl]methyl]imidazole-4-carboxylic acid
Dosage range from 10 mg/day to 40 mg/day, preferable from 20 mg/day to 40 mg/day, more preferably from 30 mg/day to 40 mg/day

Telmisartan:
2-[4-[[4-methyl-6-(1-methylbenzimidazol-2-yl)-2-propylbenzimidazol-1-yl]methyl]phenyl]benzoic acid
Dosage range from 20 mg/day to 80 mg/day, preferable from 40 mg/day to 80 mg/day, more preferably from 60 mg/day to 80 mg/day

### Mineraloreceptor antagonist (MRAs)

Eplerenone:
methyl(1R,2S,9R,10R,11S,14R,15S,17R)-2,15-dimethyl-5,5'-dioxospiro[18-oxapentacyclo[8.8.0.01,17.02,7.011,15]octadec-6-ene-14,2'-oxolane]-9-carboxylate Dosage range from 12.5 mg/day to 50 mg/day, preferable from 25 mg/day to 50 mg/day, more preferably from 37.5 mg/day to 50 mg/day

Spironolactone:
S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate
Dosage range from 12.5 mg/day to 50 mg/day, preferable from 25 mg/day to 50 mg/day, more preferably from 37.5 mg/day to 50 mg/day

Finerenone:
(4*S*)-4-(4-cyano-2-methoxyphenyl)-5-ethoxy-2,8-dimethyl-1,4-dihydro-1,6-naphthyridine-3-carboxamide
Dosage range from 10 mg/day to 20 mg/day, preferable 15 mg/day to 20 mg/day

### ARNI

Sacubitril/valsartan:
4-[[(2S,4R)-5-ethoxy-4-methyl-5-oxo-1-(4-phenylphenyl)pentan-2-yl]amino]-4-oxobutanoic acid / (2S)-3-methyl-2-[pentanoyl-[[4-[2-(2H-tetrazol-5-yl)phenyl] phenyl]methyl]amino]butanoic acidII
Dosage range from 48.5mg/51.5mg/day to 194mg/206mg/day, preferable from 97mg/103mg/day to 194mg/206mg/day, more preferably from 145.5mg/154.5mg/day to 194mg/206mg/day

### Diuretics

Furosemide:
4-chloro-2-(furan-2-ylmethylamino)-5-sulfamoylbenzoic acid
Dosage range from 10-60 mg/day to 40-240 mg/day, preferable from 20-120 mg/day to 40-240 mg/day, more preferably from 30-180 mg/day to 40-240 mg/day

Bumetanide:
3-(butylamino)-4-phenoxy-5-sulfamoylbenzoic acid
Dosage range from 0.25-1.25 mg/day to 1-5 mg/day, preferable from 0.5-2.5 mg/day to 1-5 mg/day, more preferably from 0.75-3.75 mg/day to 1-5 mg/day

Torasemide:
1-[4-(3-methylanilino)pyridin-3-yl]sulfonyl-3-propan-2-ylurea
Dosage range from 2.5-5 mg/day to 10-20 mg/day, preferable from 5-10 mg/day to 10-20 mg/day, more preferably from 7.5-15 mg/day to 10-20 mg/day

Bendroflumethiazide:
3-benzyl-1,1-dioxo-6-(trifluoromethyl)-3,4-dihydro-2H-1lambda6,2,4-benzothiadiazine-7-sulfonamide
Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day

Hydrochlorothiazide:
6-chloro-1,1-dioxo-3,4-dihydro-2H-1lambda6,2,4-benzothiadiazine-7-sulfonamide Dosage range from 3.125-25 mg/day to 12.5-100 mg/day, preferable from 6.25-50 mg/day to 12.5-100 mg/day, more preferably from 9.375-75 mg/day to 12.5-100 mg/day

Metolazone:
7-chloro-2-methyl-3-(2-methylphenyl)-4-oxo-1,2-dihydroquinazoline-6-sulfonamide Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day

Indapamide:
4-chloro-N-(2-methyl-2,3 -dihydroindol-1-yl)-3-sulfamoylbenzamide
Dosage range from 0.625-1.25 mg/day to 2.5-5 mg/day, preferable from 1.25-2.5 mg/day to 2.5-5 mg/day, more preferably from 1.875-3.75 mg/day to 2.5-5 mg/day

### Gliflozine

Dapagliflozin:
(2S,3R,4R,5S,6R)-2-[4-chloro-3-[(4-ethoxyphenyl)methyl]phenyl]-6-(hydroxymethyl)oxane-3,4,5-triol
Dosage range from 2.5 mg/day to 10 mg/day, preferable from 5 mg/day to 10 mg/day, more preferably from 7.5 mg/day to 10 mg/day

Canagliflozin:
(2S,3R,4R,5S,6R)-2-[3-[[5-(4-fluorophenyl)thiophen-2-yl]methyl]-4-methylphenyl]-6-(hydroxymethyl)oxane-3,4,5-triol
Dosage range from 75 mg/day to 300 mg/day, preferable from 150 mg/day to 300 mg/day, more preferably from 225 mg/day to 300 mg/day

Empagliflozin :
(2S,3R,4R,5S,6R)-2-[4-chloro-3-[[4-[(3S)-oxolan-3-yl]oxyphenyl]methyl]phenyl]-6-(hydroxymethyl)oxane-3,4,5-triol
Dosage range from 6.25 mg/day to 25 mg/day, preferable from 10 mg/day to 25 mg/day, more preferably from 10 mg/day to 20 mg/day

Ertugliflozin :
(1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol
Dosage range from 3.75 mg/day to 15 mg/day, preferable from 7.5 mg/day to 15 mg/day, more preferably from 11.25 mg/day to 15 mg/day

Ipragliflozin
(2S,3R,4R,5S,6R)-2-[3-(1-benzothiophen-2-ylmethyl)-4-fluorophenyl]-6-(hydroxymethyl)oxane-3,4,5-triol
Dosage range from 25 mg/day to 100 mg/day, preferable from 50 mg/day to 100 mg/day, more preferably from 75 mg/day to 100 mg/day

Tofogliflozin
(3S,3'R,4'S,5'S,6'R)-5-[(4-ethylphenyl)methyl]-6'-(hydroxymethyl)spiro[1H-2-benzofuran-3,2'-oxane]-3',4',5'-triol
Dosage range from 5 mg/day to 20 mg/day, preferable from 10 mg/day to 20 mg/day, more preferably from 15 mg/day to 20 mg/day

Luseogliflozin
(2S,3R,4R,5S,6R)-2-[5-[(4-ethoxyphenyl)methyl]-2-methoxy-4-methylphenyl]-6-(hydroxymethyl)thiane-3,4,5-triol
Dosage range from 1.25 mg/day to 5 mg/day, preferable from 2.5 mg/day to 5m g/day, more preferably from 3.75 mg/day to 5 mg/day

Sotagliflozin:
(2S,3R,4R,5S,6R)-2-[4-chloro-3-[(4-ethoxyphenyl)methyl]phenyl]-6-methylsulfanyloxane-3,4, 5-triol
Dosage range from 100 mg/day to 400 mg/day, preferable from 200mg/day to 400 mg/day, more preferably from 300 mg/day to 400 mg/day.

18. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising at least three of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), e.g. dapagliflozin or empagliflozin, and wherein said inhibitor of the sodium glucose co-transporter-2, e.g. gliflozine or empagliflozin, is used for restoring the restoration rate and/or maintaining (a normal) respiration rate and/or restoring the oxygen saturation and/or for ameliorating the symptoms of dyspnea;
e. a diuretic to reduce signs and/or symptoms of congestion in patients,
and wherein a sample of bodily fluid of said patient has a level of brain natriuretic peptide (BNP) > 200 pg/mL and/or has a level of N-terminal brain natriuretic peptide (NT-proBNP) > 600 pg/mL and/or the patient is congestive and/or has residual congestion, and wherein said sample of bodily fluid is selected from the group comprising blood, plasma and serum,
and wherein the compounds are administered to said patient at the same time in one single administration form, preferably in one single pill,
and wherein said patient is either
   - a patient with heart failure with LVEF greater and equal to 40 %, preferably 50 % or
   - a patient with no heart failure.

19. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising at least four of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor andneprilysin inhibitor (ARNi)
c. and mineralo receptor antagonist (MRA),
d. a diuretic to reduce signs and/or symptoms of congestion in patients,
   and wherein a sample of bodily fluid of said patient has a level of brain natriuretic peptide (BNP) > 200 pg/mL and/or has a level of N-terminal brain natriuretic peptide (NT-proBNP) > 600 pg/mL, and/or the patient is congestive and/or has residual congestion, and wherein said sample of bodily fluid is selected from the group comprising blood, plasma and serum,
   and wherein said patient is either
      - a patient with heart failure with LVEF greater and equal to 40 %, preferably 50 %, or
      - a patient with no heart failure.

20. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising at least four compounds wherein each of said chemical compounds is a chemical entity, wherein each chemical entity is distinct from the other two:
a. Bisoprolol as a beta blocker (BB),
b. Ramipril as one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. a diuretic to reduce or prevent occurrence of signs and/or symptoms of congestion in patients
   and wherein a sample of bodily fluid of said patient has a level of brain natriuretic peptide (BNP) > 200 pg/mL and/or has a level of N-terminal brain natriuretic peptide (NT-proBNP) > 600 pg/mL, and/or the patient is congestive and/or has residual congestion, and wherein said sample of bodily fluid is selected from the group comprising blood, plasma and serum,
   and wherein said patient is either
      - a patient with heart failure with LVEF, greater and equal to 40 %, preferably 50 % or
      - a patient with no heart failure.

21. Pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination medication according to any of embodiments 1 - 21, and wherein a sample of bodily fluid of said patient has a level of BNP > 200 pg/mL and/or has a level NT-proBNP > 600 pg/mL, and/or the patient is congestive, and/or has residual congestion and wherein said sample of bodily fluid is selected from the group comprising blood, plasma and serum.

22. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to embodiment 1 and following embodiments comprising at least three of the following compounds wherein each of said chemical compounds is a chemical entity, wherein each chemical entity is distinct from the other two :
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and inhibitor (ARNi)
c. and mineralo receptor antagonist (MRA),
d. an inhibitor of the sodium glucose co-transporter-2 (SGLT2), e.g. dapagliflozin or empagliflozin, and wherein said inhibitor of the sodium glucose co-transporter-2, e.g. gliflozine or empagliflozin, is used for restoring the restoration rate and/or maintaining the respiration rate and/or restoring or maintaining the oxygen saturation and/or for ameliorating the symptoms of dyspnea,
e. a diuretic to reduce signs and/or symptoms of congestion in patients
and wherein a sample of bodily fluid of said patient has a level of BNP > 200 pg/mL and/or has a level NT-proBNP > 600 pg/mL, and/or the patient is congestive and/or has residual congestion, and wherein said sample of bodily fluid is selected from the group comprising blood, plasma and serum,
wherein said patient is
   - a patient with heart failure with LVEF, greater and equal to 40 %, preferably 50 % or
   - a patient with no heart failure, and
wherein the treatment is a daily administration of the combination medication, preferably once a day, and
wherein said combination is a fixed combination and wherein said combination is a fixed combination in one administration form, wherein said administration form is selected from the group comprising a pill, a tablet, a liqiuid medicine, a capsule, a film tablet, a dragee, and
wherein the level of proADM and/or fragments thereof having at least 5 amino acids in a sample of bodily fluid of said patient is above a certain threshold, and wherein said sample of bodily fluid is selected from the group comprising plasma, serum, blood, and wherein said patient receives the combination medication in two different doses, which are named dose No 1 and dose No 2, and
wherein said patient receives a dose No 1 of said combination medication for up to 2 to 4 weeks and thereafter a dose No 2 for at least 90 days, and
wherein dose No 1 is a quarter dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

23. Combination medication for use in the treatment of a patient having acute and/or persistent dyspnea according to embodiment 1 and following embodiments comprising at least three of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and-neprilysin inhibitor (ARNi)
c. and mineralo receptor antagonist (MRA),
d. an inhibitor of the sodium glucose co-transporter-2 (SGLT2), e.g. dapagliflozine or empagliflozin, and wherein said inhibitor of the sodium glucose co-transporter-2, e.g. gliflozine or empagliflozin, is used for restoring the restoration rate and/or maintaining (a normal) respiration rate and/or restoring the oxygen saturation and/or for ameliorating the symptoms of dyspnea,
e. a diuretic to reduce signs and/or symptoms of congestion in patients,
and wherein a sample of bodily fluid of said patient has a level of BNP > 200 pg/mL and/or has a level NT-proBNP > 600 pg/mL, and/or the patient is congestive and/or has residual congestion, and wherein said sample of bodily fluid is selected from the group comprising blood, plasma and serum, and
wherein the treatment is a daily administration of the combination medication, preferably once a day, and
wherein the patient has no heart failure but has another cardiovascular disease and/or another disease with non cardiac cause which maybe selected from the group comprising diabetes and kidney disease,
and wherein said combination is a fixed combination and wherein said combination is a fixed combination in one administration form, wherein said administration form is selected from the group comprising a pill, a tablet, a liqiuid medicine, a capsule, a film tablet, a dragee,
and wherein the level of proADM and/or fragments thereof having at least 5 amino acids in a sample of bodily fluid of said patient is above a certain threshold, wherein said sample of bodily fluid is selected from the group comprising plasma, serum, blood,
and wherein said patient receives a dose No 1 of said combination medication for up to 2 to 4 weeks and thereafter a dose No 2 for at least 90 days, wherein dose No 1 is half dose (25%) of dose No 2, more preferably .dose No 1 is 35% of dose No 2, more preferably .dose No 1 is 40% of dose No 2, more preferably .dose No 1 is 45% of dose No 2, more preferably dose No 1 is 50% of dose No 2.

24. Combination medication for use in the treatment of a patient having acute and/or persistent dyspnea for restoring the respiration rate and/or oxygen saturation toward normal values and/or maintaining the respiration rate and/or restoring and/or maintaining the oxygen saturation and/or for ameliorating the symptoms of dyspnea comprising at least three of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozin or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea;
e. a diuretic to reduce or prevent occurrence of signs and/or symptoms of congestion in patients,
and wherein a sample of bodily fluid of said patient has a level of Brain Natriuretic peptide (BNP) > 200 pg/mL and/or has a level of N-terminal (NT)-pro hormone BNP (NT-proBNP) > 600 pg/mL and/or the patient is congestive and/or has residual congestion, and wherein said sample of bodily fluid is selected from the group comprising blood, plasma and serum;
and wherein said patient is either
- a patient with heart failure with left ventricular ejection fraction (LVEF) with less than 40 %, or
- a patient with no heart failure.

### SEQUENCES

SEQ ID No. 1 (proADM): 164 amino acids (22 - 185 of preproADM) 
ARLDVASEF RKKWNKWALS RGKRELRMSS SYPTGLADVK AGPAQTLIRP
QDMKGASRSP EDSSPDAARI RVKRYRQSMN NFQGLRSFGC RFGTCTVQKL
AHQIYQFTDK DKDNVAPRSK ISPQGYGRRR RRSLPEAGPG RTLVSSKPQA
HGAPAPPSGS APHFL
SEQ ID No. 2 (Proadrenomedullin N-20 terminal peptide, PAMP): amino acids 22 - 41 of preproADM
   ARLDVASEF RKKWNKWALS R
SEQ ID No. 3 (Midregional proAdrenomedullin, MR-proADM): amino acids 45 - 92 of preproADM
   ELRMSS SYPTGLADVK AGPAQTLIRP QDMKGASRSP EDSSPDAARI RV
SEQ ID No. 4 (mature Adrenomedullin (mature ADM); amidated ADM; bio-ADM): amino acids 95 - 146 -CONH₂
   YRQSMN NFQGLRSFGC RFGTCTVQKL AHQIYQFTDK DKDNVAPRSK ISPQGY - CONH₂
SEQ ID No. 5 (Adrenomedullin 1-52-Gly (ADM 1-52-Gly)): amino acids 95 - 147 of preproADM
   YRQSMN NFQGLRSFGC RFGTCTVQKL AHQIYQFTDK DKDNVAPRSK ISPQGYG
SEQ ID No. 6 (C-terminal proAdrenomedullin, CT-proADM): amino acids 148 - 185 of preproADM
   RRR RRSLPEAGPG RTLVSSKPQA HGAPAPPSGS APHFL

### FIGURE DESCRIPTION

Figure 1: Amount of Bisoprolol dissolved according to time for each of the formulations
Figure 2: Amount of ramipril dissolved according to time for each of the formulations
Figure 3: Amount of Spironolactone dissolved according to time for each of the formulations
Figure 4: Amount of Furosemide dissolved according to time for each of the formulations

### Examples

### Example 1

### Generation of Antibodies and determination of their affinity constants

WO2018/007588 describes the development of the mouse monoclonal antibodies binding to the N-terminal (NT-ADM), mid-regional (MR-ADM) and C-terminal (CT-ADM) part of ADM and the determination of their affinity constants as follows (Table 1):
"It should be emphasized that the antibodies, antibody fragments and non-Ig scaffolds of the example portion in accordance with the invention are binding to ADM, and thus should be considered as anti-ADM antibodies/antibody fragments/non-Ig scaffolds.

### Peptides for immunization

Peptides were supplied by JPT Peptide Technologies GmbH (Berlin, Germany). Peptides were coupled to BSA using the Sulfo-SMCC crosslinking method. The crosslinking procedure was performed according the manufacturers instructions (Thermo Fisher/ Pierce).

### Generation of murine antibodies

A Balb/c mouse was immunized with 100 µg Peptide-BSA-Conjugate at day 0 and 14 (emulsified in 100 µl complete Freund's adjuvant) and 50 µg at day 21 and 28 (in 100 µl incomplete Freund's adjuvant). Three days before the fusion experiment was performed, the animal received 50 µg of the conjugate dissolved in 100 µl saline, given as one intraperitoneal and one intra venous injection.

Splenocytes from the immunized mouse and cells of the myeloma cell line SP2/0 were fused with 1 ml 50% polyethylene glycol for 30 s at 37°C. After washing, the cells were seeded in 96-well cell culture plates. Hybrid clones were selected by growing in HAT medium (RPMI 1640 culture medium supplemented with 20% fetal calf serum and HAT-Supplement). After two weeks the HAT medium is replaced with HT medium for three passages followed by returning to the normal cell culture medium.

The cell culture supernatants were primary screened for antigen specific IgG antibodies three weeks after fusion. The positive tested microcultures were transferred into 24-well plates for propagation. After retesting the selected cultures were cloned and recloned using the limiting-dilution technique and the isotypes were determined (Lane, 1985. J. Immunol. Meth. 81: 223-228*;* Ziegler et al. 1996. Horm. Metab. Res. 28: 11-15).

**Table 1:**

| Antigen/Immunogen | ADM Region | Designation | Affinity constants Kd (M⁻¹) |
|---|---|---|---|
| | | | |
| YRQSMNNFQGLRSFGC (SEQ ID NO. 7) | 1-16 | NT-ADM | 1,6 x 10⁹ |
| CTVQKLAHQIYQ (SEQ ID NO. 8) | 21-32 | MR-ADM | 2 x 10⁹ |
| CAPRSKISPQGY-NH2 (SEQ ID NO. 9) | C-42-52 | CT-ADM | 1.1 x 10⁹ |

### Monoclonal antibody production

Antibodies were produced via standard antibody production methods (Marx et al, 1997. Monoclonal Antibody Production, ATLA 25, 121) and purified via Protein A. The antibody purities were > 95% based on SDS gel electrophoresis analysis.

### Affinity Constants

To determine the affinity of the antibodies to Adrenomedullin, the kinetics of binding of Adrenomedullin to immobilized antibody was determined by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare).

### Labelling procedure (tracer)

100 µg (100 µl) of antibody (1 mg/ ml in PBS, pH 7.4,) was mixed with 10 µl Akridinium NHS-ester (1 mg/ml in acetonitrile, InVent GmbH, Germany) (EP 0 353 971) and incubated for 20 min at room temperature. Labelled CT-H was purified by Gel-filtration HPLC on Bio-Sil^{®} SEC 400-5 (Bio-Rad Laboratories, Inc., USA). The purified labeled antibody was diluted in
(300 mmol/L potassiumphosphate, 100 mmol/L NaCl, 10 mmol/L Na-EDTA, 5 g/L Bovine Serum Albumin, pH 7.0). The final concentration was approx. 800.000 relative light units (RLU) of labelled compound (approx. 20 ng labeled antibody) per 200 µL. Akridiniumester chemiluminescence was measured by using an AutoLumat LB 953 (Berthold Technologies GmbH & Co. KG).

### Solid phase

Polystyrene tubes (Greiner Bio-One International AG, Austria) were coated (18 h at room temperature) with antibody (1.5 µg antibody/0.3 mL 100 mmol/L NaCl, 50 mmol/L TRIS/ HCl, pH 7.8). After blocking with 5% bovine serum albumine, the tubes were washed with PBS, pH 7.4 and vacuum dried.

### Calibrators

Synthetic human ADM (Bachem, Switzerland) was linearily diluted using 50 mM Tris/ HCl, 250 mM NaCl, 0.2% Triton X-100, 0.5% BSA, 20 tabs/L Protease Complete Protease Inhibitor Cocktail Tablets (Roche AG); pH 7.8. Calibrators were stored at -20 °C before use."

### Example 2

### Determination of the antibody combination that yields high signal/noise ratios

ADM Immunoassay as described in WO2018/007588:
50 µl of sample (or calibrator) was pipetted into coated tubes, after adding labeled second antibody (200 µl), the tubes were incubated for 2 h at room temperature. Unbound tracer was removed by washing 5 times (each 1 ml) with washing solution (20 mM PBS, pH 7.4, 0.1 % Triton X-100). Tube-bound chemiluminescence was measured by using the LB 953 (Berthold Technologies GmbH & Co. KG).

All antibodies were used in a sandwich immunoassay as coated tube and labeled antibody and combined in the following variations (see Table 2). Incubation was performed as described under hADM-Immunoassay. Results are given in ratio of specific signal (at 10 ng/ml ADM) /background (sample without ADM) signal.

**Table 2:**

| Signal/ noise ratio | NT-ADM tracer | MR-ADM tracer | CT-ADM tracer |
|---|---|---|---|
| NT-ADM | / | 195 | 241 |
| MR-ADM | 204 | / | 904 |
| CT-ADM | 260 | 871 | / |

Surprisingly, they found the combination of MR-ADM and CT-ADM as combination for highest signal/noise ratio.

Subsequently, they used this antibody-combination for further investigations. They used MR-ADM as solid phase antibody and CT-ADM as labeled antibody. The analytical sensitivity (average of 10 runs, ADM-free sample + 2SD) of the assay was 2 pg ADM/ml.

### Example 3

Stability of human Adrenomedullin as described in WO2018/007588:
Human ADM was diluted in human Citrate plasma (n=5, final concentration lOng ADM/ml) and incubated at 24 °C. At selected time points, aliquots were frozen at -20 °C. Immediately after thawing the samples hADM was quantified by using the hADM immunoassay described above.

**Table 3 shows the stability of hADM in human plasma at 24 °C.**

| Time (h) | Average ADM recovery (N=5) | Relative loss of immune reactivity | Loss of immune reactivity %/ h |
|---|---|---|---|
| 0 | 100 | / | / |
| 2 | 99,2 | 0,8 | 0,4 |
| 4 | 96,4 | 3,6 | 0,8 |
| 8 | 88,2 | 11,8 | 1,5 |
| | | | Average: 0.9%/ h |

Surprisingly, using the antibody-combinations MR-ADM and CT-ADM in a sandwich immune assay, the pre-analytical stability of the analyte is high (only 0.9%/h average loss of immune reactivity). In contrast, using other assay methods, a plasma half-life of only 22 min was reported (Hinson et al. 2000 Endocrine Reviews 21(2):138-167). Since the time from taking sample to analysis in hospital routine is less than 2h, the used ADM detection method is suitable for routine diagnosis. It is remarkable, that any non-routine additives to samples (like aprotinin, (Ohta et al. 1999. Clin Chem 45 (2): 244-251)) are not needed to reach acceptable ADM-immune reactivity stabilities.

### Example 4

Reproducibility of calibrator-preparations as described in WO2018/007588 as follows: They found a high variation of results, preparing calibrators for ADM assays (average CV 8.5%, see Table 4). This may be due to high adsorption of hADM to plastic and glass surfaces (Lewis et al. 1998. Clinical Chemistry 44 (3): 571-577). This effect was only slightly reduced by adding detergents (up to 1% Triton X 100 or 1% Tween 20), protein (up to 5% BSA) and high ionic strength (up to 1M NaCl) or combinations thereof. Surprisingly, if a surplus of anti ADM antibody (10µg/ml) is added to the calibrator dilution buffer, the recovery and reproducibility of ADM assay calibrator-preparations was substantially improved to < 1% of inter preparation CV (Table 4).

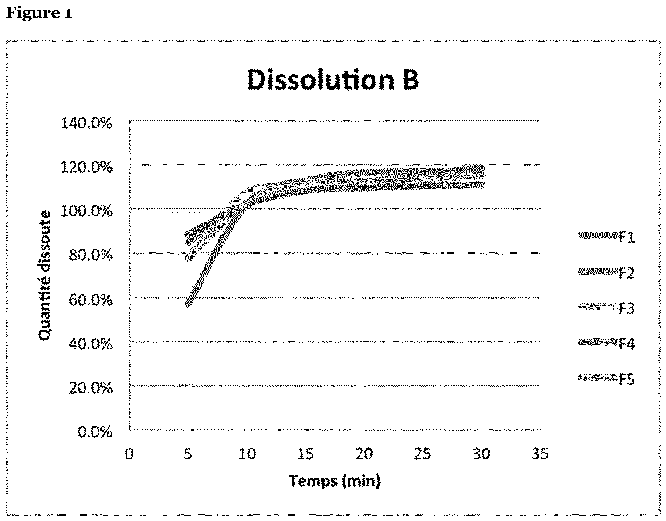

Fortunately, the presence of N-terminal antibodies did not affect the ADM-signal generated by the combination of MR- and C-terminal antibodies (Fig. 1).

**Table 4:**

| | In the presence of NT-ADM antibody (10µg/ml) | Inter preparation CV (%) | Without antibody | Inter preparation CV (%) |
|---|---|---|---|---|
| calibrator | | | | |
| 100ng/ml | 3453 s/n-r | 0.9 | 2842 s/n-r | 2.8 |
| 10ng/ml | 1946 s/n-r | 0.8 | 1050 s/n-r | 7.9 |
| 1ng/ml | 179 s/n-r | 1.1 | 77 s/n-r | 14.8 |
| | | Average: 0.93 | | Average: 8.5 |

### Interpreparation variation of calibrators

ADM assay calibrators were prepared as described above with and without 10µg/ml of NT-ADM-antibody. Coeffients of variation are given from 5 independent preparation runs. The calibrators were measured using the ADM assay described above (s/n-r = signal to noise ratio). For all following studies, we used an ADM assay, based on calibrators, prepared in the presence of 10µg/ml of NT-ADM antibody and 10µg/ml of NT-ADM antibody as supplement in the tracer buffer.

### Example 5

Determining the Sensitivity as described in WO2018/007588:
The goal of assay sensitivity was to completely cover the ADM concentration of healthy subjects.

### ADM concentration in healthy subjects

Healthy subjects (n=100, average age 56 years) were measured using the ADM assay. The median value was 24.7 pg/ml, the lowest value 11 pg/ml and the 99^{th} percentile 43 pg/ml. Since the assay sensitivity was 2 pg/ml, 100% of all healthy subjects were detectable using the described ADM assay.

A commercial fully automated homogeneous time-resolved fluoroimmunoassay was used to measure MR-proADM in plasma (BRAHMS MR-proADM KRYPTOR; BRAHMS GmbH, Hennigsdorf, Germany) (Caruhel et al. 2009. Clin Biochem. 42 (7-8): 725-8).

### Example 6:

### Study for combination medication

The aim of the study is to follow patients with dyspnea and see how therapies and frequent visits might accelerate improvement.

Patients were included in 3 emergency rooms with the following inclusion criteria : Breathlessness ; Oxygen saturation lower than 95% ; respiration rate greater than 20 /min. Exclusion criteria were age below 18 or greater than 85 ; pregnant women ; history of asthma in young age ; pulmonary embolism ; heart failure with ejection fraction below 40%. Protocol was approved by ethics committee and patients were informed and gave signed consent.

Patients were discharged from emergency room toward hospitals ward or at home when treating physicians felt appropriate. Between discharge and 90 days, patients were managed as decided by treating physicians following roughly 2 procedures : regular therapies and follow-up visit in the outpatient department once at Day 90 or intense inhibition of neuroendocrine system as previously described (Kimmoun A et al., Safety, Tolerability and efficacy of Rapid Optimization, helped by NT-proBNP and GDF-15, of Heart Failure therapies (STRONG-HF): rationale and design for a multicentre, randomized, parallel-group study.; Eur J Heart Fail. 2019 Nov;21(11):1459-1467) associated with repeated follow-up at hospital at day 15, 30, 60 and 90 days and, if needed and possible, there were extra visits at home by a nurse and/or a physician at day 7, 21, 45 and 75.

Parameters recorded at admission and during outpatient or home visits included NHYA class, heart rate, blood pressure, respiration rate, Oxygen saturation, body weight, NT-proBNP.

We report results of 172 patients admitted for breathlessness at the emergency room: 109 had cardiac cause and 63 non-cardiac cause of breathlessness. At admission, median body weight was 80.5 kg, systolic blood pressure 150 mmHg, diastolic blood pressure 80 mmHg, heart rate 100 beat per min, NYHA 2, respiration rate 26 breath/min, oxygen saturation 89 % and plasma NT-proBNP 3908 pg/ml. Patients had all appropriates therapies for breathlessness in the emergency room and in the hospital.

At discharge, 129 patients had regular procedure including usual discharge therapies and a visit at day 90 while 43 patients had intensive inhibition of neuroendocrine system and close follow up. Of note, at admission, patients with usual follow-up had similar body weight, respiration rate, heart rate, blood pressure, NYHA and oxygen saturation than patients with intensive endocrine inhibition.

Ninety days after discharge from breathlessness, treatment in the usual follow-up group included few cardiovascular medications: 25% of the patients in this group had 3 or 4 cardiovascular medications. In addition, beta-blockers when administered was mostly at quarter dose and ACE inhibitors or ARBs mostly at half dose. Treatment in intensive endocrine inhibition arm at day 90 included 3 or 4 cardiovascular medications in 79% of the patients. In addition, beta-blockers and ACE inhibitors or ARB were quasi-exclusively taken at full dose. In the latter arm, few patients also received an inhibitor of the sodium glucose co-transporter-2 (SGLT2).

At Day 90, patients in the intense group had a markedly better respiratory condition and well being compared to usual group including lower respiratory rate (median 13 versus 20 respectively, p = 0.0008), lower NYHA (1 versus 2 respectively, p = 0.006), better Oxygen saturation 97 versus 96% respectively, p = 0.0215), lower heart rate (67 versus 79 respectively, p = 0.0014), lower diastolic blood pressure 67 versus 80 mmHg, p = 0.0365) while sytolic blood pressure, NT-proBNP and body weight were similar.

In intensive endocrine inhibition arm, patients were follow-up closely. This allowed to see that improvement in respiratory condition and well being were already seen 7 days after initiation of therapy (n=16): Respiration rate was at 13 breaths/min, similar to 14 breaths/min at day 90 (p = 0.80), heart rate was at 63 bpm, similar to 66 breaths/min at day 90 (p = 1.0), oxygen saturation was at 98 %, similar to 97% at day-90 (p = 0.82). NHYA class at day 15 improved at level similar to day-90 : NHYA class 1 similar to NYHA class 1 day-90 (p = 0.50). Improvement in respiratory condition and well being in intensive group versus usual group was seen in the whole studied group ofpatients with dyspnea whether this was related to cardiac or non cardiac cause. Thus, at Day 90, when dyspnea was related to cardiac cause, patients in the intense group had a better respiratory condition and well being compared to usual group including lower respiratory rate (median 13 versus 20 respectively, p = 0.0011), lower NYHA (median 1 versus 2 (mean 1.8 versus 1.5) respectively, p = 0.178), better oxygen saturation 98 % versus 96 % respectively, p = 0.0215), lower heart rate (75 versus 67 respectively, p = 0.0014). At Day 90, when dyspnea was related to non-cardiac cause, patients in the intense group had a better respiratory condition and well being compared to usual group including lower respiratory rate (median 14 versus 19 respectively, p = 0.249), well being (median 1 versus 1, (mean 1.7 versus 1.1) respectively, p = 0.0099), better oxygen saturation 97 % versus 96 % respectively, p = 0.30), lower heart rate (68 versus 84 respectively, p = 0.0235). Furthermore, mortality was lower in intensive arm, 4.7% compared to 14.7 % in the control arm. This was true, in all studied patients, when dyspnea was from cardiac causes 4.2% versus 15.3% or non-cardiac causes. There was also a trend in favor of less readmission: 14% in the intensive arm versus 15.5 % in the control arm.

In another study we showed that combination of plasma levels of biologically active form of adrenomedullin and natriuretic peptides may identifiy patients who will better benefit from the association of beta-blockers and angiotensin converting enzyme inhibitors. In 807 patients admitted with acute dyspnea, those with plasma levels of biologically active of adrenomedulline greater than 35 pg/ml and of NT-proBNP greater than 2000 pg/ml (n=293) had a reduction by half the risk of death HR 0.50 [interquartile range 0.37-0.68] when treated with beta-blockers and angiotensin converting enzyme inhibitors.

Conclusion of the study with arguments in favour of the patent application. In patient having acute and/or persistent dyspnea, intensive treatment regimen leads to:
- lower breathing rate, lower NYHA, better oxygen saturation with the
- Safety and effectiveness of simultaneous administration
- simplification of administration

### Example 7:

### Galenic study

The objective of the present study is the development of an immediate-release capsule containing four active ingredients: Bisoprolol, Ramipril, Spironolactone and Furosemide. Thus, in a single dose, the administration of drugs is facilitated and compliance in dyspneic patients is increased.

The capsules were prepared manually using a size 1 capsule filler.

Capsule size n ° 1 was chosen taking into account any swallowing problems that may be encountered in the elderly.

The recommended doses of active ingredients were established according to the 2016 ESC Guidelines for the diagnosis and treatment of acute and chronic heart failure.

The excipients used are Crospovidone XL, CMC PH102, Granulac 140 and magnesium stearate.

Five formulations were made. From one formulation to another, only the proportion of Crospovidone (desintegrant) and CMC (binder, diluent) was changed.

Dissolution studies were carried out at t = 0 with reference to the USP monographs for the 4 active ingredients.

The results obtained show that the capsules therefore comply with the dissolution test of USP monograph for each active ingredient used and mixed in the capsule.

Stability studies are currently underway.

### Materials and methods

### • Materials

The active ingredients and the excipients used are mentioned in the table below:

**Table 1: List of active ingredients and excipients, their category, source and country of manufacture**

| **Name** | **Category** | **Source** | **Country** |
|---|---|---|---|
| Bisoprolol | Active ingredient | Supriya Lifescience LTD. | India |
| Ramipril | Active ingredient | Zhejiang Huahai Pharm. | China |
| Spironolactone | Active ingredient | Zhejiang Langhua Pharm. | China |
| Furosemide | Active ingredient | Changzhou Yabang pharm. | China |
| Crospovidone Ph. Eur VIVAPHARM PVPP XL | Disintegrant | JRS PHARMA | China |
| CMC NF, Ph. Eur VIVAPUR 102 | Binder, Diluent | JRS PHARMA | Germany |
| Lactose Monohydrate Ph. Eur Granulac 140 | Diluent | MEGGLE | Germany |
| Magnesium Stearate | Lubricant | Peter Greven | Netherlands |
| Gelatin capsule n ° 1 | - | Capsugel | Belgium |

The materials and devices used are listed in the table below:

| **Devices and Materials** | **Provider** | **City Country** | **Terms of use** |
|---|---|---|---|
| Precision scale | OHAUS Voyager.Pro | USA | Max: 210g d: 0.1 mg |
| Full Metal 400 capsule filler Series 40190064 | Feton | Strépy-Bracquegnies Belgium | Capsule size: 1 Capacity: 400 capsules |
| Charger Line 400 loaders Reference: CH400 | Feton | Strépy-Bracquegnies Belgium | Capsule size: 1 Capacity: 400 capsules |
| Receipt printer | OHAUS SF40A | USA | - |
| Porcelain mortar | - | - | Capacity 100g |
| A graduated cylinder of 250 ml | - | - | - |
| A funnel | - | - | - |
| 1250 Capsules | - | - | Size: 1 |

| | | | |
|---|---|---|---|
| *Table 2: Details of the equipment and devices used* | | | |

### •Manufacturing:

Each of the formulations were manufactured manually by a pharmacist according to the proportions and amount described below:

**Table 3: Proportions and quantities of active ingredients and excipients per capsule. Total weight per capsule: 242.9 mg**

| | **Formulation 1** | | **Formulation 2** | | **Formulation 3** | | **Formulation 4** | | **Formulation 5** | |
|---|---|---|---|---|---|---|---|---|---|---|
| | **Proport ion** | **Amoun t** | **Proporti on** | **Amount** | **Proport ion** | **Amount** | **Proport ion** | **Amount** | **Proport ion** | **Amount** |
| **Bisoprolol** | 4.12% | 10 mg | 4.12% | 10 mg | 4.12% | 10 mg | 4.12% | 10 mg | 4.12% | 10 mg |
| **Ramipril** | 4.12% | 10 mg | 4.12% | 10 mg | 4.12% | 10 mg | 4.12% | 10 mg | 4.12% | 10 mg |
| **Furosemide** | 32.94% | 80 mg | 32.94% | 80 mg | 32.94% | 80 mg | 32.94% | 80 mg | 32.94% | 80 mg |
| **Spironolacto ne** | 10.29% | 25 mg | 10.29% | 25 mg | 10.29% | 25 mg | 10.29% | 25 mg | 10.29% | 25 mg |
| **Crospovidon e XL** | **10.25%** | **24.90 mg** | **15.07%** | **36.6 mg** | **19.89%** | **48.31 mg** | **24.70%** | **60 mg** | **29.51 %** | **71.68 mg** |
| **CMC PH102** | **29.51%** | **71.68 mg** | **24.70%** | **60 mg** | **19.89%** | **48.31 mg** | **15.07%** | **36.6 mg** | **10.25%** | **24.90 g** |
| **Granulac 140** | 8.15% | 19.8 mg | 8.15% | 19.8 mg | 8.15% | 19.8 mg | 8.15% | 19.8 mg | 8.15% | 19.8 mg |
| **Mg stearate** | 0.62% | 1.5 mg | 0.62% | 1.5 mg | 0.62% | 1.5 mg | 0.62% | 1.5 mg | 0.62% | 1.5 mg |

For each formulation 250 capsules were manufactured.

The active ingredients and the excipients were carefully weighed on a precision balance.

As Furosemide is sensitive to light, it was weighed last in minimum light.

From there, the operator worked with minimal exposure of the Furosemide to light.

The mixing of the active ingredients and the excipients was carried out in a porcelain mortar in a precise order in order to guarantee the homogeneity of the mixture.

The measured volume of the mixture after several compactings was 125 ml (ie 250 capsules of 0.5 ml).

The capsule filler was placed on a flat, dry and clean surface. The level filling of the 250 capsules was carried out uniformly using all the powder.

The capsules were finally packaged in an opaque pillbox with a dehydrating cap containing 30 capsules.

### • Dissolution studies:

Dissolution of Bisoprolol:
The standard aqueous solution of Bisoprolol was prepared at a concentration of 0.005mg/ml.

Two aqueous blanks, one containing only the excipients and the other containing the excipients and the three active ingredients other than Bisoprolol were prepared.

900 ml of water were placed in each dissolving vessel, the temperature was set at 37°C and the speed of rotation at 75 rpm. 6 capsules of each formulation were transferred to each vessel. The paddles were submerged in the vessels.

At t = 5, 10, 15, 20 and 30 min, samples of 5 ml were taken and diluted by two in a mixture of methanol, triethylamine, phosphoric acid and water (160: 5: 2.5: 35).

The analysis was carried out in LC mode coupled to a UV detector at 227nm. The column used is a LiChrosorb RP-8 5 µm Hibar RT 250-4.6. The elution rate was set at 1 ml/min and the injection volume at 50 µL. The mobile phase was a mixture of methanol, triethylamine and water (34: 1: 50) at pH 4.0 ± 0.1.

Dissolution of Ramipril:
Ramipril standard solution was prepared at a concentration of 0.01 mg/ml.

Two blanks, one containing only the excipients and the other containing the excipients and the three active ingredients other than Ramipril were prepared in 0. IN hydrochloric acid.

500 ml of 0.1N hydrochloric acid were placed in each dissolution vessel, the temperature was adjusted to 37°C and the speed of rotation to 75 rpm. 6 capsules of each formulation were transferred to each vessel. The paddles were submerged in the vessels.

At t = 5, 10, 15, 20 and 30 min, samples of 5 ml were taken.

The analysis was carried out in LC mode coupled to a UV detector at 210 nm. The column used is a BDS Hypersil C18 150mm x 4.6mm 5 µm. The elution rate was set at 1 ml /min and the injection volume at 100 µL. The mobile phase was a mixture of sodium perchlorate, triethylamine, acetonitrile and water.

Dissolution of Spironolactone:
The standard solution of Spironolactone was prepared at a concentration of 0.025 mg/ml. Two blanks, one containing only the excipients and the other containing the excipients and the three active ingredients other than Spironolactone were prepared in 0.1N hydrochloric acid containing 0.1% sodium lauryl sulfate.

1L of 0.1N hydrochloric acid containing 0.1% sodium lauryl sulfate were placed in each dissolution vessel, the temperature was set at 37°C and the speed of rotation at 75 rpm. 6 capsules of each formulation were transferred to each vessel. The paddles were submerged in the vessels.

At t = 15, 30, 45 and 60 min, samples of 5 ml were taken.

Analysis was performed by UV spectroscopy at 242 nm.

Dissolution of Furosemide:
The standard solution of Furosemide was prepared at a concentration of 0.01 mg/ml in phosphate buffer at pH 5.8.

Two blanks, one containing only the excipients and the other containing the excipients and the three active ingredients other than Furosemide were prepared in the phosphate buffer at pH 5.8.

900 ml of phosphate buffer at pH 5.8 were placed in each dissolution vessels, the temperature was adjusted to 37 ° C and the speed of rotation at 50 rpm. 6 capsules of each formulation were transferred to each vessel. The paddles were submerged in the vessels.

At t = 15, 30, 45 and 60 min, samples of 5 ml were taken and diluted in the dissolution medium.

Analysis was performed by UV spectroscopy at 274 nm.

### Results

The table results of the quantities of active ingredients dissolved according to time for each of the molecules:

**Table 4: Amount of Bisoprolol dissolved according to time for each of the formulations**

| **Time (min)** | **Amount dissolved F1** | **Amount dissolved F2** | **Amount dissolved F3** | **Amount dissolved F4** | **Amount dissolved F5** |
|---|---|---|---|---|---|
| **5** | 57.0% | 84.8% | 77.8% | **88.3%** | 77.3% |
| **10** | 102.4% | 102.4% | 107.6% | **102.0%** | 103.1% |
| **15** | 112.8% | 112.6% | 108.6% | **108.2%** | 112.2% |
| **20** | 112.4% | 116.4% | 111.4% | **109.6%** | 112.2% |
| **30** | 118.8% | 117.0% | 115.2% | **111.0%** | 115.5% |

**Table 5: Amount of ramipril dissolved according to time for each of the formulations**

| **Time (min)** | **Amount dissolved F1** | **Amount dissolved F2** | **Amount dissolved F3** | **Amount dissolved F4** | **Amount dissolved F5** |
|---|---|---|---|---|---|
| **5** | 91% | 84% | 72% | **81%** | 67% |
| **10** | 94% | 87% | 90% | **95%** | 92% |
| **15** | 97% | 90% | 93% | **99%** | 95% |
| **20** | 98% | 91% | 95% | **101%** | 96% |
| **30** | 99% | 92% | 95% | **102%** | 97% |

**Table 6: Amount of dissolved Spironolactone according to time for each of the formulations**

| **Time (min)** | **Amount dissolved F1** | **Amount dissolved F2** | **Amount dissolved F3** | **Amount dissolved F4** | **Amount dissolved F5** |
|---|---|---|---|---|---|
| **15** | 71.6% | 76.4% | 70.8% | **81.6%** | 82.4% |
| **30** | 96.4% | 96.4% | 94% | **105.2%** | 101.6% |
| **45** | 104.4% | 106% | 108% | **112.8%** | 110.8% |
| **60** | 111.6% | 112.4% | 108.8% | **117.2%** | 114.8% |

**Table 7: Amount of Furosemide dissolved according to time for each of the formulations**

| **Time (min)** | **Amount dissolved F1** | **Amount dissolved F2** | **Amount dissolved F3** | **Amount dissolved F4** | **Amount dissolved F5** |
|---|---|---|---|---|---|
| **15** | 78% | 77% | 84% | **78%** | 80% |
| **30** | 88% | 88% | 94% | **90%** | 90% |
| **45** | 91% | 91% | 88% | **88%** | 88% |
| **60** | 94% | 92% | 88% | **88%** | 88% |

The graph results of the quantities of active ingredients dissolved according to time for each of the molecules are shown in Figures 1-4.

### Discussion:

According to the dissolution test of Bisoprolol USP monograph, the capsules are compliant if after 20 minutes the amount of Bisoprolol dissolved in each of the 6 capsules is not less than 85%. At the end of the 20 minutes, the amount of dissolved Bisoprolol is between 109.6% and 116.4% for the five formulations (Figure 1).

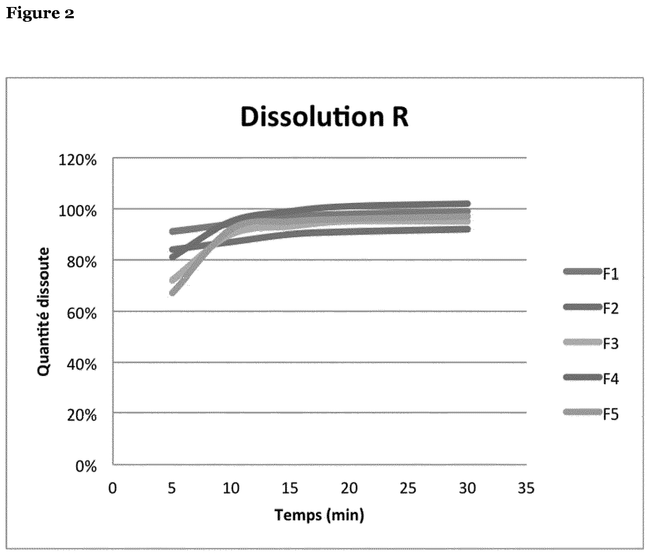

According to the dissolution test of Ramipril USP monograph, the capsules are compliant if after 30 minutes the amount of Ramipril dissolved in each of the 6 capsules is not less than 85%. At the end of the 30 minutes, the amount of ramipril dissolved is between 92% and 102% for the five formulations (Figure 2).

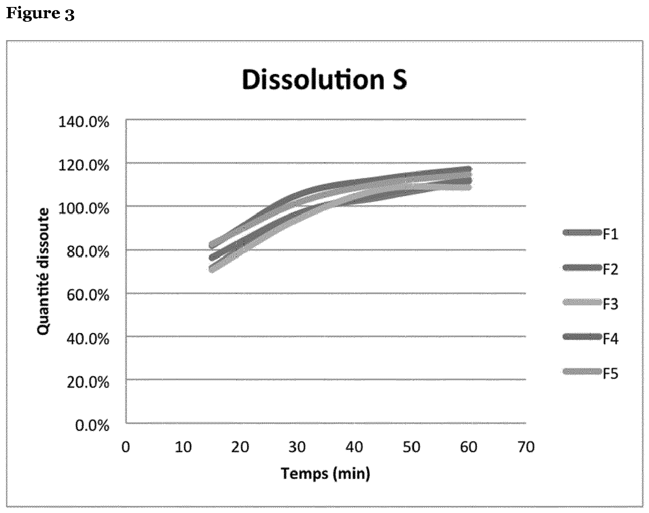

According to the dissolution test of Spironolactone USP Monograph, the capsules are compliant if after 60 minutes the amount of Spironolactone dissolved in each of the 6 capsules is not less than 80%. At the end of the 60 minutes, the amount of dissolved Spironolactone is between 108.8% and 117.2% for the five formulations (Figure 3).

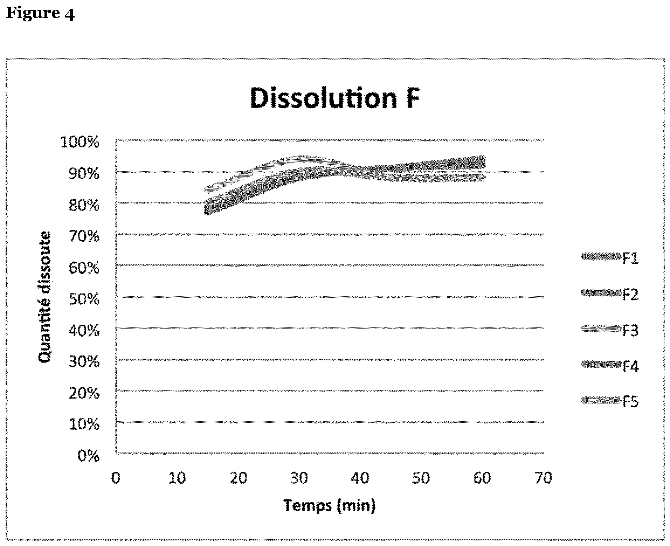

According to the dissolution test of Furosemide USP monograph, the capsules are compliant if after 60 minutes the amount of Furosemide dissolved in each of the 6 capsules is not less than 85%. At the end of the 60 minutes, the amount of dissolved Furosemide is between 88% and 94% for the five formulations (Figure 4).

### Conclusion:

All five formulations meet the USP acceptance criteria for the dissolution of Bisoprolol, Ramipril, Spironolactone and Furosemide.

Formulation 4 is that which has been validated for the development of the capsule.

A new batch was manufactured in order to carry out the stability studies under normal and accelerated conditions.

### Example 8

Specific composition for a combination medication according to the present invention:
First combination: dose No. 1 for a certain time period 1 in the form of a single pill, which comprises
Betablocker 2.5 mg
ACE inhibitor 5 mg
MRA 10 mg
Glifozine 10 mg

Second combination (after weeks of treatment with dose No. 1): dose No. 2 for a certain time period 2 in the form of a single pill, which comprises
Betablocker 5 mg
ACE inhibitor 10 mg
MRA 20 mg
Glifozine 20 mg

### Example 9

Specific composition for a combination medication according to the present invention: Dose No. 1 (one single pill) for a certain time period 1

The full dose pill produced includes:
- bisoprolol 5 mg
- ramipril 5 mg
- spironolactone 12.5 mg
- furosemide 40 mg

Dose No. 2 (one single pill) after weeks of treatment with dose No. 1 for administration for a certain time period 2

The full dose pill produced includes:
- bisoprolol 10 mg
- ramipril 10 mg
- spironolactone 25 mg
- furosemide 80 mg

## Claims

1. Combination medication for use in the treatment of a patient having acute and/or persistent dyspnea for restoring the respiration rate and/or oxygen saturation toward normal values and/or maintaining the respiration rate and/or restoring and/or maintaining the oxygen saturation and/or for ameliorating the symptoms of dyspnea comprising at least three of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozin or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea;
e. a diuretic to reduce or prevent occurrence of signs and/or symptoms of congestion in patients,
and wherein a sample of bodily fluid of said patient has a level of Brain Natriuretic peptide (BNP) > 200 pg/mL and/or has a level of N-terminal (NT)-pro hormone BNP(NT-proBNP) > 600 pg/mL and/or the patient is congestive and/or has residual congestion, and wherein said sample of bodily fluid is selected from the group comprising blood, plasma and serum;
and wherein said patient is either
- a patient with heart failure with left ventricular ejection fraction (LVEF) greater and equal to 40 %, preferably 50 % or
- a patient with no heart failure.

2. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to claim 1, wherein said combination comprises an inhibitor of the sodium glucose co-transporter-2, e.g. dapagliflozin or empagliflozin that is used for restoring the respiration rate and/or maintaining the respiration rate and/or restoring and/or maintaining the oxygen saturation and/or for ameliorating the symptoms of dyspnea.

3. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to claim 1 to 2, wherein said patient does not have diabetes.

4. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 3, wherein said patient has a myocardial dysfunction.

5. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 4, wherein the treatment is a daily administration of the combination medication, preferably once a day.

6. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 5, wherein the patient has no heart failure, but has another cardiovascular disease and/or another disease with non cardiac cause which maybe selected from the group comprising diabetes and kidney disease, and wherein a sample of bodily fluid of said patient has a level of BNP > 200 pg/mL and/or has a level NT-proBNP > 600 pg/mL, wherein the bodily fluid is a plasma sample, preferably a fasting plasma sample.

7. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 6, wherein said combination is a fixed combination and wherein said combination is a fixed combination in one single administration form, wherein said administration form is selected from the group comprising a pill, a tablet, a liqiuid medicine, a capsule, a film tablet, a dragee.

8. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 7, wherein the level of proAdrenomedullin (proADM) and/or fragments thereof, preferably mature Adrenomedullin (ADM-NH₂), having at least 5 amino acids in a sample of bodily fluid of said patient is above a certain threshold, wherein said sample of bodily fluid is selected from the group comprising plasma, serum, blood.

9. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 8, wherein the agent according to group b. (iii) of said combination medication according to claim 1 is a combination of angiotensin II inhibitor and angiotensin receptor-neprilysin inhibitor (ARNi), wherein in particular the ARNi is a combination of
• the neprilysin inhibitor 4-[[(2*S*,4*R*)-5-ethoxy-4-methyl-5-oxo-1-(4-phenylphenyl)pentan-2-yl]amino]-4-oxobutanoic acid, and
• the nonpeptide triazole-derived antagonist of angiotensin (AT) II (2*S*)-3-methyl-2-[pentanoyl-[[4-[2-(2*H*-tetrazol-5-yl)phenyl]phenyl]methyl]amino]butanoic acid II;
preferably a fixed combination.

10. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 9, wherein said patient receives the combination medication in two different doses for a certain period of time, respectively, wherein said doses are named dose No 1 and dose No 2., wherein each dose No. 1 and No.2 refers to the respective dose of each of the chemical entities of the combination, wherein dose No. 1 is initially administered to said patient in an initial daily dose of each of the chemical entities of the combination for a certain period of time and is thereafter administered in a subsequent dose No. 2 for a certain period of time that is 2 to 4-fold higher than the said initial daily dose.

11. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 10, wherein said patient receives a dose No 1 of said combination medication for up to 2 to 4 weeks and thereafter a dose No 2 for at least 90 days, wherein dose No 1 is one quarter dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

12. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 11, wherein a beta blocker is selected from the group comprising beta-1 adrenergic receptor antagonist 1-(propan-2-ylamino)-3-[4-(2-propan-2-yloxyethoxymethyl)phenoxy]propan-2-ol, beta-adrenoceptor blocking agent 1-(9H-carbazol-4-yloxy)-3-[2-(2-methoxyphenoxy)ethylamino]propan-2-ol, succinate of 1-[4-(2-methoxyethyl)phenoxy]-3-(propan-2-ylamino)propan-2-ol (CR/XL), the beta-1 adrenergic receptor antagonist 1-(6-fluoro-3,4-dihydro-2H-chromen-2-yl)-2-[[2-(6-fluoro-3,4-dihydro-2H-chromen-2-yl)-2-hydroxyethyl] amino] ethanol, (2R,3S)-5-[3-(tert-butylamino)-2-hydroxypropoxy]-1,2,3,4tetrahydronaphthalene-2,3-diol, N-[3-acetyl-4-[2-hydroxy-3-(propan-2-ylamino)propoxy]phenyl]butanamide, 2-hydroxy-5-[1-hydroxy-2-(4-phenylbutan-2-ylamino)ethyl]benzamide, 3-[3-acetyl-4-[3-(tert-butylamino)-2-hydroxypropoxy]phenyl]-1,1-diethylurea, 2-[4-[2-hydroxy-3-(propan-2-ylamino)propoxy]phenyl]acetamide, 1-naphthalen-1-yloxy-3-(propan-2-ylamino)propan-2-ol.

13. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 11, wherein the ACE inhibitor is selected from the group comprising the sulfhydryl-containing analog of proline (2S)-1-[(2S)-2-methyl-3-sulfanylpropanoyl]pyrrolidine-2-carboxylic acid, (2S)-1-[(2S)-2-[[(2S)-1-ethoxy-1-oxo-4-phenylbutan-2-yl]amino]propanoyl]pyrrolidine-2-carboxylic acid, (2S)-1-[(2S)-6-amino-2-[[(1S)-1-carboxy-3-phenylpropyl]amino]hexanoyl]pyrrolidine-2-carboxylic acid, (2S,3aS,6aS)-1-[(2S)-2-[[(2S)-1-ethoxy-1-oxo-4-phenylbutan-2-yl]amino]propanoyl]-3,3a,4,5,6,6a-hexahydro-2H-cyclopenta[b]pyrrole-2-carboxylic acid, (2S,3aR,7aS)-1-[(2S)-2-[[(2S)-1-ethoxy-1-oxo-4-phenylbutan-2-yl]amino]propanoyl]-2,3,3a,4,5,6,7,7a-octahydroindole-2-carboxylic acid, 2-[(3S)-3-[[(2S)-1-ethoxy-1-oxo-4-phenylbutan-2-yl]amino]-2-oxo-4,5-dihydro-3H-1-benzazepin-1-yl] acetic acid, (2S,4S)-4-cyclohexyl-1-[2-[(2-methyl-1-propanoyloxypropoxy)-(4-phenylbutyl)phosphoryl]acetyl]pyrrolidine-2-carboxylic acid, (3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid, (2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylicacid;(2S)-2-amino-5-(diaminomethylideneamino) pentanoic acid, (2S,3aS,7aS)-1-[(2S)-2-[[(2S)-1-ethoxy-1-oxopentan-2-yl]amino]propanoyl]-2,3,3a,4,5,6,7,7a-octahydroindole-2-carboxylic acid;2-methylpropan-2-amine, (3S)-2-[(2S)-2-[[(2S)-1-ethoxy-1-oxo-4-phenylbutan-2-yl] amino]propanoyl]-3,4-dihydro-1H-isoquinoline-3 -carboxylic acid, (4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid.

14. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 11, wherein the MRAs (mineralo receptor antagonist) is selected from the group comprising methyl (1R,2S,9R,10R,11S,14R,15S,17R)-2,15-dimethyl-5,5'-dioxospiro[18-oxapentacyclo[8.8.0.01,17.02,7.011,15]octadec-6-ene-14,2'-oxolane]-9-carboxylate, S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate and (4*S*)-4-(4-cyano-2-methoxyphenyl)-5-ethoxy-2,8-dimethyl-1,4-dihydro-1,6-naphthyridine-3-carboxamide.

15. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 11, wherein the diurectic is selected from the group comprising loop-acting diuretics such as 3-(butylamino)-4-phenoxy-5-sulfamoylbenzoic acid, 2-[2,3-dichloro-4-(2-methylidenebutanoyl)phenoxy] acetic acid, 4-chloro-2-(furan-2-ylmethylamino)-5 - sulfamoylbenzoic acid, 1-[4-(3-methylanilino)pyridin-3-yl]sulfonyl-3-propan-2-ylurea, potassium-sparing diuretics such as 3,5-diamino-6-chloro-N-(diaminomethylidene)pyrazine-2-carboxamide,, methyl (1R,2S,9R,10R,11S,14R,15S,17R)-2,15-dimethyl-5,5'-dioxospiro[18-oxapentacyclo[8.8.0.01,17.02,7.011,15]octadec-6-ene-14,2'-oxolane]-9-carboxylate, S-[(7R,8R,9S,10R,13S,14S,17R)-10,13-dimethyl-3,5'-dioxospiro[2,6,7,8,9,11,12,14,15,16-decahydro-1H-cyclopenta[a]phenanthrene-17,2'-oxolane]-7-yl]ethanethioate, 6-phenylpteridine-2,4,7-triamine, thiazide diuretics such as 6-chloro-1,1-dioxo-4H-1λ6,2,4-benzothiadiazine-7-sulfonamide, 2-chloro-5-(1-hydroxy-3-oxo-2H-isoindol-1-yl)benzenesulfonamide, 6-chloro-1,1-dioxo-3,4-dihydro-2H-1lambda6,2,4-benzothiadiazine-7-sulfonamide, 4-chloro-N-(2-methyl-2,3-dihydroindol-1-yl)-3-sulfamoylbenzamide, 7-chloro-2-methyl-3-(2-methylphenyl)-4-oxo-1,2-dihydroquinazoline-6-sulfonamide, and/or mixtures thereof.

16. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1 to 11, wherein the gliflozine is selected from the group comprising(2S,3R,4R,5S,6R)-2-[3-[[5-(4-fluorophenyl)thiophen-2-yl]methyl]-4-methylphenyl]-6-(hydroxymethyl)oxane-3,4,5-triol, (2S,3R,4R,5S,6R)-2-[4-chloro-3-[(4-ethoxyphenyl)methyl]phenyl]-6-(hydroxymethyl)oxane-3,4,5-triol, (2S,3R,4R,5S,6R)-2-[4-chloro-3-[[4-[(3S)-oxolan-3-yl]oxyphenyl]methyl]phenyl]-6-(hydroxymethyl)oxane-3,4,5-triol, (1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol, Luseogliflozin, Remogliflozin etabonate (ethyl [(2R,3S,4S,5R,6S)-3,4,5-trihydroxy-6-[5-methyl-1-propan-2-yl-4-[(4-propan-2-yloxyphenyl)methyl]pyrazol-3-yl]oxyoxan-2-yl]methyl carbonate), sergliflozin etabonate (ethyl [(2R,3S,4S,5R,6S)-3,4,5-trihydroxy-6-[2-[(4-methoxyphenyl)methyl]phenoxy]oxan-2-yl]methyl carbonate), (2S,3R,4R,5S,6R)-2-[3-(1-benzothiophen-2-ylmethyl)-4-fluorophenyl]-6-(hydroxymethyl)oxane-3,4,5-triol, (3S,3'R,4'S,5'S,6'R)-5-[(4-ethylphenyl)methyl]-6'-(hydroxymethyl)spiro[1H-2-benzofuran-3,2'-oxane]-3',4',5'-triol, (2S,3R,4R,5S,6R)-2-[5-[(4-ethoxyphenyl)methyl]-2-methoxy-4-methylphenyl]-6-(hydroxymethyl)thiane-3,4,5-triol, (2S,3R,4R,5S,6R)-2-[4-chloro-3-[(4-ethoxyphenyl)methyl]phenyl]-6-methylsulfanyloxane-3,4,5-triol and/or mixtures thereof.

17. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to any of claims 1-16, wherein the ranges of each of the chemical entities of the combination for dose No. 2 of the following agents are as follows:
**ACE-inhibitors**
Captopril:
(2S)-1-[(2S)-2-methyl-3-sulfanylpropanoyl]pyrrolidine-2-carboxylic acid Dosage range from 37.5 mg/day to 150 mg/day, preferable from 75 mg/day mg to 150 mg/day, more preferably from 112.5 mg/day to 150 mg/day,
Enalapril:
(2S)-1-[(2S)-2-[[(2S)-1-ethoxy-1-oxo-4-phenylbutan-2yl]amino]propanoyl]pyrrolidine-2-carboxylic acid
Dosage range from 5-10 mg/day to 20-40 mg/day, preferable from 10-20 mg/day to 20-40 mg/day, more preferably from 15-30 mg/day to 20-40 mg/day
Lisinopril:
(2S)-1-[(2S)-6-amino-2-[[(1S)-1-carboxy-3-phenylpropyl]amino]hexanoyl]pyrrolidine-2-carboxylic acid
Dosage range from 5-8.75 mg/day to 20-35 mg/day, preferable from 10-17.5 mg/day to 20-35 mg/day, more preferably from 15-26.25 mg/day to 20-35 mg/day
Ramipril:
(2S,3aS,6aS)-1-[(2S)-2-[[(2S)-1-ethoxy-1-oxo-4-phenylbutan-2yl] amino]propanoyl]-3,3a,4,5,6,6a-hexahydro-2H-cyclopenta[b]pyrrole-2-carboxylic acid
Dosage range from 2.5 mg/day to 10 mg/day, preferable from 5 mg/day to 10 mg/day, more preferably from 7.5 mg/day to 10 mg/day
Trandolapril:
(2S,3aR,7aS)-1-[(2S)-2-[[(2S)-1-ethoxy-1-oxo-4-phenylbutan-2-yl]amino]propanoyl]-2,3,3a,4,5,6,7,7a-octahydroindole-2-carboxylic acid
Dosage range from 1 mg/day to 4 mg/day, preferable from 2 mg/day to 4 mg/day, more preferably from 3 mg/day to 4 mg/day
Benazepril:
2-[(3S)-3-[[(2S)-1-ethoxy-1-oxo-4-phenylbutan-2-yl]amino]-2-oxo-4,5-dihydro-3H-1-benzazepin-1-yl]acetic acid
Dosage range from 2.5 mg/day to 10 mg/day, preferable from 5 mg/day to 10 mg/day, more preferably from 7.5 mg/day to 10 mg/day
Fosinopril:
(2S,4S)-4-cyclohexyl-1-[2-[(2-methyl-1-propanoyloxypropoxy)-(4-phenylbutyl)phosphoryl]acetyl]pyrrolidine-2-carboxylic acid
Dosage range from 10 mg/day to 40 mg/day, preferable from 20 mg/day to 40 mg/day, more preferably from 30 mg/day to 40 mg/day
Moexipril:
(3S)-2-[(2S)-2-[[(2S)-1-ethoxy-1-oxo-4-phenylbutan-2yl]amino]propanoyl]-6,7-dimethoxy-3,4-dihydro-1H-isoquinoline-3-carboxylic acid
Dosage range from 7.5 mg/day to 30 mg/day, preferable from 15 mg/day to 30 mg/day, more preferably from 22.5 mg/day to 30 mg/day
Perindopril Arginine:
(2S,3aS,7aS)-1-[(2S)-2-[[(2S)-1-ethoxy-1-oxopentan-2-yl]amino]propanoyl]-2, 3,3a,4,5,6,7,7a-octahydroindole-2-carboxylicacid;(2S)-2-amino-5-(diaminomethylideneamino) pentanoic acid
Dosage range from 2.5 mg/day to 10 mg/day, preferable from 5 mg/day to 10 mg/day, more preferably from 7.5 mg/day to 10 mg/day
Perindopril tert-butylamine:
(2S,3aS,7aS)-1-[(2S)-2-[[(2S)-1-ethoxy-1-oxopentan-2-yl]amino]propanoyl]-2,3,3 a,4,5,6,7,7a-octahydroindole-2-carboxylic acid;2-methylpropan-2-amine
Dosage range from 2 mg/day to 8 mg/day, preferable from 4 mg/day to 8 mg/day, more preferably from 6 mg/day to 8 mg/day
Quinapril :
(3S)-2-[(2S)-2-[[(2S)-1-ethoxy-1-oxo-4-phenylbutan-2-yl]amino]propanoyl]-3,4-dihydro-1H-isoquinoline-3-carboxylic acid
Dosage range from 10 mg/day to 40 mg/day, preferable from 20 mg/day to 40 mg/day, more preferably from 30 mg/day to 40 mg/day
Cilazapril :
(4S,7S)-7-[[(2S)-1-ethoxy-1-oxo-4-phenylbutan-2-yl]amino]-6-oxo-1,2,3,4,7,8,9,10-octahydropyridazino[1,2-a]diazepine-4-carboxylic acid
Dosage range from 1.25 mg/day to 5 mg/day, preferable from 2.5 mg/day to 5 mg/day, more preferably from 3.75 mg/day to 5 mg/day
**Beta-blockers**
Bisoprolol:
1-(propan-2-ylamino)-3-[4-(2-propan-2-yloxyethoxymethyl)phenoxy]propan-2-ol Dosage range from 2.5 mg/day to 10 mg/day, preferable from 5 mg/day to 10 mg/day, more preferably from 7.5 mg/day to 10 mg/day
Carvedilol:
1-(9H-carbazol-4-yloxy)-3-[2-(2-methoxyphenoxy)ethylamino]propan-2-ol Dosage range from 12.5 mg/day to 50 mg/day, preferable from 25 mg/day to 50 mg/day, more preferably from 37.5 mg/day to 50 mg/day
Metoprolol succinate:
succinate of 1-[4-(2-methoxyethyl)phenoxy]-3-(propan-2-ylamino)propan-2-ol Dosage range from 50 mg/day to 200 mg/day, preferable from 100 mg/day to 200 mg/day, more preferably from 150 mg/day to 200 mg/day
Nebivolol:
1-(6-fluoro-3,4-dihydro-2H-chromen-2-yl)-2-[[2-(6-fluoro-3,4-dihydro-2H-chromen-2-yl)-2-hydroxyethyl]amino]ethanol
Dosage range from 2.5 mg/day to 10 mg/day, preferable from 5 mg/day to 10 mg/day, more preferably from 7.5 mg/day to 10 mg/day
Nadolol:
(2R,3S)-5-[3-(tert-butylamino)-2-hydroxypropoxy]-1,2,3,4tetrahydronaphthalene-2,3-diol
Dosage range from 20 mg/day to 80 mg/day, preferable from 40 mg/day to 80 mg/day, more preferably from 60 mg/day to 80 mg/day
Acebutolol :
N-[3-acetyl-4-[2-hydroxy-3-(propan-2-ylamino)propoxy]phenyl]butanamide
Dosage range from 100 mg/day to 400 mg/day, preferable from 200 mg/day to 400 mg/day, more preferably from 300 mg/day to 400 mg/day
Labetolol :
2-hydroxy-5-[1-hydroxy-2-(4-phenylbutan-2-ylamino)ethyl]benzamide
Dosage range from 200 mg/day to 800 mg/day, preferable from 400 mg/day to 800 mg/day, more preferably from 600 mg/day to 800 mg/day
Celiprolol :
3-[3-acetyl-4-[3-(tert-butylamino)-2-hydroxypropoxy]phenyl]-1,1-diethylurea Dosage range from 150 mg/day to 600 mg/day, preferable from 300 mg/day to 600 mg/day, more preferably from 450 mg/day to 600 mg/day
Atenolol :
2-[4-[2-hydroxy-3-(propan-2-ylamino)propoxy]phenyl]acetamide
Dosage range from 25 mg/day to 100 mg/day, preferable from 50 mg/day to 100 mg/day, more preferably from 75 mg/day to 100 mg/day
Propranolol :
1-naphthalen-1-yloxy-3 -(propan-2-ylamino)propan-2-ol
Dosage range from 40 mg/day to 160 mg/day, preferable from 80 mg/day to 160 mg/day, more preferably from 120 mg/day to 160 mg/day
**Angiotensin Receptor Blockers**
Candesartan:
2-ethoxy-3-[[4-[2-(2H-tetrazol-5-yl)phenyl]phenyl]methyl]benzimidazole-4-carboxylic acid
Dosage range from 8 mg/day to 32 mg/day, preferable from 16 mg/day to 32 mg/day, more preferably from 24 mg/day to 32 mg/da
Valsartan:
(2S)-3-methyl-2-[pentanoyl-[[4-[2-(2H-tetrazol-5-yl)phenyl]phenyl]methyl] amino]butanoic acid
Dosage range from 80 mg/day to 320 mg/day, preferable from 160 mg/day to 320 mg/day, more preferably from 240 mg/day to 320 mg/day
Losartan:
[2-butyl-5-chloro-3-[[4-[2-(2H-tetrazol-5-yl)phenyl]phenyl]methyl]imidazol-4-yl]methanol
Dosage range from 37.5 mg/day to 150 mg/day, preferable from 75 mg/day to 150 mg/day, more preferably from 112.5 mg/day to 150 mg/day
Azilsartan:
2-ethoxy-3-[[4-[2-(5-oxo-4H-1,2,4-oxadiazol-3-yl)phenyl]phenyl]methyl] benzimidazole-4-carboxylic acid
Dosage range from 20 mg/day to 80 mg/day, preferable from 40 mg/day to 80 mg/day, more preferably from 60 mg/day to 80 mg/day
Eprosartan:
4-[[2-butyl-5-[(E)-2-carboxy-3-thiophen-2-ylprop-1-enyl]imidazol-1-yl] methyl]benzoic acid
Dosage range from 150 mg/day to 600 mg/day, preferable from 300 mg/day to 600 mg/day, more preferably from 450 mg/day to 600 mg/day
Irbesartan:
2-butyl-3-[[4-[2-(2H-tetrazol-5-yl)phenyl]phenyl]methyl]-1,3-diazaspiro[4.4]non-1-en-4-one
Dosage range from 75 mg/day to 300 mg/day, preferable from 150 mg/day to 300 mg/day, more preferably from 225 mg/day to 300 mg/day
Olmesartan:
5-(2-hydroxypropan-2-yl)-2-propyl-3-[[4-[2-(2H-tetrazol-5-yl)phenyl]phenyl]methyl]imidazole-4-carboxylic acid
Dosage range from 10 mg/day to 40 mg/day, preferable from 20 mg/day to 40 mg/day, more preferably from 30 mg/day to 40 mg/day
Telmisartan:
2-[4-[[4-methyl-6-(1-methylbenzimidazol-2-yl)-2-propylbenzimidazol-1-yl]methyl]phenyl]benzoic acid
Dosage range from 20 mg/day to 80 mg/day, preferable from 40 mg/day to 80 mg/day, more preferably from 60 mg/day to 80 mg/day
**Mineraloreceptor antagonist (MRAs)**
Eplerenone:
methyl(1R,2S,9R,10R,11S,14R,15S,17R)-2,15-dimethyl-5,5'-dioxospiro[18-oxapentacyclo[8.8.0.01,17.02,7.011,15]octadec-6-ene-14,2'-oxolane]-9-carboxylate Dosage range from 12.5 mg/day to 50 mg/day, preferable from 25 mg/day to 50 mg/day, more preferably from 37.5 mg/day to 50 mg/day
Spironolactone:
S-[(7R,8R,9S,10R,13S,14S,17R)-10,13-dimethyl-3,5'-dioxospiro [2,6,7,8,9,11,12,14,15,16-decahydro-1H-cyclopenta[a]phenanthrene-17,2'-oxolane]-7-yl] ethanethioate
Dosage range from 12.5 mg/day to 50 mg/day, preferable from 25 mg/day to 50 mg/day, more preferably from 37.5 mg/day to 50 mg/day
Finerenone:
(4*S*)-4-(4-cyano-2-methoxyphenyl)-5-ethoxy-2,8-dimethyl-1,4-dihydro-1,6-naphthyridine-3-carboxamide
Dosage range from 10 mg/day to 20 mg/day, preferable 15 mg/day to 20 mg/day
**ARNI**
Sacubitril/valsartan:
4-[[(2S,4R)-5-ethoxy-4-methyl-5-oxo-1-(4-phenylphenyl)pentan-2-yl]amino]-4-oxobutanoic acid / (2S)-3-methyl-2-[pentanoyl-[[4-[2-(2H-tetrazol-5-yl)phenyl] phenyl]methyl]amino]butanoic acidII
Dosage range from 48.5mg/51.5mg/day to 194mg/206mg/day, preferable from 97mg/103mg/day to 194mg/206mg/day, more preferably from 145.5mg/154.5mg/day to 194mg/206mg/day
**Diuretics**
Furosemide:
4-chloro-2-(furan-2-ylmethylamino)-5-sulfamoylbenzoic acid
Dosage range from 10-60 mg/day to 40-240 mg/day, preferable from 20-120 mg/day to 40-240 mg/day, more preferably from 30-180 mg/day to 40-240 mg/day
Bumetanide:
3-(butylamino)-4-phenoxy-5-sulfamoylbenzoic acid
Dosage range from 0.25-1.25 mg/day to 1-5 mg/day, preferable from 0.5-2.5 mg/day to 1-5 mg/day, more preferably from 0.75-3.75 mg/day to 1-5 mg/day
Torasemide:
1-[4-(3-methylanilino)pyridin-3-yl]sulfonyl-3-propan-2-ylurea
Dosage range from 2.5-5 mg/day to 10-20 mg/day, preferable from 5-10 mg/day to 10-20 mg/day, more preferably from 7.5-15 mg/day to 10-20 mg/day
Bendroflumethiazide:
3-benzyl-1,1-dioxo-6-(trifluoromethyl)-3,4-dihydro-2H-1lambda6,2,4-benzothiadiazine-7-sulfonamide
Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day
Hydrochlorothiazide:
6-chloro-1,1-dioxo-3,4-dihydro-2H-1lambda6,2,4-benzothiadiazine-7-sulfonamide Dosage range from 3.125-25 mg/day to 12.5-100 mg/day, preferable from 6.25-50 mg/day to 12.5-100 mg/day, more preferably from 9.375-75 mg/day to 12.5-100 mg/day
Metolazone:
7-chloro-2-methyl-3-(2-methylphenyl)-4-oxo-1,2-dihydroquinazoline-6-sulfonamide Dosage range from 0.625-2.5 mg/day to 2.5-10 mg/day, preferable from 1.25-5 mg/day to 2.5-10 mg/day, more preferably from 1.875-7.5 mg/day to 2.5-10 mg/day
Indapamide:
4-chloro-N-(2-methyl-2,3-dihydroindol-1-yl)-3-sulfamoylbenzamide
Dosage range from 0.625-1.25 mg/day to 2.5-5 mg/day, preferable from 1.25-2.5 mg/day to 2.5-5 mg/day, more preferably from 1.875-3.75 mg/day to 2.5-5 mg/day
**Gliflozine**
Dapagliflozin:
(2S,3R,4R,5S,6R)-2-[4-chloro-3-[(4-ethoxyphenyl)methyl]phenyl]-6-(hydroxymethyl)oxane-3,4,5-triol
Dosage range from 2.5 mg/day to 10 mg/day, preferable from 5 mg/day to 10 mg/day, more preferably from 7.5 mg/day to 10 mg/day
Canagliflozin:
(2S,3R,4R,5S,6R)-2-[3-[[5-(4-fluorophenyl)thiophen-2-yl]methyl]-4-methylphenyl]-6-(hydroxymethyl)oxane-3,4,5-triol
Dosage range from 75 mg/day to 300 mg/day, preferable from 150 mg/day to 300 mg/day, more preferably from 225 mg/day to 300 mg/day
Empagliflozin :
(2S,3R,4R,5S,6R)-2-[4-chloro-3-[[4-[(3S)-oxolan-3-yl]oxyphenyl]methyl]phenyl]-6-(hydroxymethyl)oxane-3,4,5-triol
Dosage range from 6.25 mg/day to 25 mg/day, preferable from 10 mg/day to 25 mg/day, more preferably from 10 mg/day to 20 mg/day
Ertugliflozin :
(1S,2S,3S,4R,5S)-5-[4-chloro-3-[(4-ethoxyphenyl)methyl]phenyl]-1-(hydroxymethyl)-6,8-dioxabicyclo[3.2.1]octane-2,3,4-triol
Dosage range from 3.75 mg/day to 15 mg/day, preferable from 7.5 mg/day to 15 mg/day, more preferably from 11.25 mg/day to 15 mg/day
Ipragliflozin
(2S,3R,4R,5S,6R)-2-[3-(1-benzothiophen-2-ylmethyl)-4-fluorophenyl]-6-(hydroxymethyl)oxane-3,4,5-triol
Dosage range from 25 mg/day to 100 mg/day, preferable from 50 mg/day to 100 mg/day, more preferably from 75 mg/day to 100 mg/day
Tofogliflozin
(3S,3'R,4'S,5'S,6'R)-5-[(4-ethylphenyl)methyl]-6'-(hydroxymethyl)spiro[1H-2-benzofuran-3,2'-oxane]-3',4',5'-triol
Dosage range from 5 mg/day to 20 mg/day, preferable from 10 mg/day to 20 mg/day, more preferably from 15 mg/day to 20 mg/day
Luseogliflozin
(2S,3R,4R,5S,6R)-2-[5-[(4-ethoxyphenyl)methyl]-2-methoxy-4-methylphenyl]-6-(hydroxymethyl)thiane-3,4,5-triol
Dosage range from 1.25 mg/day to 5 mg/day, preferable from 2.5 mg/day to 5m g/day, more preferably from 3.75 mg/day to 5 mg/day
Sotagliflozin:
(2S,3R,4R,5S,6R)-2-[4-chloro-3-[(4-ethoxyphenyl)methyl]phenyl]-6-methylsulfanyloxane-3,4, 5-triol
Dosage range from 100 mg/day to 400 mg/day, preferable from 200mg/day to 400 mg/day, more preferably from 300 mg/day to 400 mg/day.

18. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising at least three of the following compounds wherein each of said chemical compounds is a chemical entity, wherein each chemical entity is distinct from the other two:
f. a beta blocker (BB),
g. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
h. and mineralo receptor antagonist (MRA),
i. an inhibitor of the sodium glucose co-transporter-2 (SGLT2), e.g. dapagliflozin or empagliflozin, and wherein said inhibitor of the sodium glucose co-transporter-2, e.g. gliflozine or empagliflozin, is used for restoring the restoration rate and/or maintaining (a normal) respiration rate and/or restoring the oxygen saturation and/or for ameliorating the symptoms of dyspnea;
j. a diuretic to reduce signs and/or symptoms of congestion in patients,
and wherein a sample of bodily fluid of said patient has a level of brain natriuretic peptide (BNP) > 200 pg/mL and/or has a level of N-terminal brain natriuretic peptide (NT-proBNP) > 600 pg/mL and/or the patient is congestive and/or has residual congestion, and wherein said sample of bodily fluid is selected from the group comprising blood, plasma and serum,
and wherein the compounds are administered to said patient at the same time in one single administration form, preferably in one single pill,
and wherein said patient is either
- a patient with heart failure with LVEF greater and equal to 40 %, preferably 50 % or
- a patient with no heart failure.

19. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising at least four of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor andneprilysin inhibitor (ARNi)
c. and mineralo receptor antagonist (MRA),
d. a diuretic to reduce signs and/or symptoms of congestion in patients,
and wherein a sample of bodily fluid of said patient has a level of brain natriuretic peptide (BNP) > 200 pg/mL and/or has a level of N-terminal brain natriuretic peptide (NT-proBNP) > 600 pg/mL, and/or the patient is congestive and/or has residual congestion, and wherein said sample of bodily fluid is selected from the group comprising blood, plasma and serum,
and wherein said patient is either
- a patient with heart failure with LVEF greater and equal to 40 %, preferably 50 %, or
- a patient with no heart failure.

20. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising at least four compounds wherein each of said chemical compounds is a chemical entity, wherein each chemical entity is distinct from the other two:
a. Bisoprolol as a beta blocker (BB),
b. Ramipril as one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. a diuretic to reduce or prevent occurrence of signs and/or symptoms of congestion in patients
and wherein a sample of bodily fluid of said patient has a level of brain natriuretic peptide (BNP) > 200 pg/mL and/or has a level of N-terminal brain natriuretic peptide (NT-proBNP) > 600 pg/mL, and/or the patient is congestive and/or has residual congestion, and wherein said sample of bodily fluid is selected from the group comprising blood, plasma and serum,
and wherein said patient is either
- a patient with heart failure with LVEF, greater and equal to 40 %, preferably 50 % or
- a patient with no heart failure.

21. Pharmaceutical oral formulation for use in the treatment of a patient having acute and/or persistent dyspnea for restoring the respiration rate and/or maintaining the respiration rate and/or restoring and/or maintaining the oxygen saturation and/or for ameliorating the symptoms of dyspnea comprising a combination medication according to any of claims 1 - 21, and wherein a sample of bodily fluid of said patient has a level of BNP > 200 pg/mL and/or has a level NT-proBNP > 600 pg/mL, and/or the patient is congestive, and/or has residual congestion and wherein said sample of bodily fluid is selected from the group comprising blood, plasma and serum.

22. Combination medication for use in the treatment of a patient having acute and/or persistent dyspnea for restoring the respiration rate and/or maintaining the respiration rate and/or restoring and/or maintaining the oxygen saturation and/or for ameliorating the symptoms of dyspnea according to claim 1 and following claims comprising at least three of the following compounds wherein each of said chemical compounds is a chemical entity, wherein each chemical entity is distinct from the other two :
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and inhibitor (ARNi)
c. and mineralo receptor antagonist (MRA),
d. an inhibitor of the sodium glucose co-transporter-2 (SGLT2), e.g. dapagliflozin or empagliflozin, and wherein said inhibitor of the sodium glucose co-transporter-2, e.g. gliflozine or empagliflozin, is used for restoring the restoration rate and/or maintaining the respiration rate and/or restoring or maintaining the oxygen saturation and/or for ameliorating the symptoms of dyspnea,
e. a diuretic to reduce signs and/or symptoms of congestion in patients
and wherein a sample of bodily fluid of said patient has a level of BNP > 200 pg/mL and/or has a level NT-proBNP > 600 pg/mL, and/or the patient is congestive and/or has residual congestion, and wherein said sample of bodily fluid is selected from the group comprising blood, plasma and serum,
wherein said patient is
- a patient with heart failure with LVEF, greater and equal to 40 %, preferably 50 % or
- a patient with no heart failure, and
wherein the treatment is a daily administration of the combination medication,
preferably once a day, and
wherein said combination is a fixed combination and wherein said combination is a fixed combination in one administration form, wherein said administration form is selected from the group comprising a pill, a tablet, a liqiuid medicine, a capsule, a film tablet, a dragee, and
wherein the level of proADM and/or fragments thereof having at least 5 amino acids in a sample of bodily fluid of said patient is above a certain threshold, and wherein said sample of bodily fluid is selected from the group comprising plasma, serum, blood, and
wherein said patient receives the combination medication in two different doses, which are named dose No 1 and dose No 2, and
wherein said patient receives a dose No 1 of said combination medication for up to 2 to 4 weeks and thereafter a dose No 2 for at least 90 days, and
wherein dose No 1 is a quarter dose (25%) of dose No 2, more preferably dose No 1 is 35% of dose No 2, more preferably dose No 1 is 40% of dose No 2, more preferably dose No 1 is 45% of dose No 2, more preferably dose No 1 is 50% of dose No 2.

23. Combination medication for use in the treatment of a patient having acute and/or persistent dyspnea according to claim 1 and following claims comprising at least three of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and-neprilysin inhibitor (ARNi)
c. and mineralo receptor antagonist (MRA),
d. an inhibitor of the sodium glucose co-transporter-2 (SGLT2), e.g. dapagliflozine or empagliflozin, and wherein said inhibitor of the sodium glucose co-transporter-2, e.g. gliflozine or empagliflozin, is used for restoring the restoration rate and/or maintaining (a normal) respiration rate and/or restoring the oxygen saturation and/or for ameliorating the symptoms of dyspnea,
e. a diuretic to reduce signs and/or symptoms of congestion in patients,
and wherein a sample of bodily fluid of said patient has a level of BNP > 200 pg/mL and/or has a level NT-proBNP > 600 pg/mL, and/or the patient is congestive and/or has residual congestion, and wherein said sample of bodily fluid is selected from the group comprising blood, plasma and serum, and
wherein the treatment is a daily administration of the combination medication, preferably once a day, and
wherein the patient has no heart failure but has another cardiovascular disease and/or another disease with non cardiac cause which maybe selected from the group comprising diabetes and kidney disease,
and wherein said combination is a fixed combination and wherein said combination is a fixed combination in one administration form, wherein said administration form is selected from the group comprising a pill, a tablet, a liqiuid medicine, a capsule, a film tablet, a dragee,
and wherein the level of proADM and/or fragments thereof having at least 5 amino acids in a sample of bodily fluid of said patient is above a certain threshold, wherein said sample of bodily fluid is selected from the group comprising plasma, serum, blood,
and wherein said patient receives a dose No 1 of said combination medication for up to 2 to 4 weeks and thereafter a dose No 2 for at least 90 days, wherein dose No 1 is half dose (25%) of dose No 2, more preferably .dose No 1 is 35% of dose No 2, more preferably .dose No 1 is 40% of dose No 2, more preferably .dose No 1 is 45% of dose No 2, more preferably dose No 1 is 50% of dose No 2.

24. Combination medication for use in the treatment of a patient having acute and/or persistent dyspnea for restoring the respiration rate and/or oxygen saturation toward normal values and/or maintaining the respiration rate and/or restoring and/or maintaining the oxygen saturation and/or for ameliorating the symptoms of dyspnea comprising at least three of the following compounds wherein each of said chemical compounds is a chemical entity, wherein each chemical entity is distinct from the other two:
a. a beta blocker (BB),
b. one agent of the group of *renin-angiotensin-aldosterone system* (RAAS) *inhibitors* consisting of (i) an angiotensin-converting enzyme (ACE) inhibitor, (ii) an angiotensin II inhibitor and (iii) a combination of angiotensin II inhibitor and neprilysin inhibitor (ARNi)
c. and mineralo receptor antagonist (MRA),
d. an inhibitor of the sodium glucose co-transporter-2 (SGLT2), also called glifozins, e.g. dapagliflozin or empagliflozin, and wherein said inhibitor of the sodium glucose co-transporter-2, e.g. dapagliflozin or empagliflozin, is used for restoring the restoration rate and/or maintaining respiration rate and/or restoring and/or maintaining the oxygen saturation and/or for ameliorating the symptoms of dyspnea;
e. a diuretic to reduce or prevent occurrence of signs and/or symptoms of congestion in patients,
and wherein a sample of bodily fluid of said patient has a level of Brain Natriuretic peptide (BNP) > 200 pg/mL and/or has a level of N-terminal (NT)-pro hormone BNP (NT-proBNP) > 600 pg/mL and/or the patient is congestive and/or has residual congestion, and wherein said sample of bodily fluid is selected from the group comprising blood, plasma and serum;
and wherein said patient is either
- a patient with heart failure with left ventricular ejection fraction (LVEF) with less than 40 %, or
- a patient with no heart failure.
